# EUROPEAN PATENT APPLICATION

(11) **EP 4 420 513 A1**
(43) Date of publication of application: **28.08.2024**
(21) Application number: 22883673.0
(22) Date of filing: 21.10.2022
(51) Int. Cl.: A01H 6/40, C12N 15/29

(54) **EUSTOMA PLANT HAVING EUSTOMA DIEBACK RESISTANCE**

(30) Priority: 22.10.2021 JP 2021173508
(71) Applicant: Takii & Co., Ltd., Umekoji-dori, Shimogyo-ku Kyoto-shi Kyoto 600-8686 (JP)
(72) Inventor: ARIMOTO Ryuuhei, Kyoto-shi Kyoto 600-8686 (JP); SENDO Takahiro, Kyoto-shi Kyoto 600-8686 (JP)
(74) Representative: J A Kemp LLP
(86) International application number: PCT/JP2022/039401
(87) International publication number: WO 2023/068371

(57) **Abstract**

The present disclosure provides a *Eustoma* plant that exhibits resistance to *Eustoma* fusarium wilt. The *Eustoma* plant according to the present disclosure is a *Eustoma* fusarium wilt-resistant *Eustoma* plant including a *Eustoma* fusarium wilt resistance locus on chromosome 20.

## Description

### TECHNICAL FIELD

The present disclosure relates to a *Eustoma* plant with *Eustoma* fusarium wilt resistance (hereinafter also referred to as *"Eustoma* fusarium wilt-resistant *Eustoma* plant").

### BACKGROUND ART

*Fusarium oxysporum*, which is a species of ascomycete fungus, is the pathogen causing *Eustoma* fusarium wilt in plants of the genus *Eustoma* (*Eustoma* plants). In the *Eustoma* fusarium wilt, the ascomycete fungus present in soils infects *Eustoma* plants via their roots. In the *Eustoma* fusarium wilt, severe wilting symptoms then appear in aerial parts of the infected *Eustoma* plants, eventually leading to withering of 10% to 30% of the infected *Eustoma* plants (Non-Patent Literature 1). Accordingly, an epidemic of the *Eustoma* fusarium wilt causes a decrease in the yield of flowering plants.

Soil sterilization is known as a method for controlling the *Eustoma* fusarium wilt. Although this method is effective in reducing the percentage of plant individuals that develop the disease, it cannot completely prevent the disease (Non-Patent Literature 2).

### Citation List

### Non-Patent Literatures

Non-Patent Literature 1: Chiara Bertoldo et al., "Genetic diversity and virulence of Italian strains of Fusarium oxysporum isolated from Eustoma grandiflora," European Journal of Plant Pathology, 2015, vol. 141, pages 83-97.
Non-Patent Literature 2: "Seisansya to gijyutsu-shidousya no tame no torukogikyou no tachigare-byo taisaku jirei-syu" (Collection of Case Studies on Measures Against Fusarium Wilt of Prairie Gentians for Producers and Technical Leaders), March 2021, published by the Institute of Vegetable and Floriculture Science of the National Agriculture and Food Research Organization (NARO), pages 1-41.

### SUMMARY OF INVENTION

### Technical Problem

The resistance of various cultivars of *Eustoma* plants to the pathogen of *Eustoma* fusarium wilt has been studied. Although a certain *Eustoma* cultivar (Celeb Rich White) had been expected to have *Eustoma* plant resistance, this cultivar developed *Eustoma* fusarium wilt when cultivated in an agricultural field with sterilized soil (Non-Patent Literature 2). Thus, *Eustoma* plants resistant to *Eustoma* fusarium wilt are not known at present. Therefore, there is demand for a *Eustoma* plant that exhibits resistance to *Eustoma* fusarium wilt.

In light of the above circumstances, it is an object of the present disclosure to provide a *Eustoma* plant that exhibits resistance to *Eustoma* fusarium wilt.

### Solution to Problem

In order to achieve the above object, the present disclosure provides a *Eustoma* fusarium wilt-resistant *Eustoma* plant including:
a *Eustoma* fusarium wilt resistance locus on chromosome 20.

The present disclosure also provides a part of a plant, which is a part of the *Eustoma* fusarium wilt-resistant *Eustoma* plant of the present disclosure.

The present disclosure also provides a method for producing a *Eustoma* fusarium wilt-resistant *Eustoma* plant (hereinafter also referred to simply as "production method"), including the following steps (a) and (b):
(a) crossing the *Eustoma* fusarium wilt-resistant *Eustoma* plant of the present disclosure with another *Eustoma* plant; and
(b) selecting a *Eustoma* fusarium wilt-resistant *Eustoma* plant from one or more *Eustoma* plants obtained in step (a) or one or more progeny lines thereof.

The present disclosure also provides a method for conferring *Eustoma* fusarium wilt resistance to a *Eustoma* plant (hereinafter also referred to simply as the "conferring method"), including:
an introduction step of introducing a *Eustoma* fusarium wilt resistance locus on chromosome 20 to a *Eustoma* plant.

The present disclosure also provides a method for detecting *Eustoma* fusarium wilt resistance of a *Eustoma* plant, including:
a detection step of detecting a *Eustoma* fusarium wilt resistance locus on chromosome 20 in a *Eustoma* plant to be examined.

The present disclosure also provides a screening method for a *Eustoma* fusarium wilt-resistant *Eustoma* plant, including:
a selection step of selecting, from one or more *Eustoma* plants to be examined, a *Eustoma* plant that includes a *Eustoma* fusarium wilt resistance locus on chromosome 20 as a *Eustoma* fusarium wilt-resistant *Eustoma* plant.

### Advantageous Effects of the Invention

The present disclosure can provide a *Eustoma* plant that exhibits resistance to *Eustoma* fusarium wilt.

### BRIEF DESCRIPTION OF THE DRAWINGS

[FIG. 1] FIG. 1 is a schematic view showing relative locations of single nucleotide polymorphisms (SNPs) etc. on chromosome 20.
[FIG. 2] FIG. 2 shows photographs indicating criteria for evaluating the disease index of *Eustoma* plants in Example 1.
[FIG. 3] FIG. 3 is a graph showing the LOD score of chromosome 20 in Example 1.

### DESCRIPTION OF EMBODIMENTS

### <Definitions>

The term "*Eustoma* plant" as used herein refers to a plant classified under the genus *Eustoma* of the family *Gentianaceae.* The *Eustoma* plant is also referred to as, for example, prairie gentian or lisianthus.

The term *"Eustoma* fusarium wilt" as used herein refers to a soil-borne disease caused by *Fusarium* sp., in particular, *Fusarium oxysporum.* It is known that, in the *Eustoma* fusarium wilt, infected plant bodies show wilting symptoms in their aerial parts and may even cause withering.

The term "pathogen of *Eustoma* fusarium wilt" or "pathogen" (hereinafter also referred to as *"Eustoma* fusarium wilt pathogenic fungus") refers to a fungus classified as *Fusarium* sp., in particular, as *Fusarium oxysporum.*

The term *"Eustoma* fusarium wilt resistance" as used herein refers to the ability to inhibit or suppress the occurrence and/or progression of the disease due to infection with the *Eustoma* fusarium wilt pathogenic fungus. As a specific example, the term "*Eustoma* fusarium wilt resistance" means, for example, preventing the occurrence of the disease due to the infection with the *Eustoma* fusarium wilt pathogenic fungus and/or to stopping, inhibiting, suppressing, slowing, or delaying the progression of the disease that has occurred. The *Eustoma* fusarium wilt resistance also can be referred to as *Eustoma* fusarium wilt tolerance or *Eustoma* fusarium wilt endurance.

The term "*Eustoma* fusarium wilt resistance locus (loci)" as used herein (hereinafter also referred to simply as "resistance locus (loci)") refers to a quantitative trait locus (loci) (QTL) or a gene region(s) that confers *Eustoma* fusarium wilt resistance. In general, a quantitative trait locus means a chromosome region associated with the expression of a quantitative trait.

The term "plant body" or "plant" as used herein refers to a plant individual representing the whole plant.

The term "a part of a plant body" or "a part of a plant" as used herein refers to a part of a plant individual.

The present disclosure will be described below with reference to illustrative examples. It is to be noted, however, that the present disclosure is not limited to the following examples, etc, and any changes and modifications may be made therein. In the present disclosure, descriptions regarding one aspect or embodiment can also be applied to another aspect or embodiment, and vice versa, unless otherwise stated. In the present specification, when a numerical range is delimited with the use of "to," numerical values or values representing physical quantities indicated before and after "to" are also included in this numerical range. When an expression like "A and/or B" is used in the present specification, it encompasses the cases of "only A," "only B," and "both A and B."

### <Eustoma Fusarium Wilt Resistance Marker for Eustoma Plant>

In a certain aspect, the present disclosure provides a marker that can be used as an indicator of the *Eustoma* fusarium wilt resistance in a *Eustoma* plant. The marker of the present disclosure is a *Eustoma* fusarium wilt resistance marker for a *Eustoma* plant, characterized in that it includes a *Eustoma* fusarium wilt resistance locus on chromosome 20, i.e., it uses a *Eustoma* fusarium wilt resistance locus on chromosome 20 as an indicator. The marker of the present disclosure is capable of detecting the presence or absence of *Eustoma* fusarium wilt resistance.

Examples of the *Eustoma* plant include *Eustoma grandiflorum* and *Eustoma exaltatum.* The *Eustoma* plant may be a pure line of each *Eustoma* plant species, or may be a hybrid between a *Eustoma* plant of interest and its related species. Examples of the related species include various plant species belonging to the genus *Eustoma.*

The *Eustoma* plant has chromosomes 1 to 36. The respective chromosomes in the *Eustoma* plant can be determined using base sequence (nucleic acid sequence) information on a reference genome (genomic DNA). For example, with reference to base sequence information on the genome of *Eustoma grandiflorum* (cultivar name: 10B-620, available from the National Agriculture and Food Research Organization (NARO), the NARO Genebank), the respective chromosomes of the *Eustoma* plant can be determined, for example, by comparing base sequence information on the genome of the *Eustoma* plant of interest with the base sequence information on the genome of *Eustoma grandiflorum* (10B-620). The comparison can be performed using, for example, analysis software such as BLAST or FASTA with default parameters. As the base sequence information on the genome of *Eustoma grandiflorum* (10B-620), the sequence name Egra_v1.0 is available, for example, on the website (https://plantgarden.jp/en/list/t52518) of plant genome database (Plant GARDEN: https://plantgarden.jp/) provided by Kazusa DNA Research Institute.

In the marker of the present disclosure, the resistance locus on chromosome 20 serves as an indicator. It is to be noted, however, that, in a *Eustoma* plant having the resistance locus, the resistance locus on chromosome 20 may be, for example, on any chromosome other than chromosome 20, instead of on chromosome 20. That is, the *Eustoma* plant having the resistance locus may have the resistance locus on chromosome 20 on any of chromosomes 1, 2, 3, 4, 5, 6, 7, 8, 9, 10, 11, 12, 13, 14, 15, 16, 17, 18, 19, 21, 22, 23, 24, 25, 26, 27, 28, 29, 30, 31, 32, 33, 34, 35, and 36.

The resistance locus is a dominant locus. Thus, in the marker of the present disclosure, the genotype at the resistance locus on chromosome 20 may be either heterozygous or homozygous, for example. In the latter case, the resistant *Eustoma* plant may include at least one of the resistance markers on a chromosome other than chromosome 20. For example, one of the resistance loci may be on a chromosome other than chromosome 20 or the two resistance loci may be on a chromosome(s) other than chromosome 20. When the resistant *Eustoma* plant includes two resistance loci on a chromosome(s) other than chromosome 20, the two resistance loci may be on the same chromosome or on different chromosomes, for example.

The number of *Eustoma* fusarium wilt-resistant genes contributing to the *Eustoma* fusarium wilt resistance of the resistance locus may be one gene or a plurality of genes. As shown in the Examples described below, considering the mode of inheritance of the *Eustoma* fusarium wilt resistance of the present disclosure, it is presumed that the *Eustoma* fusarium wilt resistance conferred by the resistance locus is dominated by a single dominant *Eustoma* fusarium wilt resistance gene. It is to be noted, however, that the above presumption does not limit the present disclosure by any means.

The resistance locus can be specified using a molecular marker that indicates a locus, or location, on a chromosome, i.e., the resistance locus can be identified using a molecular marker linked to a locus of interest. As a technique for specifying a genetic locus such as QTL using the molecular marker, those well known in the art can be utilized.

The molecular marker used for specifying the resistance locus is not limited to particular markers. The molecular marker may be, for example, an SNP marker, an amplified fragment length polymorphism (AFLP) marker, a restriction fragment length polymorphism (RFLP) marker, a microsatellite marker, a sequence-characterized amplified region (SCAR) marker, or a cleaved amplified polymorphic sequence (CAPS) marker. In the present disclosure, one type of molecular marker may be used, or two or more types of molecular markers may be used in combination. When the molecular marker is an SNP marker, one SNP may be used as an SNP marker, or a combination of two or more SNPs may be used as SNP markers, i.e., as an SNP marker set, for example.

The resistance locus is linked to SNP markers as described below. Accordingly, in the present disclosure, the SNP markers can be used to specify the resistance locus, for example. As a specific example, the resistance locus may be specified (also referred to as "identified" hereinafter) by, for example: (1) an SNP marker; (2) a base sequence including an SNP marker; (3) the base sequence of a region between sites of two SNP markers; or any combination thereof. When any combination of (1) to (3) is used to specify the resistance locus, the combination is not limited to particular combinations, and examples thereof include the following combinations:
the combination of (1) and (2);
the combination of (1) and (3);
the combination of (2) and (3); and
the combination of (1), (2), and (3).

### (1) Identification by SNP Marker

The resistance locus may be specified by an SNP marker, as described in (1) above, for example. The SNP marker is not limited to particular SNP markers, and examples thereof include Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378. Regarding the analysis of these SNPs, reference can be made to Reference Literature 1 below, for example. Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 are SNP markers newly identified by the present inventors, and those skilled in the art can identify the chromosomal locations of these SNP markers on the basis of base sequences including these SNP markers, which will be described below.
Reference Literature 1: Erik W. Ohlson et.al., "Identification and Mapping of Late Blight Resistance Quantitative Trait Loci in Tomato Accession PI163245," The Plant Genome, vol. 11, No. 3, 180007.

Egra20_3703734 (also referred to as "SNP (a)" hereinafter) is a polymorphism in which the underlined base in brackets in SEQ ID NO: 1 (the 101st base in SEQ ID NO: 1) is T. That is, SNP (a) indicates that a *Eustoma* plant is resistant to *Eustoma* fusarium wilt when the underlined base is T and is susceptible to *Eustoma* fusarium wilt when the underlined base is other than T (e.g., G). The base sequence of SEQ ID NO: 1 can be obtained, for example, from the *Eustoma* plant deposited under Accession No. FERM BP-22427, as described below. SNP (a) also can be identified, for example, on the basis of known information in the database of the above-described web site etc., and as a specific example, SNP (a) can be identified as the 3703734th base on chromosome 20 of Egra_v1.0.

SEQ ID NO: 1:
5'-TTGACCAACACAAGGTGTCATTCTGCAAACAGGCTTTCGGTATAGCAGCAGTGAA
ACACTCAAACTGTACGTGTCCATGGGTTCAAATTTTTGATCTCTT[T]GAGCAAATCCC
AACCCGAGTACTAACTGAGATATGTCCTGGGGACCGTAGCAAGATATCTAAATGAATG
TGCAGTCTATTCCTATTTTTAACTTACATAT-3'.

Egra20_3759258 (also referred to as "SNP (b)" hereinafter) is a polymorphism in which the underlined base in brackets in the base sequence of SEQ ID NO: 2 below (the 101^{st} base in SEQ ID NO: 2) is A. That is, SNP (b) indicates that a Eustoma plant is resistant to Eustoma fusarium wilt when the underlined base is A and is susceptible to Eustoma fusarium wilt when the underlined base is other than A (e.g., G). The base sequence of SEQ ID NO: 2 can be obtained, for example, from the *Eustoma* plant deposited under Accession No. FERM BP-22427, as described below. SNP (b) also can be identified, for example, on the basis of known information in the database of the above-described web site, and, as a specific example, SNP (b) can be identified as the 3759258th base on chromosome 20 of Egra_v1.0.

SEQ ID NO: 2:
5'-GCTCAAATTTTTTCCAGCTATGGAATCAGCCCCAGAACAGGTTTCCTTTCTTTTTTC
TTCCTGTTCAAAAGCATCCAATTTTATGCATTTTGGTACCTAG[A]TTTGGTGAATTTTG
ATAAAGATTTGATCTTTATAATTTTTTATTGGGGCTTAGGATTCTGAGAAAGTCGGGTC
TCTAAAGGAGGTGAAGATGAATGAAAC-3'.

SNP (b) may also be identified as a polymorphism in which the underlined base in brackets in the base sequence of SEQ ID NO: 79 below (the 51st base in SEQ ID NO: 79) is A.

SEQ ID NO: 79:
5'-TTTTTCTTCCTGTTCAAAAGCATCCAATTTTATGCATTTTGGTACCTAG[A]TTTGGT
GAATTTTGATAAAGATTTGATCTTTATAATTTTTTATTGGGGCT-3'.

Egra20_3839851 (also referred to as "SNP (c)" hereinafter) is a polymorphism in which the underlined base in brackets in the base sequence of SEQ ID NO: 3 below (the ¹⁰1st base in SEQ ID NO: 3) is A. That is, SNP (c) indicates that a Eustoma plant is resistant to Eustoma fusarium wilt when the underlined base is A and is susceptible to Eustoma fusarium wilt when the underlined base is other than A (e.g., C). The base sequence of SEQ ID NO: 3 can be obtained from the *Eustoma* plant deposited under Accession No. FERM BP-22427, as described below, for example. SNP (c) also can be identified, for example, on the basis of known information in the database of the above-described web site, and, as a specific example, SNP (c) can be identified as the 3839851st base on chromosome 20 of Egra_v1.0.

SEQ ID NO: 3:
5'-TTTACAGGAGCGTAACGAGAGGTTATTTTACAAGCTTCTAATTGACAATGTGGACG
AACTCCTTCCTGTGGTGTACACTCCCACTGTTGGTGAGGCTTGC[A]AGAAATATGGAA
GCATTTTCAAGCGTCCTCAGGGTCTCTTCATCAGTTTGAAAGAAAGGTTCTGTCATTT
GTTTTTAATTTGTCTTAAAACGACTACTGT-3'.

SNP (c) may also be identified as a polymorphism in which the underlined base in brackets in the base sequence of SEQ ID NO: 80 below (the 51st base in SEQ ID NO: 80) is A.

SEQ ID NO: 80:
5'-TGGACGAACTCCTTCCTGTGGTGTACACTCCCACTGTTGGTGAGGCTTGC[A]AGA
AATATGGAAGCATTTTCAAGCGTCCTCAGGGTCTCTTCATCAGTTTG-3'.

Egra20_3961378 (also referred to as "SNP (d)" hereinafter) is a polymorphism in which the underlined base in brackets in the base sequence of SEQ ID NO: 4 below (the 76th base in SEQ ID NO: 4) is G. That is, SNP (d) indicates that a Eustoma plant is resistant to Eustoma fusarium wilt when the underlined base is G and is susceptible to Eustoma fusarium wilt when the underlined base is other than G (e.g., A). The base sequence of SEQ ID NO: 4 can be obtained, for example, from the *Eustoma* plant deposited under Accession No. FERM BP-22427, as described below. SNP (d) also can be identified, for example, on the basis of known information in the database of the above-described web site, and, as a specific example, SNP (d) can be identified as the 3961378th base on chromosome 20 of Egra_v1.0.

SEQ ID NO: 4:
5'-TGAAGTTTGGAGAGTCTCTAGGGCTATTAAACATGCATCATTAGTACTTCATGGTGA
TTTCACATGAAGGTGCTT[G]CAGAAAGAATTCACAGGAATGCTTTTTTGTTGTATATC
AAAATATTCGTATTGAATGTCTTTTTTTTTTTCTGGC-3'.

Egra20_3833476 (also referred to as "SNP (f)" hereinafter) is a polymorphism in which the underlined base in brackets in the base sequence of SEQ ID NO: 72 below (the 51st base in SEQ ID NO: 72) is A. That is, SNP (f) indicates that a Eustoma plant is resistant to Eustoma fusarium wilt when the underlined base is A and is susceptible to Eustoma fusarium wilt when the underlined base is other than A (e.g., G). The base sequence of SEQ ID NO: 72 can be obtained, for example, from the *Eustoma* plant deposited under Accession No. FERM BP-22427, as described below. SNP (f) also can be identified, for example, on the basis of known information in the database of the above-described web site, and, as a specific example, SNP (f) can be identified as the 3833476th base on chromosome 20 of Egra_v1.0.

SEQ ID NO: 72:
5'-ATGTCACTTCTAGAACTGATTTGTGCCTTTTATTTGTGTGGTTTTTGGTT[A]GATCAT
CTGTATGAGTACGCAATATTTTGGTTTAATGTTGATCTTAATCA-3'.

Egra20_3849059 (also referred to as "SNP (g)" hereinafter) is a polymorphism in which the underlined base in brackets in the base sequence of SEQ ID NO: 73 below (the 51^{st} base in SEQ ID NO: 73) is T. That is, SNP (g) indicates that a Eustoma plant is resistant to Eustoma fusarium wilt when the underlined base is T and is susceptible to Eustoma fusarium wilt when the underlined base is other than T (e.g., G). The base sequence of SEQ ID NO: 73 can be obtained, for example, from the *Eustoma* plant deposited under Accession No. FERM BP-22427, as described below. SNP (g) also can be identified, for example, on the basis of known information in the database of the above-described web site, and, as a specific example, SNP (g) can be identified as the 3849059th base on chromosome 20 of Egra_v1.0.

SEQ ID NO: 73:
5'-TTTGTTGCAGATAATTACCATCTGAAGAGGATGCAGAAACAAGAATAATC[T]GTGT
AAAATCCCAAGAATCATATTCAGGAACCTCGTTCAACCAAATAACA-3'.

Egra20_3854638 (also referred to as "SNP (h)" hereinafter) is a polymorphism in which the underlined base in brackets in the base sequence of SEQ ID NO: 74 below (the 51st base in SEQ ID NO: 74) is T. That is, SNP (h) indicates that a Eustoma plant is resistant to Eustoma fusarium wilt when the underlined base is T and is susceptible to Eustoma fusarium wilt when the underlined base is other than T (e.g., C). The base sequence of SEQ ID NO: 74 can be obtained, for example, from the *Eustoma* plant deposited under Accession No. FERM BP-22427, as described below. SNP (h) also can be identified, for example, on the basis of known information in the database of the above-described web site, and, as a specific example, SNP (h) can be identified as the 3854638th base on chromosome 20 of Egra_v1.0.

SEQ ID NO: 74:
5'-GAATACAATTTTTACAATATTCAGCCTCCAAATTCCATCCTTCCATTTTA[T]AATTGT
TCTTACATATTCCACAATGTGTGATCTTCTAAATGTTCTTTAAA-3'.

Egra20_3857016 (also referred to as "SNP (i)" hereinafter) is a polymorphism in which the underlined base in brackets in the base sequence of SEQ ID NO: 75 below (the 51^{st} base in SEQ ID NO: 75) is A. That is, SNP (i) indicates that a Eustoma plant is resistant to Eustoma fusarium wilt when the underlined base is A and is susceptible to Eustoma fusarium wilt when the underlined base is other than A (e.g., G). The base sequence of SEQ ID NO: 75 can be obtained, for example, from the *Eustoma* plant deposited under Accession No. FERM BP-22427, as described below. SNP (i) also can be identified, for example, on the basis of known information in the database of the above-described web site, and, as a specific example, SNP (i) can be identified as the 3857016th base on chromosome 20 of Egra_v1.0.

SEQ ID NO: 75:
5'-ACACAACAGGTGCAGTGCTCAACTCAGCCAAACTACTATAAGTATTGACC[A]AGCT
GAGCTGACCACAAGTGTACAACTACGTACTGCTGATACCCTTTCAT-3'.

Egra20_3879982 (also referred to as "SNP (j)" hereinafter) is a polymorphism in which the underlined base in brackets in the base sequence of SEQ ID NO: 76 below (the 51^{st} base in SEQ ID NO: 76) is G. That is, SNP (j) indicates that a Eustoma plant is resistant to Eustoma fusarium wilt when the underlined base is G and is susceptible to Eustoma fusarium wilt when the underlined base is other than G (e.g., A). The base sequence of SEQ ID NO: 76 can be obtained, for example, from the *Eustoma* plant deposited under Accession No. FERM BP-22427, as described below. SNP (j) also can be identified, for example, on the basis of known information in the database of the above-described web site, and, as a specific example, SNP (j) can be identified as the 3879982nd base on chromosome 20 of Egra_v1.0.

SEQ ID NO: 76:
5'-TGATACCGGTATGTTTCGAAGGTGGAGTGAGGATGCTGATTACCTGCTAG[G]ATCC
GGGTTTCAGCAAATAAATAGTTTCTTACCTGCGGTTGCGAGTATCA-3'.

Egra20_3914662 (also referred to as "SNP (k)" hereinafter) is a polymorphism in which the underlined base in brackets in the base sequence of SEQ ID NO: 77 below (the 51st base in SEQ ID NO: 77) is T. That is, SNP (k) indicates that a Eustoma plant is resistant to Eustoma fusarium wilt when the underlined base is T and is susceptible to Eustoma fusarium wilt when the underlined base is other than T (e.g., G). The base sequence of SEQ ID NO: 77 can be obtained, for example, from the *Eustoma* plant deposited under Accession No. FERM BP-22427, as described below. SNP (k) also can be identified, for example, on the basis of known information in the database of the above-described web site, and, as a specific example, SNP (k) can be identified as the 3914662nd base on chromosome 20 of Egra_v1.0.

SEQ ID NO: 77:
5'-GCTGCATCGAGATTTCAAGAATCAAGATCAAAGCACATCAATCCCCAATT[T]CCCT
AACGTTCTGAGCAATTCTTCAAAATAAAAGTACTCCCCAGTCAACA-3'.

Egra20_3939402 (also referred to as "SNP (1)" hereinafter) is a polymorphism in which the underlined base in brackets in the base sequence of SEQ ID NO: 78 below (the 51^{st} base in SEQ ID NO: 78) is A. That is, SNP (1) indicates that a Eustoma plant is resistant to Eustoma fusarium wilt when the underlined base is A and is susceptible to Eustoma fusarium wilt when the underlined base is other than A (e.g., C). The base sequence of SEQ ID NO: 78 can be obtained, for example, from the *Eustoma* plant deposited under Accession No. FERM BP-22427, as described below. SNP (1) also can be identified, for example, on the basis of known information in the database of the above-described web site, and, as a specific example, SNP (1) can be identified as the 3939402nd base on chromosome 20 of Egra_v1.0.

SEQ ID NO: 78:
5'-TTATTCTTTATTTTGTGCAAGAGGTTGTTTCCACGGCTCGAACTCCTAAC[A]TCAAA
GTCACAAAACAGCAACCGTACCAGTTGCTACAAGAACCGTCGTTC-3'.

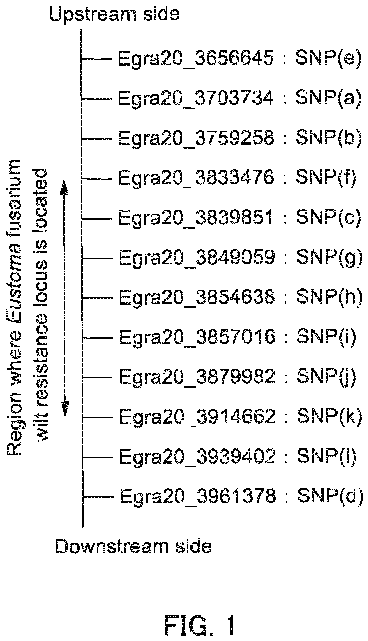

The locations of the SNP markers on the chromosome are not limited to particular locations. For example, as shown in FIG. 1, on chromosome 20 of a *Eustoma* plant, the SNP markers are located in the following order from the upstream side (the Egra20_3656645 side) toward the downstream side (the Egra20_3961378 side): Egra20_3656645, Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378. Although, in FIG. 1, Egra20_3656645 is used to indicate the locations of Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 on chromosome 20 of the *Eustoma* plant, it also may be used as an SNP marker. In this case, in the following description regarding Egra20_3656645, Egra20_3656645 indicates that *a Eustoma* plant is, for example, resistant to *Eustoma* fusarium wilt when Egra20_3656645 is the same base as the corresponding polymorphism in the *Eustoma* plant deposited under Accession No. FERM BP-22427, and is, for example, susceptible to *Eustoma* fusarium wilt when Egra20_3656645 is a base other than the corresponding polymorphism in the *Eustoma* plant deposited under Accession No. FERM BP-22427. When Egra20_3656645 is used as an SNP marker, it may be used in combination with at least one selected from the group consisting of Egra20_3703734, Egra20_3759258, Egra20_3833476, Egra20_3839851, Egra20_3849059, Egra20_3854638, Egra20_3857016, Egra20 _3879982, Egra20_3914662, Egra20_3939402, and Egra20_3961378.

The number of SNP markers present in the resistance locus is not limited to particular values. For example, the resistance locus may include any one of the SNP markers or two or more of the SNP markers, i.e., 2, 3, 4, 5, 6, 7, 8, 9, or 10 of the SNP markers or all the 11 SNP markers. The relevance of these 11 types of polymorphisms (SNP markers) with the *Eustoma* fusarium wilt resistance has not been reported heretofore. They are novel polymorphisms discovered first by the present inventors as being involved in the *Eustoma* fusarium wilt resistance.

The combination of the SNP markers is not limited to particular combinations, and examples thereof include the following combinations:
the combination of SNP (a) and SNP (b);
the combination of SNP (a) and SNP (c);
the combination of SNP (a) and SNP (d);
the combination of SNP (b) and SNP (c);
the combination of SNP (b) and SNP (d);
the combination of SNP (b) and SNP (f);
the combination of SNP (b) and SNP (g);
the combination of SNP (b) and SNP (h);
the combination of SNP (b) and SNP (i);
the combination of SNP (b) and SNP (j);
the combination of SNP (b) and SNP (k);
the combination of SNP (b) and SNP (l);
the combination of SNP (c) and SNP (d);
the combination of SNP (c) and SNP (f);
the combination of SNP (c) and SNP (g);
the combination of SNP (c) and SNP (h);
the combination of SNP (c) and SNP (i);
the combination of SNP (c) and SNP (j);
the combination of SNP (c) and SNP (k);
the combination of SNP (c) and SNP (l);
the combination of SNP (f) and SNP (g);
the combination of SNP (f) and SNP (h);
the combination of SNP (f) and SNP (i);
the combination of SNP (f) and SNP (j);
the combination of SNP (f) and SNP (k);
the combination of SNP (f) and SNP (l);
the combination of SNP (g) and SNP (h);
the combination of SNP (g) and SNP (i);
the combination of SNP (g) and SNP (j);
the combination of SNP (g) and SNP (k);
the combination of SNP (g) and SNP (l);
the combination of SNP (h) and SNP (i);
the combination of SNP (h) and SNP (j);
the combination of SNP (h) and SNP (k);
the combination of SNP (h) and SNP (l);
the combination of SNP (i) and SNP (j);
the combination of SNP (i) and SNP (k);
the combination of SNP (i) and SNP (l);
the combination of SNP (j) and SNP (k);
the combination of SNP (j) and SNP (l);
the combination of SNP (k) and SNP (l);
the combination of SNP (a), SNP (b), and SNP (c);
the combination of SNP (a), SNP (b), and SNP (d);
the combination of SNP (a), SNP (c), and SNP (d);
the combination of SNP (b), SNP (c), and SNP (d);
the combination of SNP (b), SNP (c), and SNP (f);
the combination of SNP (b), SNP (c), and SNP (g);
the combination of SNP (b), SNP (c), and SNP (h);
the combination of SNP (b), SNP (c), and SNP (i);
the combination of SNP (b), SNP (c), and SNP (j);
the combination of SNP (b), SNP (c), and SNP (k);
the combination of SNP (b), SNP (c), and SNP (l);
the combination of SNP (b), SNP (f), and SNP (g);
the combination of SNP (b), SNP (f), and SNP (h);
the combination of SNP (b), SNP (f), and SNP (i);
the combination of SNP (b), SNP (f), and SNP (j);
the combination of SNP (b), SNP (f), and SNP (k);
the combination of SNP (b), SNP (f), and SNP (l);
the combination of SNP (b), SNP (g), and SNP (h);
the combination of SNP (b), SNP (g), and SNP (i);
the combination of SNP (b), SNP (g), and SNP (j);
the combination of SNP (b), SNP (g), and SNP (k);
the combination of SNP (b), SNP (g), and SNP (l);
the combination of SNP (b), SNP (h), and SNP (i);
the combination of SNP (b), SNP (h), and SNP (j);
the combination of SNP (b), SNP (h), and SNP (k);
the combination of SNP (b), SNP (h), and SNP (l);
the combination of SNP (b), SNP (i), and SNP (j);
the combination of SNP (b), SNP (i), and SNP (k);
the combination of SNP (b), SNP (i), and SNP (l);
the combination of SNP (b), SNP (j), and SNP (k);
the combination of SNP (b), SNP (j), and SNP (l);
the combination of SNP (b), SNP (k), and SNP (l);
the combination of SNP (c), SNP (f), and SNP (g);
the combination of SNP (c), SNP (f), and SNP (h);
the combination of SNP (c), SNP (f), and SNP (i);
the combination of SNP (c), SNP (f), and SNP (j);
the combination of SNP (c), SNP (f), and SNP (k);
the combination of SNP (c), SNP (f), and SNP (l);
the combination of SNP (c), SNP (g), and SNP (h);
the combination of SNP (c), SNP (g), and SNP (i);
the combination of SNP (c), SNP (g), and SNP (j);
the combination of SNP (c), SNP (g), and SNP (k);
the combination of SNP (c), SNP (g), and SNP (l);
the combination of SNP (c), SNP (h), and SNP (i);
the combination of SNP (c), SNP (h), and SNP (j);
the combination of SNP (c), SNP (h), and SNP (k);
the combination of SNP (c), SNP (h), and SNP (l);
the combination of SNP (c), SNP (i), and SNP (j);
the combination of SNP (c), SNP (i), and SNP (k);
the combination of SNP (c), SNP (i), and SNP (l);
the combination of SNP (c), SNP (j), and SNP (k);
the combination of SNP (c), SNP (j), and SNP (l);
the combination of SNP (c), SNP (k), and SNP (l);
the combination of SNP (f), SNP (g), and SNP (h);
the combination of SNP (f), SNP (g), and SNP (i);
the combination of SNP (f), SNP (g), and SNP (j);
the combination of SNP (f), SNP (g), and SNP (k);
the combination of SNP (f), SNP (g), and SNP (l);
the combination of SNP (f), SNP (h), and SNP (i);
the combination of SNP (f), SNP (h), and SNP (j);
the combination of SNP (f), SNP (h), and SNP (k);
the combination of SNP (f), SNP (h), and SNP (l);
the combination of SNP (f), SNP (i), and SNP (j);
the combination of SNP (f), SNP (i), and SNP (k);
the combination of SNP (f), SNP (k), and SNP (l);
the combination of SNP (f), SNP (i), and SNP (l);
the combination of SNP (f), SNP (j), and SNP (k);
the combination of SNP (f), SNP (j), and SNP (l);
the combination of SNP (g), SNP (h), and SNP (i);
the combination of SNP (g), SNP (h), and SNP (j);
the combination of SNP (g), SNP (h), and SNP (k);
the combination of SNP (g), SNP (h), and SNP (l);
the combination of SNP (g), SNP (i), and SNP (j);
the combination of SNP (g), SNP (i), and SNP (k);
the combination of SNP (g), SNP (i), and SNP (l);
the combination of SNP (g), SNP (j), and SNP (k);
the combination of SNP (g), SNP (j), and SNP (l);
the combination of SNP (g), SNP (k), and SNP (l);
the combination of SNP (h), SNP (i), and SNP (j);
the combination of SNP (h), SNP (i), and SNP (k);
the combination of SNP (h), SNP (i), and SNP (l);
the combination of SNP (h), SNP (j), and SNP (k);
the combination of SNP (h), SNP (j), and SNP (l);
the combination of SNP (h), SNP (k), and SNP (l);
the combination of SNP (i), SNP (j), and SNP (k);
the combination of SNP (i), SNP (j), and SNP (l);
the combination of SNP (i), SNP (k), and SNP (l);
the combination of SNP (j), SNP (k), and SNP (l);
the combination of SNP (a), SNP (b), SNP (c), and SNP (d);
the combination of SNP (b), SNP (c), SNP (f), and SNP (g);
the combination of SNP (b), SNP (c), SNP (f), and SNP (h);
the combination of SNP (b), SNP (c), SNP (f), and SNP (i);
the combination of SNP (b), SNP (c), SNP (f), and SNP (j);
the combination of SNP (b), SNP (c), SNP (f), and SNP (k);
the combination of SNP (b), SNP (c), SNP (f), and SNP (l);
the combination of SNP (b), SNP (c), SNP (g), and SNP (h);
the combination of SNP (b), SNP (c), SNP (g), and SNP (i);
the combination of SNP (b), SNP (c), SNP (g), and SNP (j);
the combination of SNP (b), SNP (c), SNP (g), and SNP (k);
the combination of SNP (b), SNP (c), SNP (g), and SNP (l);
the combination of SNP (b), SNP (c), SNP (h), and SNP (i);
the combination of SNP (b), SNP (c), SNP (h), and SNP (j);
the combination of SNP (b), SNP (c), SNP (h), and SNP (k);
the combination of SNP (b), SNP (c), SNP (h), and SNP (l);
the combination of SNP (b), SNP (c), SNP (i), and SNP (j);
the combination of SNP (b), SNP (c), SNP (i), and SNP (k);
the combination of SNP (b), SNP (c), SNP (i), and SNP (l);
the combination of SNP (b), SNP (c), SNP (j), and SNP (k);
the combination of SNP (b), SNP (c), SNP (j), and SNP (l);
the combination of SNP (b), SNP (c), SNP (k), and SNP (l);
the combination of SNP (b), SNP (f), SNP (g), and SNP (h);
the combination of SNP (b), SNP (f), SNP (g), and SNP (i);
the combination of SNP (b), SNP (f), SNP (g), and SNP (j);
the combination of SNP (b), SNP (f), SNP (g), and SNP (k);
the combination of SNP (b), SNP (f), SNP (g), and SNP (l);
the combination of SNP (b), SNP (f), SNP (h), and SNP (i);
the combination of SNP (b), SNP (f), SNP (h), and SNP (j);
the combination of SNP (b), SNP (f), SNP (h), and SNP (k);
the combination of SNP (b), SNP (f), SNP (h), and SNP (l);
the combination of SNP (b), SNP (f), SNP (i), and SNP (j);
the combination of SNP (b), SNP (f), SNP (i), and SNP (k);
the combination of SNP (b), SNP (f), SNP (i), and SNP (l);
the combination of SNP (b), SNP (f), SNP (j), and SNP (k);
the combination of SNP (b), SNP (f), SNP (j), and SNP (l);
the combination of SNP (b), SNP (f), SNP (k), and SNP (l);
the combination of SNP (b), SNP (g), SNP (h), and SNP (i);
the combination of SNP (b), SNP (g), SNP (h), and SNP (j);
the combination of SNP (b), SNP (g), SNP (h), and SNP (k);
the combination of SNP (b), SNP (g), SNP (h), and SNP (l);
the combination of SNP (b), SNP (g), SNP (i), and SNP (j);
the combination of SNP (b), SNP (g), SNP (i), and SNP (k);
the combination of SNP (b), SNP (g), SNP (i), and SNP (l);
the combination of SNP (b), SNP (g), SNP (j), and SNP (k);
the combination of SNP (b), SNP (g), SNP (j), and SNP (l);
the combination of SNP (b), SNP (g), SNP (k), and SNP (l);
the combination of SNP (b), SNP (h), SNP (i), and SNP (j);
the combination of SNP (b), SNP (h), SNP (i), and SNP (k);
the combination of SNP (b), SNP (h), SNP (i), and SNP (l);
the combination of SNP (b), SNP (h), SNP (j), and SNP (k);
the combination of SNP (b), SNP (h), SNP (j), and SNP (l);
the combination of SNP (b), SNP (h), SNP (k), and SNP (l);
the combination of SNP (b), SNP (i), SNP (j), and SNP (k);
the combination of SNP (b), SNP (i), SNP (j), and SNP (l);
the combination of SNP (b), SNP (i), SNP (k), and SNP (l);
the combination of SNP (b), SNP (j), SNP (k), and SNP (l);
the combination of SNP (c), SNP (f), SNP (g), and SNP (h);
the combination of SNP (c), SNP (f), SNP (g), and SNP (i);
the combination of SNP (c), SNP (f), SNP (g), and SNP (j);
the combination of SNP (c), SNP (f), SNP (g), and SNP (k);
the combination of SNP (c), SNP (f), SNP (g), and SNP (l);
the combination of SNP (c), SNP (f), SNP (h), and SNP (i);
the combination of SNP (c), SNP (f), SNP (h), and SNP (j);
the combination of SNP (c), SNP (f), SNP (h), and SNP (k);
the combination of SNP (c), SNP (f), SNP (h), and SNP (l);
the combination of SNP (c), SNP (f), SNP (i), and SNP (j);
the combination of SNP (c), SNP (f), SNP (i), and SNP (k);
the combination of SNP (c), SNP (f), SNP (i), and SNP (l);
the combination of SNP (c), SNP (f), SNP (j), and SNP (k);
the combination of SNP (c), SNP (f), SNP (j), and SNP (l);
the combination of SNP (c), SNP (f), SNP (k), and SNP (l);
the combination of SNP (c), SNP (g), SNP (h), and SNP (i);
the combination of SNP (c), SNP (g), SNP (h), and SNP (j);
the combination of SNP (c), SNP (g), SNP (h), and SNP (k);
the combination of SNP (c), SNP (g), SNP (h), and SNP (l);
the combination of SNP (c), SNP (g), SNP (i), and SNP (j);
the combination of SNP (c), SNP (g), SNP (i), and SNP (k);
the combination of SNP (c), SNP (g), SNP (i), and SNP (l);
the combination of SNP (c), SNP (g), SNP (j), and SNP (k);
the combination of SNP (c), SNP (g), SNP (j), and SNP (l);
the combination of SNP (c), SNP (g), SNP (k), and SNP (l);
the combination of SNP (c), SNP (h), SNP (i), and SNP (j);
the combination of SNP (c), SNP (h), SNP (i), and SNP (k);
the combination of SNP (c), SNP (h), SNP (i), and SNP (l);
the combination of SNP (c), SNP (h), SNP (j), and SNP (k);
the combination of SNP (c), SNP (h), SNP (j), and SNP (l);
the combination of SNP (c), SNP (h), SNP (k), and SNP (l);
the combination of SNP (c), SNP (i), SNP (j), and SNP (k);
the combination of SNP (c), SNP (i), SNP (j), and SNP (l);
the combination of SNP (c), SNP (i), SNP (k), and SNP (l);
the combination of SNP (c), SNP (j), SNP (k), and SNP (l);
the combination of SNP (f), SNP (g), SNP (h), and SNP (i);
the combination of SNP (f), SNP (g), SNP (h), and SNP (j);
the combination of SNP (f), SNP (g), SNP (h), and SNP (k);
the combination of SNP (f), SNP (g), SNP (h), and SNP (l);
the combination of SNP (f), SNP (g), SNP (i), and SNP (j);
the combination of SNP (f), SNP (g), SNP (i), and SNP (k);
the combination of SNP (f), SNP (g), SNP (i), and SNP (l);
the combination of SNP (f), SNP (g), SNP (j), and SNP (k);
the combination of SNP (f), SNP (g), SNP (j), and SNP (l);
the combination of SNP (f), SNP (g), SNP (k), and SNP (l);
the combination of SNP (f), SNP (h), SNP (i), and SNP (j);
the combination of SNP (f), SNP (h), SNP (i), and SNP (k);
the combination of SNP (f), SNP (h), SNP (i), and SNP (l);
the combination of SNP (f), SNP (h), SNP (j), and SNP (k);
the combination of SNP (f), SNP (h), SNP (j), and SNP (l);
the combination of SNP (f), SNP (h), SNP (k), and SNP (l);
the combination of SNP (f), SNP (i), SNP (j), and SNP (k);
the combination of SNP (f), SNP (i), SNP (j), and SNP (l);
the combination of SNP (f), SNP (i), SNP (k), and SNP (l);
the combination of SNP (f), SNP (j), SNP (k), and SNP (l);
the combination of SNP (g), SNP (h), SNP (i), and SNP (j);
the combination of SNP (g), SNP (h), SNP (i), and SNP (k);
the combination of SNP (g), SNP (h), SNP (i), and SNP (l);
the combination of SNP (g), SNP (h), SNP (j), and SNP (k);
the combination of SNP (g), SNP (h), SNP (j), and SNP (l);
the combination of SNP (g), SNP (h), SNP (k), and SNP (l);
the combination of SNP (g), SNP (i), SNP (j), and SNP (k);
the combination of SNP (g), SNP (i), SNP (j), and SNP (l);
the combination of SNP (g), SNP (i), SNP (k), and SNP (l);
the combination of SNP (g), SNP (j), SNP (k), and SNP (l);
the combination of SNP (h), SNP (i), SNP (j), and SNP (k);
the combination of SNP (h), SNP (i), SNP (j), and SNP (l);
the combination of SNP (h), SNP (i), SNP (k), and SNP (l);
the combination of SNP (h), SNP (j), SNP (k), and SNP (l);
the combination of SNP (i), SNP (j), SNP (k), and SNP (l);
the combination of SNP (b), SNP (c), SNP (f), SNP (g), and SNP (h);
the combination of SNP (b), SNP (c), SNP (f), SNP (g), and SNP (i);
the combination of SNP (b), SNP (c), SNP (f), SNP (g), and SNP (j);
the combination of SNP (b), SNP (c), SNP (f), SNP (g), and SNP (k);
the combination of SNP (b), SNP (c), SNP (f), SNP (g), and SNP (l);
the combination of SNP (b), SNP (c), SNP (f), SNP (h), and SNP (i);
the combination of SNP (b), SNP (c), SNP (f), SNP (h), and SNP (j);
the combination of SNP (b), SNP (c), SNP (f), SNP (h), and SNP (k);
the combination of SNP (b), SNP (c), SNP (f), SNP (h), and SNP (l);
the combination of SNP (b), SNP (c), SNP (f), SNP (i), and SNP (j);
the combination of SNP (b), SNP (c), SNP (f), SNP (i), and SNP (k);
the combination of SNP (b), SNP (c), SNP (f), SNP (i), and SNP (l);
the combination of SNP (b), SNP (c), SNP (f), SNP (j), and SNP (k);
the combination of SNP (b), SNP (c), SNP (f), SNP (j), and SNP (l);
the combination of SNP (b), SNP (c), SNP (f), SNP (k), and SNP (l);
the combination of SNP (b), SNP (c), SNP (g), SNP (h), and SNP (i);
the combination of SNP (b), SNP (c), SNP (g), SNP (h), and SNP (j);
the combination of SNP (b), SNP (c), SNP (g), SNP (h), and SNP (k);
the combination of SNP (b), SNP (c), SNP (g), SNP (h), and SNP (l);
the combination of SNP (b), SNP (c), SNP (g), SNP (i), and SNP (j);
the combination of SNP (b), SNP (c), SNP (g), SNP (i), and SNP (k);
the combination of SNP (b), SNP (c), SNP (g), SNP (i), and SNP (l);
the combination of SNP (b), SNP (c), SNP (g), SNP (j), and SNP (k);
the combination of SNP (b), SNP (c), SNP (g), SNP (j), and SNP (l);
the combination of SNP (b), SNP (c), SNP (g), SNP (k), and SNP (l);
the combination of SNP (b), SNP (c), SNP (h), SNP (i), and SNP (j);
the combination of SNP (b), SNP (c), SNP (h), SNP (i), and SNP (k);
the combination of SNP (b), SNP (c), SNP (h), SNP (i), and SNP (l);
the combination of SNP (b), SNP (c), SNP (h), SNP (j), and SNP (k);
the combination of SNP (b), SNP (c), SNP (h), SNP (j), and SNP (l);
the combination of SNP (b), SNP (c), SNP (h), SNP (k), and SNP (l);
the combination of SNP (b), SNP (c), SNP (i), SNP (j), and SNP (k);
the combination of SNP (b), SNP (c), SNP (i), SNP (j), and SNP (l);
the combination of SNP (b), SNP (c), SNP (i), SNP (k), and SNP (l);
the combination of SNP (b), SNP (c), SNP (j), SNP (k), and SNP (l);
the combination of SNP (b), SNP (f), SNP (g), SNP (h), and SNP (i);
the combination of SNP (b), SNP (f), SNP (g), SNP (h), and SNP (j);
the combination of SNP (b), SNP (f), SNP (g), SNP (h), and SNP (k);
the combination of SNP (b), SNP (f), SNP (g), SNP (h), and SNP (l);
the combination of SNP (b), SNP (f), SNP (g), SNP (i), and SNP (j);
the combination of SNP (b), SNP (f), SNP (g), SNP (i), and SNP (k);
the combination of SNP (b), SNP (f), SNP (g), SNP (i), and SNP (l);
the combination of SNP (b), SNP (f), SNP (g), SNP (j), and SNP (k);
the combination of SNP (b), SNP (f), SNP (g), SNP (j), and SNP (l);
the combination of SNP (b), SNP (f), SNP (g), SNP (k), and SNP (l);
the combination of SNP (b), SNP (f), SNP (h), SNP (i), and SNP (j);
the combination of SNP (b), SNP (f), SNP (h), SNP (i), and SNP (k);
the combination of SNP (b), SNP (f), SNP (h), SNP (i), and SNP (l);
the combination of SNP (b), SNP (f), SNP (h), SNP (j), and SNP (k);
the combination of SNP (b), SNP (f), SNP (h), SNP (j), and SNP (l);
the combination of SNP (b), SNP (f), SNP (h), SNP (k), and SNP (l);
the combination of SNP (b), SNP (f), SNP (i), SNP (j), and SNP (k);
the combination of SNP (b), SNP (f), SNP (i), SNP (j), and SNP (l);
the combination of SNP (b), SNP (f), SNP (i), SNP (k), and SNP (l);
the combination of SNP (b), SNP (f), SNP (j), SNP (k), and SNP (l);
the combination of SNP (b), SNP (g), SNP (h), SNP (i), and SNP (j);
the combination of SNP (b), SNP (g), SNP (h), SNP (i), and SNP (k);
the combination of SNP (b), SNP (g), SNP (h), SNP (i), and SNP (l);
the combination of SNP (b), SNP (g), SNP (h), SNP (j), and SNP (k);
the combination of SNP (b), SNP (g), SNP (h), SNP (j), and SNP (l);
the combination of SNP (b), SNP (g), SNP (h), SNP (k), and SNP (l);
the combination of SNP (b), SNP (g), SNP (i), SNP (j), and SNP (k);
the combination of SNP (b), SNP (g), SNP (i), SNP (j), and SNP (l);
the combination of SNP (b), SNP (g), SNP (i), SNP (k), and SNP (l);
the combination of SNP (b), SNP (g), SNP (j), SNP (k), and SNP (l);
the combination of SNP (b), SNP (h), SNP (i), SNP (j), and SNP (k);
the combination of SNP (b), SNP (h), SNP (i), SNP (j), and SNP (l);
the combination of SNP (b), SNP (h), SNP (i), SNP (k), and SNP (l);
the combination of SNP (b), SNP (h), SNP (j), SNP (k), and SNP (l);
the combination of SNP (b), SNP (i), SNP (j), SNP (k), and SNP (l);
the combination of SNP (c), SNP (f), SNP (g), SNP (h), and SNP (i);
the combination of SNP (c), SNP (f), SNP (g), SNP (h), and SNP (j);
the combination of SNP (c), SNP (f), SNP (g), SNP (h), and SNP (k);
the combination of SNP (c), SNP (f), SNP (g), SNP (h), and SNP (l);
the combination of SNP (c), SNP (f), SNP (g), SNP (i), and SNP (j);
the combination of SNP (c), SNP (f), SNP (g), SNP (i), and SNP (k);
the combination of SNP (c), SNP (f), SNP (g), SNP (i), and SNP (l);
the combination of SNP (c), SNP (f), SNP (g), SNP (j), and SNP (k);
the combination of SNP (c), SNP (f), SNP (g), SNP (j), and SNP (l);
the combination of SNP (c), SNP (f), SNP (g), SNP (k), and SNP (l);
the combination of SNP (c), SNP (f), SNP (h), SNP (i), and SNP (j);
the combination of SNP (c), SNP (f), SNP (h), SNP (i), and SNP (k);
the combination of SNP (c), SNP (f), SNP (h), SNP (i), and SNP (l);
the combination of SNP (c), SNP (f), SNP (h), SNP (j), and SNP (k);
the combination of SNP (c), SNP (f), SNP (h), SNP (j), and SNP (l);
the combination of SNP (c), SNP (f), SNP (h), SNP (k), and SNP (l);
the combination of SNP (c), SNP (f), SNP (i), SNP (j), and SNP (k);
the combination of SNP (c), SNP (f), SNP (i), SNP (j), and SNP (l);
the combination of SNP (c), SNP (f), SNP (i), SNP (k), and SNP (l);
the combination of SNP (c), SNP (f), SNP (j), SNP (k), and SNP (l);
the combination of SNP (c), SNP (g), SNP (h), SNP (i), and SNP (j);
the combination of SNP (c), SNP (g), SNP (h), SNP (i), and SNP (k);
the combination of SNP (c), SNP (g), SNP (h), SNP (i), and SNP (l);
the combination of SNP (c), SNP (g), SNP (h), SNP (j), and SNP (k);
the combination of SNP (c), SNP (g), SNP (h), SNP (j), and SNP (l);
the combination of SNP (c), SNP (g), SNP (h), SNP (k), and SNP (l);
the combination of SNP (c), SNP (g), SNP (i), SNP (j), and SNP (k);
the combination of SNP (c), SNP (g), SNP (i), SNP (j), and SNP (l);
the combination of SNP (c), SNP (g), SNP (i), SNP (k), and SNP (l);
the combination of SNP (c), SNP (g), SNP (j), SNP (k), and SNP (l);
the combination of SNP (c), SNP (h), SNP (i), SNP (j), and SNP (k);
the combination of SNP (c), SNP (h), SNP (i), SNP (j), and SNP (l);
the combination of SNP (c), SNP (h), SNP (i), SNP (k), and SNP (l);
the combination of SNP (c), SNP (h), SNP (j), SNP (k), and SNP (l);
the combination of SNP (c), SNP (i), SNP (j), SNP (k), and SNP (l);
the combination of SNP (f), SNP (g), SNP (h), SNP (i), and SNP (j);
the combination of SNP (f), SNP (g), SNP (h), SNP (i), and SNP (k);
the combination of SNP (f), SNP (g), SNP (h), SNP (i), and SNP (l);
the combination of SNP (f), SNP (g), SNP (h), SNP (j), and SNP (k);
the combination of SNP (f), SNP (g), SNP (h), SNP (j), and SNP (l);
the combination of SNP (f), SNP (g), SNP (h), SNP (k), and SNP (l);
the combination of SNP (f), SNP (g), SNP (i), SNP (j), and SNP (k);
the combination of SNP (f), SNP (g), SNP (i), SNP (j), and SNP (l);
the combination of SNP (f), SNP (g), SNP (i), SNP (k), and SNP (l);
the combination of SNP (f), SNP (g), SNP (j), SNP (k), and SNP (l);
the combination of SNP (f), SNP (h), SNP (i), SNP (j), and SNP (k);
the combination of SNP (f), SNP (h), SNP (i), SNP (j), and SNP (l);
the combination of SNP (f), SNP (h), SNP (i), SNP (k), and SNP (l);
the combination of SNP (f), SNP (h), SNP (j), SNP (k), and SNP (l);
the combination of SNP (f), SNP (i), SNP (j), SNP (k), and SNP (l);
the combination of SNP (g), SNP (h), SNP (i), SNP (j), and SNP (k);
the combination of SNP (g), SNP (h), SNP (i), SNP (j), and SNP (l);
the combination of SNP (g), SNP (h), SNP (i), SNP (k), and SNP (l);
the combination of SNP (g), SNP (h), SNP (j), SNP (k), and SNP (l);
the combination of SNP (g), SNP (i), SNP (j), SNP (k), and SNP (l);
the combination of SNP (h), SNP (i), SNP (j), SNP (k), and SNP (l);
the combination of SNP (b), SNP (c), SNP (f), SNP (g), SNP (h), and SNP (i);
the combination of SNP (b), SNP (c), SNP (f), SNP (g), SNP (h), and SNP (j);
the combination of SNP (b), SNP (c), SNP (f), SNP (g), SNP (h), and SNP (k);
the combination of SNP (b), SNP (c), SNP (f), SNP (g), SNP (h), and SNP (l);
the combination of SNP (b), SNP (c), SNP (f), SNP (g), SNP (i), and SNP (j);
the combination of SNP (b), SNP (c), SNP (f), SNP (g), SNP (i), and SNP (k);
the combination of SNP (b), SNP (c), SNP (f), SNP (g), SNP (i), and SNP (l);
the combination of SNP (b), SNP (c), SNP (f), SNP (g), SNP (j), and SNP (k);
the combination of SNP (b), SNP (c), SNP (f), SNP (g), SNP (j), and SNP (l);
the combination of SNP (b), SNP (c), SNP (f), SNP (g), SNP (k), and SNP (l);
the combination of SNP (b), SNP (c), SNP (f), SNP (h), SNP (i), and SNP (j);
the combination of SNP (b), SNP (c), SNP (f), SNP (h), SNP (i), and SNP (k);
the combination of SNP (b), SNP (c), SNP (f), SNP (h), SNP (i), and SNP (l);
the combination of SNP (b), SNP (c), SNP (f), SNP (h), SNP (j), and SNP (k);
the combination of SNP (b), SNP (c), SNP (f), SNP (h), SNP (j), and SNP (l);
the combination of SNP (b), SNP (c), SNP (f), SNP (h), SNP (k), and SNP (l);
the combination of SNP (b), SNP (c), SNP (f), SNP (i), SNP (j), and SNP (k);
the combination of SNP (b), SNP (c), SNP (f), SNP (i), SNP (j), and SNP (l);
the combination of SNP (b), SNP (c), SNP (f), SNP (i), SNP (k), and SNP (l);
the combination of SNP (b), SNP (c), SNP (f), SNP (j), SNP (k), and SNP (l);
the combination of SNP (b), SNP (c), SNP (g), SNP (h), SNP (i), and SNP (j);
the combination of SNP (b), SNP (c), SNP (g), SNP (h), SNP (i), and SNP (k);
the combination of SNP (b), SNP (c), SNP (g), SNP (h), SNP (i), and SNP (l);
the combination of SNP (b), SNP (c), SNP (g), SNP (h), SNP (j), and SNP (k);
the combination of SNP (b), SNP (c), SNP (g), SNP (h), SNP (j), and SNP (l);
the combination of SNP (b), SNP (c), SNP (g), SNP (h), SNP (k), and SNP (l);
the combination of SNP (b), SNP (c), SNP (g), SNP (i), SNP (j), and SNP (k);
the combination of SNP (b), SNP (c), SNP (g), SNP (i), SNP (j), and SNP (l);
the combination of SNP (b), SNP (c), SNP (g), SNP (i), SNP (k), and SNP (l);
the combination of SNP (b), SNP (c), SNP (g), SNP (j), SNP (k), and SNP (l);
the combination of SNP (b), SNP (c), SNP (h), SNP (i), SNP (j), and SNP (k);
the combination of SNP (b), SNP (c), SNP (h), SNP (i), SNP (j), and SNP (l);
the combination of SNP (b), SNP (c), SNP (h), SNP (i), SNP (k), and SNP (l);
the combination of SNP (b), SNP (c), SNP (h), SNP (j), SNP (k), and SNP (l);
the combination of SNP (b), SNP (c), SNP (i), SNP (j), SNP (k), and SNP (l);
the combination of SNP (b), SNP (f), SNP (g), SNP (h), SNP (i), and SNP (j);
the combination of SNP (b), SNP (f), SNP (g), SNP (h), SNP (i), and SNP (k);
the combination of SNP (b), SNP (f), SNP (g), SNP (h), SNP (i), and SNP (l);
the combination of SNP (b), SNP (f), SNP (g), SNP (h), SNP (j), and SNP (k);
the combination of SNP (b), SNP (f), SNP (g), SNP (h), SNP (j), and SNP (l);
the combination of SNP (b), SNP (f), SNP (g), SNP (h), SNP (k), and SNP (l);
the combination of SNP (b), SNP (f), SNP (g), SNP (i), SNP (j), and SNP (k);
the combination of SNP (b), SNP (f), SNP (g), SNP (i), SNP (j), and SNP (l);
the combination of SNP (b), SNP (f), SNP (g), SNP (i), SNP (k), and SNP (l);
the combination of SNP (b), SNP (f), SNP (g), SNP (j), SNP (k), and SNP (l);
the combination of SNP (b), SNP (f), SNP (h), SNP (i), SNP (j), and SNP (k);
the combination of SNP (b), SNP (f), SNP (h), SNP (i), SNP (j), and SNP (l);
the combination of SNP (b), SNP (f), SNP (h), SNP (i), SNP (k), and SNP (l);
the combination of SNP (b), SNP (f), SNP (h), SNP (j), SNP (k), and SNP (l);
the combination of SNP (b), SNP (f), SNP (i), SNP (j), SNP (k), and SNP (l);
the combination of SNP (b), SNP (g), SNP (h), SNP (i), SNP (j), and SNP (k);
the combination of SNP (b), SNP (g), SNP (h), SNP (i), SNP (j), and SNP (l);
the combination of SNP (b), SNP (g), SNP (h), SNP (i), SNP (k), and SNP (l);
the combination of SNP (b), SNP (g), SNP (h), SNP (j), SNP (k), and SNP (l);
the combination of SNP (b), SNP (g), SNP (i), SNP (j), SNP (k), and SNP (l);
the combination of SNP (b), SNP (h), SNP (i), SNP (j), SNP (k), and SNP (l);
the combination of SNP (c), SNP (f), SNP (g), SNP (h), SNP (i), and SNP (j);
the combination of SNP (c), SNP (f), SNP (g), SNP (h), SNP (i), and SNP (k);
the combination of SNP (c), SNP (f), SNP (g), SNP (h), SNP (i), and SNP (l);
the combination of SNP (c), SNP (f), SNP (g), SNP (h), SNP (j), and SNP (k);
the combination of SNP (c), SNP (f), SNP (g), SNP (h), SNP (j), and SNP (l);
the combination of SNP (c), SNP (f), SNP (g), SNP (h), SNP (k), and SNP (l);
the combination of SNP (c), SNP (f), SNP (g), SNP (i), SNP (j), and SNP (k);
the combination of SNP (c), SNP (f), SNP (g), SNP (i), SNP (j), and SNP (l);
the combination of SNP (c), SNP (f), SNP (g), SNP (i), SNP (k), and SNP (l);
the combination of SNP (c), SNP (f), SNP (g), SNP (j), SNP (k), and SNP (l);
the combination of SNP (c), SNP (f), SNP (h), SNP (i), SNP (j), and SNP (k);
the combination of SNP (c), SNP (f), SNP (h), SNP (i), SNP (j), and SNP (l);
the combination of SNP (c), SNP (f), SNP (h), SNP (i), SNP (k), and SNP (l);
the combination of SNP (c), SNP (f), SNP (h), SNP (j), SNP (k), and SNP (l);
the combination of SNP (c), SNP (f), SNP (i), SNP (j), SNP (k), and SNP (l);
the combination of SNP (c), SNP (g), SNP (h), SNP (i), SNP (j), and SNP (k);
the combination of SNP (c), SNP (g), SNP (h), SNP (i), SNP (j), and SNP (l);
the combination of SNP (c), SNP (g), SNP (h), SNP (i), SNP (k), and SNP (l);
the combination of SNP (c), SNP (g), SNP (h), SNP (j), SNP (k), and SNP (l);
the combination of SNP (c), SNP (g), SNP (i), SNP (j), SNP (k), and SNP (l);
the combination of SNP (c), SNP (h), SNP (i), SNP (j), SNP (k), and SNP (l);
the combination of SNP (f), SNP (g), SNP (h), SNP (i), SNP (j), and SNP (k);
the combination of SNP (f), SNP (g), SNP (h), SNP (i), SNP (j), and SNP (l);
the combination of SNP (f), SNP (g), SNP (h), SNP (i), SNP (k), and SNP (l);
the combination of SNP (f), SNP (g), SNP (h), SNP (j), SNP (k), and SNP (l);
the combination of SNP (f), SNP (g), SNP (i), SNP (j), SNP (k), and SNP (l);
the combination of SNP (f), SNP (h), SNP (i), SNP (j), SNP (k), and SNP (l);
the combination of SNP (g), SNP (h), SNP (i), SNP (j), SNP (k), and SNP (l);
the combination of SNP (b), SNP (c), SNP (f), SNP (g), SNP (h), SNP (i), and SNP (j);
the combination of SNP (b), SNP (c), SNP (f), SNP (g), SNP (h), SNP (i), and SNP (k);
the combination of SNP (b), SNP (c), SNP (f), SNP (g), SNP (h), SNP (i), and SNP (l);
the combination of SNP (b), SNP (c), SNP (f), SNP (g), SNP (h), SNP (j), and SNP (k);
the combination of SNP (b), SNP (c), SNP (f), SNP (g), SNP (h), SNP (j), and SNP (l);
the combination of SNP (b), SNP (c), SNP (f), SNP (g), SNP (h), SNP (k), and SNP (l);
the combination of SNP (b), SNP (c), SNP (f), SNP (g), SNP (i), SNP (j), and SNP (k);
the combination of SNP (b), SNP (c), SNP (f), SNP (g), SNP (i), SNP (j), and SNP (l);
the combination of SNP (b), SNP (c), SNP (f), SNP (g), SNP (i), SNP (k), and SNP (l);
the combination of SNP (b), SNP (c), SNP (f), SNP (g), SNP (j), SNP (k), and SNP (l);
the combination of SNP (b), SNP (c), SNP (f), SNP (h), SNP (i), SNP (j), and SNP (k);
the combination of SNP (b), SNP (c), SNP (f), SNP (h), SNP (i), SNP (j), and SNP (l);
the combination of SNP (b), SNP (c), SNP (f), SNP (h), SNP (i), SNP (k), and SNP (l);
the combination of SNP (b), SNP (c), SNP (f), SNP (h), SNP (j), SNP (k), and SNP (l);
the combination of SNP (b), SNP (c), SNP (f), SNP (i), SNP (j), SNP (k), and SNP (l);
the combination of SNP (b), SNP (c), SNP (g), SNP (h), SNP (i), SNP (j), and SNP (k);
the combination of SNP (b), SNP (c), SNP (g), SNP (h), SNP (i), SNP (j), and SNP (l);
the combination of SNP (b), SNP (c), SNP (g), SNP (h), SNP (i), SNP (k), and SNP (l);
the combination of SNP (b), SNP (c), SNP (g), SNP (h), SNP (j), SNP (k), and SNP (l);
the combination of SNP (b), SNP (c), SNP (g), SNP (i), SNP (j), SNP (k), and SNP (l);
the combination of SNP (b), SNP (c), SNP (h), SNP (i), SNP (j), SNP (k), and SNP (l);
the combination of SNP (b), SNP (f), SNP (g), SNP (h), SNP (i), SNP (j), and SNP (k);
the combination of SNP (b), SNP (f), SNP (g), SNP (h), SNP (i), SNP (j), and SNP (l);
the combination of SNP (b), SNP (f), SNP (g), SNP (h), SNP (i), SNP (k), and SNP (l);
the combination of SNP (b), SNP (f), SNP (g), SNP (h), SNP (j), SNP (k), and SNP (l);
the combination of SNP (b), SNP (f), SNP (g), SNP (i), SNP (j), SNP (k), and SNP (l);
the combination of SNP (b), SNP (f), SNP (h), SNP (i), SNP (j), SNP (k), and SNP (l);
the combination of SNP (b), SNP (g), SNP (h), SNP (i), SNP (j), SNP (k), and SNP (l);
the combination of SNP (c), SNP (f), SNP (g), SNP (h), SNP (i), SNP (j), and SNP (k);
the combination of SNP (c), SNP (f), SNP (g), SNP (h), SNP (i), SNP (j), and SNP (l);
the combination of SNP (c), SNP (f), SNP (g), SNP (h), SNP (i), SNP (k), and SNP (l);
the combination of SNP (c), SNP (f), SNP (g), SNP (h), SNP (j), SNP (k), and SNP (l);
the combination of SNP (c), SNP (f), SNP (g), SNP (i), SNP (j), SNP (k), and SNP (l);
the combination of SNP (c), SNP (f), SNP (h), SNP (i), SNP (j), SNP (k), and SNP (l);
the combination of SNP (c), SNP (g), SNP (h), SNP (i), SNP (j), SNP (k), and SNP (l);
the combination of SNP (f), SNP (g), SNP (h), SNP (i), SNP (j), SNP (k), and SNP (l);
the combination of SNP (b), SNP (c), SNP (f), SNP (g), SNP (h), SNP (i), SNP (j), and SNP (k);
the combination of SNP (b), SNP (c), SNP (f), SNP (g), SNP (h), SNP (i), SNP (j), and SNP (l);
the combination of SNP (b), SNP (c), SNP (f), SNP (g), SNP (h), SNP (i), SNP (k), and SNP (l);
the combination of SNP (b), SNP (c), SNP (f), SNP (g), SNP (h), SNP (j), SNP (k), and SNP (l);
the combination of SNP (b), SNP (c), SNP (f), SNP (g), SNP (i), SNP (j), SNP (k), and SNP (l);
the combination of SNP (b), SNP (c), SNP (f), SNP (h), SNP (i), SNP (j), SNP (k), and SNP (l);
the combination of SNP (b), SNP (c), SNP (g), SNP (h), SNP (i), SNP (j), SNP (k), and SNP (l);
the combination of SNP (b), SNP (f), SNP (g), SNP (h), SNP (i), SNP (j), SNP (k), and SNP (l);
the combination of SNP (c), SNP (f), SNP (g), SNP (h), SNP (i), SNP (j), SNP (k), and SNP (l); and
the combination of SNP (b), SNP (c), SNP (f), SNP (g), SNP (h), SNP (i), SNP (j), SNP (k), and SNP (l).

Of these combinations, for example, the following combinations are preferable because they show higher correlation with the *Eustoma* fusarium wilt resistance:
the combination of SNP (f) and SNP (k);
the combination of SNP (a), SNP (b), and SNP (c);
the combination of SNP (a), SNP (b), SNP (c), and SNP (d); and
the combination of SNP (b), SNP (c), SNP (f), SNP (g), SNP (h), SNP (i), SNP (j), SNP (k), and SNP (l).

In the present disclosure, as the SNP marker(s) described in the above item (1), any one or more of SNPs 1 to 66, as described below, may be used, in addition to or instead of the above-described SNPs (a) to (d) and (f) to (l).In Table 1 and Tables 2A and 2B below, SNPs 1 to 66 each indicate a polymorphism in which the base [N₁/N₂] in brackets is N₂. That is, SNPs 1 to 66 indicate that a *Eustoma* plant is resistant to *Eustoma* fusarium wilt when the base in brackets is N₂ and is susceptible to *Eustoma* fusarium wilt when the base in brackets is other than N₂ (for example, N₁). The base sequences of SEQ ID Nos: 1 to 66 in Table 1 and Tables 2A and 2B can be obtained, for example, from the *Eustoma* plant deposited under Accession No. FERM BP-22427, as described below. In each SNP, the sign "-" means a deletion-type polymorphism.

**[Table 1]**

| SNP ID | SNP Marker | [SNP] and Flanking Sequences (5' → 3'), SNP [N1/N2]: N1 = Susceptible-type Polymorphism, N2 = Disease resistance-type Polymorphism | SEQ ID NO. |
|---|---|---|---|
| SNP 1 | Egra20_3656645 SNP (e) | CACAGGTGGTAGGATGGCCTCCAGTTCGATCGTTCCGAAAGAATTCAATGGCAAACAATCCTCACAAGGCTGATGAAGA AGCAGATGCAATGACTGGAAG[T/C]AGTCGTCTTTACGTCAAAGTTAGCATGGATGGTGCCCCTTATCTGAGGAAAGTTG ATATCAAGACATACAACAGCTACAAGGACCTGTCCTCTGCACTTG | 6 |
| SNP 2 | Egra20_3703734 SNP (a) | TTGACCAACACAAGGTGTCATTCTGCAAACAGGCTTTCGGTATAGCAGCAGTGAAACACTCAAACTGTACGTGTCCATG GGTTCAAATTTTTGATCTCTT[G/T]GAGCAAATCCCAACCCGAGTACTAACTGAGATATGTCCTGGGGACCGTAGCAAGA TATCTAAATGAATGTGCAGTCTATTCCTATTTTTAACTTACATAT | 7 |
| SNP 3 | Egra20_3759258 SNP (b) | GCTCAAATTTTTTCCAGCTATGGAATCAGCCCCAGAACAGGTTTCCTTTCTTTTTTCTTCCTGTTCAAAAGCATCCAATTT TATGCATTTTGGTACCTA[G/A]TTTGGTGAATTTTGATAAAGATTTGATCTTTATAATTTTTTATTGGGGCTTAGGATTCT GAGAAAGTCGGGTCTCTAAAGGAGGTGAAGATGAATGAAAC | 8 |
| SNP 4 | Egra20_3839851 SNP (c) | TTTACAGGAGCGTAACGAGAGGTTATTTTACAAGCTTCTAATTGACAATGTGGACGAACTCCTTCCTGTGGTGTACACTC CCACTGTTGGTGAGGCTTG[C/A]AGAAATATGGAAGCATTTTCAAGCGTCCTCAGGGTCTCTTCATCAGTTTGAAAGAA AGGTTCTGTCATTTGTTTTTAATTTGTCTTAAAACGACTACTGT | 9 |
| SNP 5 | Egra20_3961378 SNP (d) | TGAAGTTTGGAGAGTCTCTAGGGCTATTAAACATGCATCATTAGTACTTCATGGTGATTTCACATGAAGGTGCTT[A/G]C AGAAAGAATTCACAGGAATGCTTTTTTGTTGTATATCAAAATATTCGTATTGAATGTCTTTTTTTTTTTCTGGC | 10 |

**[Table 2A]**

| SNP ID | SNP Marker | [SNP] and Flanking Sequences (5' → 3'), SNP [N1/N2]: N1 = Susceptible-type Polymorphism, N2 = Disease resistance-type Polymorphism | SEQ ID NO. |
|---|---|---|---|
| SNP 6 | Egra20_3710974 | AAAAAGTGAGGATTTTTTTTGGGGTTGTGTTTGACAAAGGGTTCTTAAC[T/G]GCAAGAAAATGTATTTTCTGTTGCTTT GACAGAATTATTTCCGACAATT | 11 |
| SNP 7 | Egra20_3713669 | AGACGCCGTTGTCTGTCTCCGCTTGACATGCCCAAACCATCTCAGTTTCC[A/T]ATTTCTTATCTTATCCGCTATACTAATC ACCCCTAGCCTTTTCCGAAATA | 12 |
| SNP 8 | Egra20_3743241 | TTTCTGCAGTATGTGAAAATGGGTATCCGAAATGGACGTTCTAGATAAAA[T/A]ATTTACATTGGCATGAAAAAGCAAA AGTAAGCAAAACATAATTATTCAT | 13 |
| SNP 9 | Egra20_3759258 | TTTTTTCTTCCTGTTCAAAAGCATCCAATTTTATGCATTTTGGTACCTA[G/A]TTTGGTGAATTTTGATAAAGATTTGATC TTTATAATTTTTTATTGGGGCT | 14 |
| SNP 10 | Egra20_3773671 | ATCAAAAATCAGATAAAAAAGGATTAGATTTAATTTTTAAACATGTAG[T/G]TTTCAATTTTAGTTTTTAATCTTATTA ATTAATAATATTGTTGTTAAGAT | 15 |
| SNP 11 | Egra20_3820383 | TATTCCGTTGACTGGTGTTGACTAACAGACGATTAATTATGGTTTATCAC[C/A]CTCAATTAACACCAAACTCTATTTGTC TGATCTGACGGAATAACAATACC | 16 |
| SNP 12 | Egra20_3826673 | GTTGTTGTTGTTCTATTGTGTCTCTAGTTACTCTTTACTTATTCTCAAAG[T/C]AACATAAAACCTCCCAGTAACCAAATTG TCATTTAGATCATCATCATCAT | 17 |
| SNP 13 | Egra20_3833476 | ATGTCACTTCTAGAACTGATTTGTGCCTTTTATTTGTGTGGTTTTTGGTT[G/A]GATCATCTGTATGAGTACGCAATATTTT GGTTTAATGTTGATCTTAATCA | 18 |
| SNP 14 | Egra20_3839851 | TGGACGAACTCCTTCCTGTGGTGTACACTCCCACTGTTGGTGAGGCTTG[C/A]AGAAATATGGAAGCATTTTCAAGCGT CCTCAGGGTCTCTTCATCAGTTTG | 19 |
| SNP 15 | Egra20_3841316 | TTCTGAAGTTGTTTATGAATGGATCTGAGAGATCATACTGGTTGTTCAAT[T/C]AATTGAAGGCAGCTGTGGTTGATTGCT GTGGTCAATGAAACATGGAAAAA | 20 |
| SNP 16 | Egra20_3841463 | TGGTTCAGAAGTTTGCATGAAATGACACTAACTGATTGAATTTAAACAGT[G/T]TAGCAGCTTGTTAGGACAGTGTGTTC AAATTTTTCATAAGGGTACCTCTC | 21 |
| SNP 17 | Egra20_3849059 | TTTGTTGCAGATAATTACCATCTGAAGAGGATGCAGAAACAAGAATAATC[G/T]GTGTAAAATCCCAAGAATCATATTCA GGAACCTCGTTCAACCAAATAACA | 22 |
| SNP 18 | Egra20_3851 104 | TCAGATATACGCAGTCATACCCATGTGTTAGAAACATACAAAGGTTGTTC[C/T]CGATGACCATAATGAAAATTGTGTCT GTCGATCTGTAGCGCCCCGCTCCC | 23 |
| SNP 19 | Egra20_3854638 | GAATACAATTTTTACAATATTCAGCCTCCAAATTCCATCCTTCCATTTTA[C/T]AATTGTTCTTACATATTCCACAATGTGT GATCTTCTAAATGTTCTTTAAA | 24 |
| SNP 20 | Egra20_3854800 | GTGCAGAAGAAAAAAAAGAAGAAGAAGAAGCTGCGAAGAGAATATG[A/T]CTGGCCAAAATATTTCTCTTCTAT TGAAAACTGTTTTAGAAAAGACTC | 25 |
| SNP 21 | Egra20_3854923 | GCTAAAACACTTTCTTCAAAACTCTTCACTCTTCGTCTTGATACCATTTT[T/G]TAAAAATGTTTTTCATCTCTTGGCTGTG AAGAGTCATTGCCTTTGGCAGG | 26 |
| SNP 22 | Egra20_3855240 | ACTACTTTAAAACTTTGTTTCCAACAGGCAGTGAAGAGCCTTAGTCTTGT[G/C]CTGAAATCTTCTAACCTTCAAATCTTT TTCAATAGACTGTGAAGGGCCTT | 27 |
| SNP 23 | Egra20_3856538 | TGAACTCAACCTGATGTCATTAAAGGCTTCTTGTTTCAACTCATACTG[C/T]TCAAACATACAAACTTTTCTAAAACTA TGCCATTGAATCTCCTAATATAT | 28 |
| SNP 24 | Egra20_3857016 | ACACAACAGGTGCAGTGCTCAACTCAGCCAAACTACTATAAGTATTGACC[G/A]AGCTGAGCTGACCACAAGTGTACAA CTACGTACTGCTGATACCCTTTCAT | 29 |
| SNP 25 | Egra20_3859796 | CATGTGGAAAATGGTCAATGATTGTGAGCTGATTGACCTGGGGTACTCTG[A/G]TCCAGCATTCACTTGGACCAATCTCA GGGATGACGAGGACAATATCATGG | 30 |
| SNP 26 | Egra20_3860514 | TACACTACACAAATCACAACTCCCTGCCCCCCACACATGATCTCATTCTC[G/A]CCACTTACAGAGGGGGACCTCACGAG CCTGAATAGGGAGGTGATCCCTCA | 31 |
| SNP 27 | Egra20_3862588 | CCATTC[C/T]GCCAAAACATATCTTAATTGCCAGGATTTTGAAGGAAACAGGC[G/A]AAATTTTGAAGGTTCTGGAGAAG GAAAACGAAAGGAAAGAGGTGGAATGG | 32 |
| SNP 28 | Egra20_3877909 | ATT[T/A]AC[T/C]T[C/G]ATCCGTTACAGTCAATTATATAA[C/T]CTTTACAG[A/G][A/G]ACGCAAGTTGCTTATGGTTCATA TTAGCCACTCGGGTCCTAATATACAG T | 33 |
| SNP 29 | Egra20_3878239 | CCATTGGGATGGACTGCTAGGGAGATAACTTTGTTCTGCTTGAATGTGCT[T/G]TAAGGTCAATTGGTAGAGGCAAATGT ATTCTGCTGCTTTAGGTAAATACC | 34 |
| SNP 30 | Egra20_3878308 | GCAAATGTATTCTGCTGCTTTAGGTAAATACCGTATCCTGCCTT[C/T]ATTTT[A/G]TATCTTAAAAGTTGTTTGCTTTAAA GTTATCAATCATTATTACTCCATAA | 35 |
| SNP 31 | Egra20_3878364 | TAAAAGTTGTTTGCTTTAAAGTTATCAATCATTATTACTCCATAAGGAAA[A/T]GCAATTATTTCTTAGGCGAAATGCAAC TATTACTAGTAGCTTTTTGGTGG | 36 |
| SNP 32 | Egra20_3879248 | CAATTCTTCCTAATCTCGTTTTCTACTCTTTTTTCGCTTCACATCATTTT[T/C]TCTCATCATCTAACACCTGTTGTTACTAG GGGGAAGATTCCAGATACATA | 37 |
| SNP 33 | Egra20_3879982 | TGATACCGGTATGTTTCGAAGGTGGAGTGAGGATGCTGATTACCTGCTAG[A/G]ATCCGGGTTTCAGCAAATAAATAGTT TCTTACCTGCGGTTGCGAGTATCA | 38 |
| SNP 34 | Egra20_3880604 | ACAATCATTTTGGCGATATTGAACGTTGCAGTTTATAAATTGAAGCAAAT[T/A]TGCAAATTCACATTCTGGCAGGAGAA AGATATGCAATCAGCTCCAGATAA | 39 |
| SNP 35 | Egra20_3880790 | TTCGGTAAAGTATACAGAGGTTTTCTTTACGGGCGTCCAGAAATTCTTGC[T/C]ATAAAGCGATTGAAGCCCGACTCCAA ACAAGGGGCACATGAATTTTGGCG | 40 |
| SNP 36 | Egra20_3881177 | GAGAATTTTGTTGCTAAGATTTCAGATTTTGGGCTATCAAAACTGGGTAG[A/G]ACAGGGGAAATCCTGAGCCAACTTAG CACAAAAATTAAAGGCACA[G/C]TTGG | 41 |
| SNP 37 | Egra20_3881246 | CAACTTAGCACAAAAATTAAAGGCACAGTTGGTTATCTGGATCCTGATTA[T/C]CTTATGACTCATAAACCTTCAACAAA AAG[T/C]GATATATATGCATTTGGCAT | 42 |

**[Table 2B]**

| SNP ID | SNP Marker | [SNP] and Flanking Sequences (5' → 3'), SNP [N1/N2]: N1 = Susceptible-type Polymorphism, N2 = Disease resistance-type Polymorphism | SEQ ID NO. |
|---|---|---|---|
| SNP 38 | Egra20_3881918 | CGTATCTTCTCTCTAAAAGAAATTAAATCAGCCACCAAAAACTTTAGC[A/G]A[C/T]AGTTTCCTTATTGGCCAGGGAGGA TACGGTAAAGTGTACAAAGGTTTCAT | 43 |
| SNP 39 | Egra20_3882045 | TCAAGAGATGGAGCTCTTACTCCACGCAGAGTGCTTCA[A/G]ATTTCCACACA[C/G]AAGTTCAATTACTACCCAAGCTCC AGTTCATTCATCTCGTATCTTTTGTG | 44 |
| SNP 40 | Egra20_3882198 | AAGGTTCACTTGATACTCATCTCTATGGAGGCACTGTAAACAGAAAGAAC[G/A]TTACGTCTCTCACTTGGGAGCAAAGA CTCATAATCTGCAC[A/T]GGTGCTGCT | 45 |
| SNP 41 | Egra20_3883102 | AGCAAGAGAAAATGCAGCTGCCACCCTTTTTAGCTTATCAATCTTCGATG[G/A]GAACAAGGTAG[T/C]TATAGGTGCAG CTGGGGCCATACCGCCCCTCGTTGATT | 46 |
| SNP 42 | Egra20_3913473 | GATCATTGTTCAAGGAGATGACCAACAAGTAGACCAGATGAGAAGAGAGC[A/T]CCAGCTTACTGAATAAGGAATGATT TATGTTAAAGGCATGTTTGAGAGAT | 47 |
| SNP 43 | Egra20_3913783 | GGCCTTCAGAAACAGAGAATCAGTGCTGCTATTAGGCATCGGTTTGGATC[G/A]AAAAGCAGAGGCAGCCATGCAGATA TTATGTTAATGGTAGCTATACGTTG | 48 |
| SNP 44 | Egra20_3914662 | GCTGCATCGAGATTTCAAGAATCAAGATCAAAGCACATCAATCCCCAATT[G/T]CCCTAACGTTCTGAGCAATTCTTCAA AATAAAAGTACTCCCCAGTCAACA | 49 |
| SNP 45 | Egra20_3915194 | ATCCCATCGCTTTTAGAAGAAAACAAAGTCCAAGCCCATTCATAAATAAC[A/G]ATTTAATGTATTTTGAAACTTGTTTT AGATCACT | 50 |
| SNP 46 | Egra20_3915247 | TTAATGTATTTTGAAACTTGTTTTAGATCACTTCGCCAACACGAAACCCA[T/C]ATTCCAGCCCACTTAATAAAAGATAA AGCCCACAACATTTGGGCCTTTTA | 51 |
| SNP 47 | Egra20_3915749 | GTCGTCTGATTGGACATGAAAAAAATTCGGCCATAAATTAGACCTGCCAA[T/C]AAAACACCCTTAATGATAATTACTTT CGTCAAGTCTCATA | 52 |
| SNP 48 | Egra20_3919102 | AAAAGTTCATGCTTAAAAGAATTTATAACCTATGAAACCACACGAACTTT[T/A]AGTACATTAGGGCAAATATTTGCTTT GCAGGCACCCCCTCCATCATTGCA | 53 |
| SNP 49 | Egra20_3919326 | ATTATAAAAACGAATAATCGTACAAAAATTAAAGTTGTCTATTTAATTAT[T/C]AACTTTTGTACCGCACTTTCAATTGCT TTACGTTGAAAGATGGAAAAGGT | 54 |
| SNP 50 | Egra20_3919723 | CACTGCTTAGCTAAATGCCAGTTTGCTGGGGGACACCTTGTGTGGGTCAC[C/T]GCATAAGTCATCACCCAACCTAGAAG GCCGCTGAGCTCCTGATTTATATC | 55 |
| SNP 51 | Egra20_3922048 | AACACTATTGTTTATTTTTTTTACATAGGTATACTAAGATAAGATTTGA[G/A]CTCTAATTTTACCAATATGGCGTTTTA AAACACTACTCTTTGTTTTTTT | 56 |
| SNP 52 | Egra20_3922401 | ACTTAAA[-/A]CAGTCTTAGTAAACATGCATCTATCAATAATAAATTCCATTAG[G/A]AGGTGTT[T/-]CTAAGTTTTGCCTT AAAAGTAAAATCTACTTTGGAGGGCAAT | 57 |
| SNP 53 | Egra20_3922452 | AGGTGTT[T/-]CTAAGTTTGCCTTAAAAGTAAAATCTACTTTGGAGGGCAAT[A/G]GTGTCCTCAAAAGATTCTAAGAAG GAGTGATGGACTTTTTGGTGGGGAAA | 58 |
| SNP 54 | Egra20_3922509 | TCAAAAGATTCTAAGAAGGAGTGATGGACTTTTGGTGGGAAATTACCC[A/C]ATCGGAAAACAAACGTG[T/G]GTTTC TAACACATG[G/C][G/A]TAT[T/G]ATTAC[A/G]TA[C/T]ATC | 59 |
| SNP 55 | Egra20_3939402 | TTATTCTTTATTTTGTGCAAGAGGTTGTTTCCACGGCTCGAACTCCTAA[C/A]TCAAAGTCACAAAACAGCAACCGTACC AGTTGCTACAAGAACCGTCGTTC | 60 |
| SNP 56 | Egra20_3939771 | TATAGGGGCATCTAGAGTACACTTAGGCGTTTAGATGCTCTCTAGACTTT[A/T]TGTTGACTTTGCACTTTTTTGTACTTTT GTGTTTATGTTTTGGTCTTTAA | 61 |
| SNP 57 | Egra20_3940204 | TCTTATCCCGCATTTCGCCCCTTGGTTACTTTCAATGAAGAGACACCCAT[A/G]CGAACAAATTTTCTTTTCGTTTCTCA GTCTGGAAGCGATGTGCCAATT | 62 |
| SNP 58 | Egra20_3940277 | TTCTCAGTCTGGAAGCGATGTGCCAATTTTTTTTTCAATTAATTGATAAA[T/G]TGCTATCTAATTAAATCAAATATATAT ATATGAAGAAAAGAAAATATTTA | 63 |
| SNP 59 | Egra20_3943764 | AGTTCGGTTCGCTTAACACCTCTAACAATGTTATATTAAAAGTTGTAAAA[C/A]TACAATATTACAGCAAGCACCAGATG GGATCTACTACCAACCTTAGATAT | 64 |
| SNP 60 | Egra20_3957769 | ACATAAAAGTGAAAAGTGCAGGTTGGAAGGCTAGGAGCAGGATCAGATTA[T/C]GCAGCATTTCTACAACATGTTGGTG TTCCAGCTGTTGACATGGCTTTTGG | 65 |
| SNP 61 | Egra20_3957848 | CAGCTGTTGACATGGCTTTTGGAGAAGGTAATAGAACAGACTTGTCTACA[T/G]ATATTCCTTTTTCATACGGCATACCAT GAAAAGTGCATTGTAGTACAACA | 66 |
| SNP 62 | Egra20_3957996 | CACTTGTCCACCATAGACTGCTTCCTTCCTTTCTGTGAAATCCTTTTACC[T/C]CAGCACAGCTACAGGGCAGTCGCAAGG GAGAAGAAGAAACAAAAAAAGTC | 67 |
| SNP 63 | Egra20_3958538 | AAACTAATATTAGTCATAGATCGAGTAAAACAGAATATCTAACTACATAG[A/C]ACATAATCAACTAGGTAAACAGCTA CTAGCGACTGTAATTGGCAACTTCG | 68 |
| SNP 64 | Egra20_3959383 | TTTTCCTTTGAGTTTTCCTTATGGAAGAGTTATGATCATTATCTTTGTGT[T/C]TGAAACATTCACGATTAGCATTTAAACT AGATGACCCATAGTCTCTGTGG | 69 |
| SNP 65 | Egra20_3960416 | AATGCAGATTAAGGTACAGCTTGACTTGAGCTGTTCAGGTTTTAGTAAAA[C/T]TTTATCCTGTTTAGATTTTGATAAAGG AAACTGGTCAAGGCACTACAAGG | 70 |
| SNP 66 | Egra20_3960500 | GTCAAGGCACTACAAGGCAGCAAAGGCTGGGTATCAGCTTGGAGATATGA[G/A]GCAGGGAAAGTGAGAGAGGTGAAT GACAGACTAATGATGGCAGAAAGAGC | 71 |

The number of the above-described SNP markers present in the resistance locus is not limited to particular values. For example, the resistance locus may include any one of the SNP markers or two or more of the SNP markers, i.e., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, or 65 of the SNP markers or all of the 66 SNP markers. The correlation of these 66 types of polymorphisms (SNP markers) with *Eustoma* fusarium wilt resistance has not been reported heretofore. They are novel polymorphisms discovered first by the present inventors as being involved in *Eustoma* fusarium wilt resistance.

### (2) Identification by Base Sequence Including SNP Marker

The resistance locus may be specified by a base sequence including an SNP marker, as described in (2) above, for example. The resistance locus may consist of the base sequence or may include the base sequence, for example.

The base sequence including the SNP marker is not limited to particular base sequences, and examples thereof include polynucleotides (a), (b), (c), (d), (f), (g), (h), (i), (j), (k), and (l), as described below. The polynucleotides (a), (b), (c), (d), (f), (g), (h), (i), (j), (k), and (l) correspond to base sequences including, as the SNP marker, SNP (a), SNP (b), SNP (c), SNP (d), SNP (f), SNP (g), SNP (h), SNP (i), SNP (j), SNP (k), and SNP (l), respectively.

The polynucleotide (a) has a base sequence including SNP (a), i.e., Egra20_3703734, and is, for example, a polynucleotide (a1), (a2), or (a3), as described below. The polynucleotides (a2) and (a3) are polynucleotides each having a function equivalent to that of the polynucleotide (a1) regarding *Eustoma* fusarium wilt resistance in the resistance locus. The equivalent function means, for example, that a *Eustoma* plant having the *Eustoma* fusarium wilt resistance locus identified by the polynucleotide (a2) or (a3) exhibits *Eustoma* fusarium wilt resistance.
(a) A polynucleotide (a1), (a2), or (a3) below:
   (a1) a polynucleotide that consists of a base sequence of SEQ ID NO: 1;
   (a2) a polynucleotide that consists of a base sequence corresponding to the base sequence of the polynucleotide (a1) in which one or several bases are deleted, substituted, inserted, and/or added with a 101st base (T) being conserved and has a function equivalent to that of the polynucleotide (a1) regarding *Eustoma* fusarium wilt resistance in the *Eustoma* fusarium wilt resistance locus; or
   (a3) a polynucleotide that consists of a base sequence having at least 80% sequence identity to the base sequence of the polynucleotide (a1) with the 101st base (T) of the polynucleotide (a1) being conserved and has a function equivalent to that of the polynucleotide (a1) regarding *Eustoma* fusarium wilt resistance in the *Eustoma* fusarium wilt resistance locus.

In the polynucleotide (a1), the underlined base (T) in brackets in SEQ ID NO: 1 is a base corresponding to the polymorphism of SNP (a). The polynucleotide (a1) can be obtained from the *Eustoma* plant deposited under Accession No. FERM BP-22427, as described below, for example.

In the polynucleotide (a2), the term "one or several" means, for example, 1 to 40, 1 to 35, 1 to 30, 1 to 25, 1 to 20, 1 to 18, 1 to 12, 1 to 8, 1 to 4, 1 to 3, or 1 or 2. In the present disclosure, the numerical range regarding the number of bases or the like discloses all of the positive integers falling within that range, for example. That is, for example, the description "one to five" is intended to disclose all of "one, two, three, four, and five" (the same applies hereinafter).

In the polynucleotide (a3), the sequence identity is, for example, at least 80%, at least 85%, at least 89%, at least 90%, at least 95%, at least 96%, at least 97%, at least 98%, or at least 99%. The sequence identity can be determined by aligning two base sequences (the same applies hereinafter). Specifically, the sequence identity can be calculated using, for example, analysis software such as BLAST or FASTA with default parameters (the same applies hereinafter).

The polynucleotide (b) has a base sequence including SNP (b), i.e., Egra20_3759258, and is, for example, a polynucleotide (b1), (b2), or (b3), as described below. The polynucleotides (b2) and (b3) are polynucleotides each having a function equivalent to that of the polynucleotide (b1) regarding *Eustoma* fusarium wilt resistance in the resistance locus. The equivalent function means, for example, that a *Eustoma* plant having the *Eustoma* fusarium wilt resistance locus identified by the polynucleotide (b2) or (b3) exhibits *Eustoma* fusarium wilt resistance.
(b) A polynucleotide (b1), (b2), or (b3) below:
(b1) a polynucleotide that consists of the base sequence of SEQ ID NO: 2;
(b2) a polynucleotide that consists of a base sequence corresponding to the base sequence of the polynucleotide (b1) in which one or several bases are deleted, substituted, inserted, and/or added with a 101st base (A) being conserved; or
(b3) a polynucleotide that consists of a base sequence having at least 80% sequence identity to the base sequence of the polynucleotide (b1) with the 101st base (A) in the polynucleotide (b1) being conserved.

In the polynucleotide (b1), the underlined base (A) in brackets in SEQ ID NO: 2 is a base corresponding to the polymorphism of SNP (b). The polynucleotide (b1) can be obtained from the *Eustoma* plant deposited under Accession No. FERM BP-22427, as described below, for example. The polynucleotide (b1) may be a polynucleotide that consists of a base sequence resulting from truncation of the base sequence of SEQ ID NO: 2. In this case, the polynucleotide (b1) may be a polynucleotide that consists of the base sequence of SEQ ID NO: 79, for example. The above descriptions regarding the polynucleotides (b2) and (b3) apply to this case by replacing the term "101st base (A)" with the term "51st base (A)."

In the polynucleotide (b2), the term "one or several" means, for example, 1 to 40, 1 to 35, 1 to 30, 1 to 25, 1 to 20, 1 to 18, 1 to 15, 1 to 12, 1 to 10, 1 to 9, 1 to 8, 1 to 5, 1 to 4, 1 to 3, or 1 or 2.

In the polynucleotide (b3), the sequence identity is, for example, at least 80%, at least 85%, at least 89%, at least 90%, at least 95%, at least 96%, at least 97%, at least 98%, or at least 99%.

The polynucleotide (c) is a base sequence including SNP (c), i.e., Egra20_383985, and is, for example, a polynucleotide (c1), (c2), or (c3), as described below. The polynucleotides (c2) and (c3) are polynucleotides each having a function equivalent to that of the polynucleotide (c1) regarding *Eustoma* fusarium wilt resistance in the resistance locus. The equivalent function means, for example, that a *Eustoma* plant having the *Eustoma* fusarium wilt resistance locus identified by the polynucleotide (c2) or (c3) exhibits *Eustoma* fusarium wilt resistance.
(c) A polynucleotide (c1), (c2), or (c3) below:
(c1) a polynucleotide that consists of the base sequence of SEQ ID NO: 3;
(c2) a polynucleotide that consists of a base sequence corresponding to the base sequence of the polynucleotide (c1) in which one or several bases are deleted, substituted, inserted, and/or added with a 101st base (A) being conserved; or
(c3) a polynucleotide that consists of a base sequence having at least 80% sequence identity to the base sequence of the polynucleotide (c1) with the 101st base (A) in the polynucleotide (c1) being conserved.

In the polynucleotide (c1), the underlined base (A) in brackets in SEQ ID NO: 3 is a base corresponding to the polymorphism of SNP (c). The polynucleotide (c1) can be obtained from the *Eustoma* plant deposited under Accession No. FERM BP-22427, as described below, for example. The polynucleotide (c1) may be a polynucleotide that consists of a base sequence resulting from truncation of the base sequence of SEQ ID NO: 3. In this case, the polynucleotide (c1) may be a polynucleotide that consists of the base sequence of SEQ ID NO: 80, for example. The above descriptions regarding the polynucleotides (c2) and (c3) apply to this case by replacing the term "101st base (A)" with the term "51st base (A)."

In the polynucleotide (c2), the term "one or several" means, for example, 1 to 40, 1 to 35, 1 to 30, 1 to 25, 1 to 20, 1 to 18, 1 to 12, 1 to 10, 1 to 8, 1 to 4, 1 to 3, or 1 or 2.

In the polynucleotide (c3), the sequence identity is, for example, at least 80%, at least 85%, at least 89%, at least 90%, at least 95%, at least 96%, at least 97%, at least 98%, or at least 99%.

The polynucleotide (d) is a base sequence including SNP (d), i.e., Egra20_3961378, and is, for example, a polynucleotide (d1), (d2), or (d3), as described below. The polynucleotides (d2) and (d3) are polynucleotides each having a function equivalent to that of the polynucleotide (d1) regarding *Eustoma* fusarium wilt resistance in the resistance locus. The equivalent function means, for example, that a *Eustoma* plant having the *Eustoma* fusarium wilt resistance locus identified by the polynucleotide (d2) or (d3) exhibits *Eustoma* fusarium wilt resistance.
(d) A polynucleotide (d1), (d2), or (d3) below:
(d1) a polynucleotide that consists of the base sequence of SEQ ID NO: 4;
(d2) a polynucleotide that consists of a base sequence corresponding to the base sequence of the polynucleotide (d1) in which one or several bases are deleted, substituted, inserted, and/or added with a 76th base (G) being conserved; or
(d3) a polynucleotide that consists of a base sequence having at least 80% sequence identity to the base sequence of the polynucleotide (d1) with the 76th base (G) in the base sequence of the polynucleotide (d1) being conserved.

In the polynucleotide (d1), the underlined base (G) in brackets in SEQ ID NO: 4 is a base corresponding to the polymorphism of SNP (d). The polynucleotide (d1) can be obtained from the *Eustoma* plant deposited under Accession No. FERM BP-22427, as described below, for example.

In the polynucleotide (d2), the term "one or several" means, for example, 1 to 30, 1 to 25, 1 to 20, 1 to 18, 1 to 15, 1 to 12, 1 to 8, 1 to 6, 1 to 4, 1 to 3, or 1 or 2.

In the polynucleotide (d3), the sequence identity is, for example, at least 80%, at least 85%, at least 89%, at least 90%, at least 95%, at least 96%, at least 97%, at least 98%, or at least 99%.

The polynucleotide (f) is a base sequence including SNP (f), i.e., Egra20_3833476, and is, for example, a polynucleotide (f1), (f2), or (f3), as described below. The polynucleotides (f2) and (f3) are polynucleotides each having a function equivalent to that of the polynucleotide (f1) regarding *Eustoma* fusarium wilt resistance in the resistance locus. The equivalent function means, for example, that a *Eustoma* plant having the *Eustoma* fusarium wilt resistance locus identified by the polynucleotide (f2) or (f3) exhibits *Eustoma* fusarium wilt resistance.
(f) A polynucleotide (f1), (f2), or (f3) below:
(f1) a polynucleotide that consists of the base sequence of SEQ ID NO: 72;
(f2) a polynucleotide that consists of a base sequence corresponding to the base sequence of the polynucleotide (f1) in which one or several bases are deleted, substituted, inserted, and/or added with a 51st base (A) being conserved; or
(f3) a polynucleotide that consists of a base sequence having at least 80% sequence identity to the base sequence of the polynucleotide (f1) with the 51st base (A) in the polynucleotide (f1) being conserved.

In the polynucleotide (f1), the underlined base (A) in brackets in SEQ ID NO: 72 is a base corresponding to the polymorphism of SNP (f). The polynucleotide (f1) can be obtained from the *Eustoma* plant deposited under Accession No. FERM BP-22427, as described below, for example.

In the polynucleotide (f2), the term "one or several" means, for example, 1 to 20, 1 to 15, 1 to 10, 1 to 9, 1 to 8, 1 to 7, 1 to 6, 1 to 5, 1 to 4, 1 to 3, or 1 or 2.

In the polynucleotide (f3), the sequence identity is, for example, at least 80%, at least 85%, at least 89%, at least 90%, at least 95%, at least 96%, at least 97%, at least 98%, or at least 99%.

The polynucleotide (g) is a base sequence including SNP (g), i.e., Egra20_3849059, and is, for example, a polynucleotide (g1), (g2), or (g3), as described below. The polynucleotides (g2) and (g3) are polynucleotides each having a function equivalent to that of the polynucleotide (g1) regarding *Eustoma* fusarium wilt resistance in the resistance locus. The equivalent function means, for example, that a *Eustoma* plant having the *Eustoma* fusarium wilt resistance locus identified by the polynucleotide (g2) or (g3) exhibits *Eustoma* fusarium wilt resistance.
(g) A polynucleotide (g1), (g2), or (g3) below:
(g1) a polynucleotide that consists of the base sequence of SEQ ID NO: 73;
(g2) a polynucleotide that consists of a base sequence corresponding to the base sequence of the polynucleotide (g1) in which one or several bases are deleted, substituted, inserted, and/or added with a 51st base (T) being conserved; or
(g3) a polynucleotide that consists of a base sequence having at least 80% sequence identity to the base sequence of the polynucleotide (g1) with the 51st base (T) in the polynucleotide (g1) being conserved.

In the polynucleotide (g1), the underlined base (T) in brackets in SEQ ID NO: 73 is a base corresponding to the polymorphism of SNP (g). The polynucleotide (g1) can be obtained from the *Eustoma* plant deposited under Accession No. FERM BP-22427, as described below, for example.

In the polynucleotide (g2), the term "one or several" means, for example, 1 to 20, 1 to 15, 1 to 10, 1 to 9, 1 to 8, 1 to 7, 1 to 6, 1 to 5, 1 to 4, 1 to 3, or 1 or 2.

In the polynucleotide (g3), the sequence identity is, for example, at least 80%, at least 85%, at least 89%, at least 90%, at least 95%, at least 96%, at least 97%, at least 98%, or at least 99%.

The polynucleotide (h) is a base sequence including SNP (h), i.e., Egra20_3854638, and is, for example, a polynucleotide (h1), (h2), or (h3), as described below. The polynucleotides (h2) and (h3) are polynucleotides each having a function equivalent to that of the polynucleotide (h1) regarding *Eustoma* fusarium wilt resistance in the resistance locus. The equivalent function means, for example, that a *Eustoma* plant having the *Eustoma* fusarium wilt resistance locus identified by the polynucleotide (h2) or (h3) exhibits *Eustoma* fusarium wilt resistance.
(h) A polynucleotide (h1), (h2), or (h3) below:
(h1) a polynucleotide that consists of the base sequence of SEQ ID NO: 74;
(h2) a polynucleotide that consists of a base sequence corresponding to the base sequence of the polynucleotide (h1) in which one or several bases are deleted, substituted, inserted, and/or added with a 51st base (T) being conserved; or
(h3) a polynucleotide that consists of a base sequence having at least 80% sequence identity to the base sequence of the polynucleotide (h1) with the 51st base (T) in the polynucleotide (h1) being conserved.

In the polynucleotide (h1), the underlined base (T) in brackets in SEQ ID NO: 74 is a base corresponding to the polymorphism of SNP (h). The polynucleotide (h1) can be obtained from the *Eustoma* plant deposited under Accession No. FERM BP-22427, as described below, for example.

In the polynucleotide (h2), the term "one or several" means, for example, 1 to 20, 1 to 15, 1 to 10, 1 to 9, 1 to 8, 1 to 7, 1 to 6, 1 to 5, 1 to 4, 1 to 3, or 1 or 2.

In the polynucleotide (h3), the sequence identity is, for example, at least 80%, at least 85%, at least 89%, at least 90%, at least 95%, at least 96%, at least 97%, at least 98%, or at least 99%.

The polynucleotide (i) is a base sequence including SNP (i), i.e., Egra20_3857016, and is, for example, a polynucleotide (i1), (i2), or (i3), as described below. The polynucleotides (i2) and (i3) are polynucleotides each having a function equivalent to that of the polynucleotide (i1) regarding *Eustoma* fusarium wilt resistance in the resistance locus. The equivalent function means, for example, that a *Eustoma* plant having the *Eustoma* fusarium wilt resistance locus identified by the polynucleotide (i2) or (i3) exhibits *Eustoma* fusarium wilt resistance.
(i) A polynucleotide (i1), (i2), or (i3) below:
(11) a polynucleotide that consists of the base sequence of SEQ ID NO: 75;
(i2) a polynucleotide that consists of a base sequence corresponding to the base sequence of the polynucleotide (i1) in which one or several bases are deleted, substituted, inserted, and/or added with a 51st base (A) being conserved; or
(i3) a polynucleotide that consists of a base sequence having at least 80% sequence identity to the base sequence of the polynucleotide (i1) with the 51st base (A) in the polynucleotide (i1) being conserved.

In the polynucleotide (i1), the underlined base (A) in brackets in SEQ ID NO: 75 is a base corresponding to the polymorphism of SNP (i). The polynucleotide (i1) can be obtained from the *Eustoma* plant deposited under Accession No. FERM BP-22427, as described below, for example.

In the polynucleotide (i2), the term "one or several" means, for example, 1 to 20, 1 to 15, 1 to 10, 1 to 9, 1 to 8, 1 to 7, 1 to 6, 1 to 5, 1 to 4, 1 to 3, or 1 or 2.

In the polynucleotide (i3), the sequence identity is, for example, at least 80%, at least 85%, at least 89%, at least 90%, at least 95%, at least 96%, at least 97%, at least 98%, or at least 99%.

The polynucleotide (j) is a base sequence including SNP (j), i.e., Egra20_3879982, and is, for example, a polynucleotide (j 1), (j2), or (j3), as described below. The polynucleotides (i2) and (i3) are polynucleotides each having a function equivalent to that of the polynucleotide (j 1) regarding *Eustoma* fusarium wilt resistance in the resistance locus. The equivalent function means, for example, that a *Eustoma* plant having the *Eustoma* fusarium wilt resistance locus identified by the polynucleotide (j2) or (j3) exhibits *Eustoma* fusarium wilt resistance.
(j) A polynucleotide (j 1), (j2) or (j3) below:
(j 1) a polynucleotide that consists of the base sequence of SEQ ID NO: 76;
(j2) a polynucleotide that consists of a base sequence corresponding to the base sequence of the polynucleotide (j 1) in which one or several bases are deleted, substituted, inserted, and/or added with a 51st base (G) being conserved; or
(j3) a polynucleotide that consists of a base sequence having at least 80% sequence identity to the base sequence of the polynucleotide (j 1) with the 51st base (G) in the base sequence of the polynucleotide (j 1) being conserved.

In the polynucleotide (j 1), the underlined base (G) in brackets in SEQ ID NO: 76 is a base corresponding to the polymorphism of SNP (j). The polynucleotide (j 1) can be obtained from the *Eustoma* plant deposited under Accession No. FERM BP-22427, as described below, for example.

In the polynucleotide (j2), the term "one or several" means, for example, 1 to 20, 1 to 15, 1 to 10, 1 to 9, 1 to 8, 1 to 7, 1 to 6, 1 to 5, 1 to 4, 1 to 3, or 1 or 2.

In the polynucleotide (j3), the sequence identity is, for example, at least 80%, at least 85%, at least 89%, at least 90%, at least 95%, at least 96%, at least 97%, at least 98%, or at least 99%.

The polynucleotide (k) is a base sequence including SNP (k), i.e., Egra20_3914662, and is, for example, a polynucleotide (k1), (k2), or (k3), as described below. The polynucleotides (k2) and (k3) are polynucleotides each having a function equivalent to that of the polynucleotide (k1) regarding *Eustoma* fusarium wilt resistance in the resistance locus. The equivalent function means, for example, that a *Eustoma* plant having the *Eustoma* fusarium wilt resistance locus identified by the polynucleotide (k2) or (k3) exhibits *Eustoma* fusarium wilt resistance.
(k) A polynucleotide (k1), (k2) or (k3) below:
(k1) a polynucleotide that consists of the base sequence of SEQ ID NO: 77;
(k2) a polynucleotide that consists of a base sequence corresponding to the base sequence of the polynucleotide (k1) in which one or several bases are deleted, substituted, inserted, and/or added with a 51st base (T) being conserved; or
(k3) a polynucleotide that consists of a base sequence having at least 80% sequence identity to the base sequence of the polynucleotide (k1) with the 51st base (T) in the polynucleotide (k1) being conserved.

In the polynucleotide (k1), the underlined base (T) in brackets in SEQ ID NO: 77 is a base corresponding to the polymorphism of SNP (k). The polynucleotide (k1) can be obtained from the *Eustoma* plant deposited under Accession No. FERM BP-22427, as described below, for example.

In the polynucleotide (j2), the term "one or several" means, for example, 1 to 20, 1 to 15, 1 to 10, 1 to 9, 1 to 8, 1 to 7, 1 to 6, 1 to 5, 1 to 4, 1 to 3, or 1 or 2.

In the polynucleotide (k3), the sequence identity is, for example, at least 80%, at least 85%, at least 89%, at least 90%, at least 95%, at least 96%, at least 97%, at least 98%, or at least 99%.

The polynucleotide (1) is a base sequence including SNP (l), i.e., Egra20_3939402, and is, for example, a polynucleotide (l1), (l2), or (l3), as described below. The polynucleotides (l2) and (l3) are polynucleotides each having a function equivalent to that of the polynucleotide (l1) regarding *Eustoma* fusarium wilt resistance in the resistance locus. The equivalent function means, for example, that a *Eustoma* plant having the *Eustoma* fusarium wilt resistance locus identified by the polynucleotide (12) or (l3) exhibits *Eustoma* fusarium wilt resistance.
(l) A polynucleotide (11), (12) or (l3) below:
(l1) a polynucleotide that consists of the base sequence of SEQ ID NO: 78;
(12) a polynucleotide that consists of a base sequence corresponding to the base sequence of the polynucleotide (l1) in which one or several bases are deleted, substituted, inserted, and/or added with a 51st base (A) being conserved; or
(l3) a polynucleotide that consists of a base sequence having at least 80% sequence identity to the base sequence of the polynucleotide (l1) with the 51st base (A) in the polynucleotide (l1) being conserved.

In the polynucleotide (11), the underlined base (A) in brackets in SEQ ID NO: 78 is a base corresponding to the polymorphism of SNP (l).The polynucleotide (l1) can be obtained from the *Eustoma* plant deposited under Accession No. FERM BP-22427, as described below, for example.

In the polynucleotide (l2), the term "one or several" means, for example, 1 to 20, 1 to 15, 1 to 10, 1 to 9, 1 to 8, 1 to 7, 1 to 6, 1 to 5, 1 to 4, 1 to 3, or 1 or 2.

In the polynucleotide (l3), the sequence identity is, for example, at least 80%, at least 85%, at least 89%, at least 90%, at least 95%, at least 96%, at least 97%, at least 98%, or at least 99%.

The number of base sequences respectively including the SNP markers in the resistance locus, i.e., the number of base sequences respectively including the SNP markers linked to the resistance locus, is not limited to particular values, and for example, the resistance locus may include any one of the polynucleotides (a), (b), (c), (d), (f), (g), (h), (i), (j), (k), and (l) or two or more of these polynucleotides, i.e., 2, 3, 4, 5, 6, 7, 8, 9, or 10 of these polynucleotides or all of the 11 polynucleotides.

The combination of the base sequences including the SNP markers is not limited to particular combinations, and examples thereof include the following combinations:
the combination of the polynucleotides (a) and (b);
the combination of the polynucleotides (a) and (c);
the combination of the polynucleotides (a) and (d);
the combination of the polynucleotides (b) and (c);
the combination of the polynucleotides (b) and (d);
the combination of the polynucleotides (b) and (f);
the combination of the polynucleotides (b) and (g);
the combination of the polynucleotides (b) and (h);
the combination of the polynucleotides (b) and (i);
the combination of the polynucleotides (b) and (j);
the combination of the polynucleotides (b) and (k);
the combination of the polynucleotides (b) and (l);
the combination of the polynucleotides (c) and (d);
the combination of the polynucleotides (c) and (f);
the combination of the polynucleotides (c) and (g);
the combination of the polynucleotides (c) and (h);
the combination of the polynucleotides (c) and (i);
the combination of the polynucleotides (c) and (j);
the combination of the polynucleotides (c) and (k);
the combination of the polynucleotides (c) and (l);
the combination of the polynucleotides (f) and (g);
the combination of the polynucleotides (f) and (h);
the combination of the polynucleotides (f) and (i);
the combination of the polynucleotides (f) and (j);
the combination of the polynucleotides (f) and (k);
the combination of the polynucleotides (f) and (l);
the combination of the polynucleotides (g) and (h);
the combination of the polynucleotides (g) and (i);
the combination of the polynucleotides (g) and (j);
the combination of the polynucleotides (g) and (k);
the combination of the polynucleotides (g) and (l);
the combination of the polynucleotides (h) and (i);
the combination of the polynucleotides (h) and (j);
the combination of the polynucleotides (h) and (k);
the combination of the polynucleotides (h) and (l);
the combination of the polynucleotides (i) and (j);
the combination of the polynucleotides (i) and (k);
the combination of the polynucleotides (i) and (l);
the combination of the polynucleotides (j) and (k);
the combination of the polynucleotides (j) and (l);
the combination of the polynucleotides (k) and (l);
the combination of the polynucleotides (a), (b), and (c);
the combination of the polynucleotides (a), (b), and (d);
the combination of the polynucleotides (a), (c), and (d);
the combination of the polynucleotides (b), (c), and (d);
the combination of the polynucleotides (b), (c), and (f);
the combination of the polynucleotides (b), (c), and (g);
the combination of the polynucleotides (b), (c), and (h);
the combination of the polynucleotides (b), (c), and (i);
the combination of the polynucleotides (b), (c), and (j);
the combination of the polynucleotides (b), (c), and (k);
the combination of the polynucleotides (b), (c), and (l);
the combination of the polynucleotides (b), (f), and (g);
the combination of the polynucleotides (b), (f), and (h);
the combination of the polynucleotides (b), (f), and (i);
the combination of the polynucleotides (b), (f), and (j);
the combination of the polynucleotides (b), (f), and (k);
the combination of the polynucleotides (b), (f), and (l);
the combination of the polynucleotides (b), (g), and (h);
the combination of the polynucleotides (b), (g), and (i);
the combination of the polynucleotides (b), (g), and (j);
the combination of the polynucleotides (b), (g), and (k);
the combination of the polynucleotides (b), (g), and (l);
the combination of the polynucleotides (b), (h), and (i);
the combination of the polynucleotides (b), (h), and (j);
the combination of the polynucleotides (b), (h), and (k);
the combination of the polynucleotides (b), (h), and (l);
the combination of the polynucleotides (b), (i), and (j);
the combination of the polynucleotides (b), (i), and (k);
the combination of the polynucleotides (b), (i), and (l);
the combination of the polynucleotides (b), (j), and (k);
the combination of the polynucleotides (b), (j), and (l);
the combination of the polynucleotides (b), (k), and (l);
the combination of the polynucleotides (c), (f), and (g);
the combination of the polynucleotides (c), (f), and (h);
the combination of the polynucleotides (c), (f), and (i);
the combination of the polynucleotides (c), (f), and (j);
the combination of the polynucleotides (c), (f), and (k);
the combination of the polynucleotides (c), (f), and (l);
the combination of the polynucleotides (c), (g), and (h);
the combination of the polynucleotides (c), (g), and (i);
the combination of the polynucleotides (c), (g), and (j);
the combination of the polynucleotides (c), (g), and (k);
the combination of the polynucleotides (c), (g), and (l);
the combination of the polynucleotides (c), (h), and (i);
the combination of the polynucleotides (c), (h), and (j);
the combination of the polynucleotides (c), (h), and (k);
the combination of the polynucleotides (c), (h), and (l);
the combination of the polynucleotides (c), (i), and (j);
the combination of the polynucleotides (c), (i), and (k);
the combination of the polynucleotides (c), (i), and (l);
the combination of the polynucleotides (c), (j), and (k);
the combination of the polynucleotides (c), (j), and (l);
the combination of the polynucleotides (c), (k), and (l);
the combination of the polynucleotides (f), (g), and (h);
the combination of the polynucleotides (f), (g), and (i);
the combination of the polynucleotides (f), (g), and (j);
the combination of the polynucleotides (f), (g), and (k);
the combination of the polynucleotides (f), (g), and (l);
the combination of the polynucleotides (f), (h), and (i);
the combination of the polynucleotides (f), (h), and (j);
the combination of the polynucleotides (f), (h), and (k);
the combination of the polynucleotides (f), (h), and (l);
the combination of the polynucleotides (f), (i), and (j);
the combination of the polynucleotides (f), (i), and (k);
the combination of the polynucleotides (f), (k), and (l);
the combination of the polynucleotides (f), (i), and (l);
the combination of the polynucleotides (f), (j), and (k);
the combination of the polynucleotides (f), (j), and (l);
the combination of the polynucleotides (g), (h), and (i);
the combination of the polynucleotides (g), (h), and (j);
the combination of the polynucleotides (g), (h), and (k);
the combination of the polynucleotides (g), (h), and (l);
the combination of the polynucleotides (g), (i), and (j);
the combination of the polynucleotides (g), (i), and (k);
the combination of the polynucleotides (g), (i), and (l);
the combination of the polynucleotides (g), (j), and (k);
the combination of the polynucleotides (g), (j), and (l);
the combination of the polynucleotides (g), (k), and (l);
the combination of the polynucleotides (h), (i), and (j);
the combination of the polynucleotides (h), (i), and (k);
the combination of the polynucleotides (h), (i), and (l);
the combination of the polynucleotides (h), (j), and (k);
the combination of the polynucleotides (h), (j), and (l);
the combination of the polynucleotides (h), (k), and (l);
the combination of the polynucleotides (i), (j), and (k);
the combination of the polynucleotides (i), (j), and (l);
the combination of the polynucleotides (i), (k), and (l);
the combination of the polynucleotides (j), (k), and (l);
the combination of the polynucleotides (a), (b), (c), and (d);
the combination of the polynucleotides (b), (c), (f), and (g);
the combination of the polynucleotides (b), (c), (f), and (h);
the combination of the polynucleotides (b), (c), (f), and (i);
the combination of the polynucleotides (b), (c), (f), and (j);
the combination of the polynucleotides (b), (c), (f), and (k);
the combination of the polynucleotides (b), (c), (f), and (l);
the combination of the polynucleotides (b), (c), (g), and (h);
the combination of the polynucleotides (b), (c), (g), and (i);
the combination of the polynucleotides (b), (c), (g), and (j);
the combination of the polynucleotides (b), (c), (g), and (k);
the combination of the polynucleotides (b), (c), (g), and (l);
the combination of the polynucleotides (b), (c), (h), and (i);
the combination of the polynucleotides (b), (c), (h), and (j);
the combination of the polynucleotides (b), (c), (h), and (k);
the combination of the polynucleotides (b), (c), (h), and (l);
the combination of the polynucleotides (b), (c), (i), and (j);
the combination of the polynucleotides (b), (c), (i), and (k);
the combination of the polynucleotides (b), (c), (i), and (l);
the combination of the polynucleotides (b), (c), (j), and (k);
the combination of the polynucleotides (b), (c), (j), and (l);
the combination of the polynucleotides (b), (c), (k), and (l);
the combination of the polynucleotides (b), (f), (g), and (h);
the combination of the polynucleotides (b), (f), (g), and (i);
the combination of the polynucleotides (b), (f), (g), and (j);
the combination of the polynucleotides (b), (f), (g), and (k);
the combination of the polynucleotides (b), (f), (g), and (l);
the combination of the polynucleotides (b), (f), (h), and (i);
the combination of the polynucleotides (b), (f), (h), and (j);
the combination of the polynucleotides (b), (f), (h), and (k);
the combination of the polynucleotides (b), (f), (h), and (l);
the combination of the polynucleotides (b), (f), (i), and (j);
the combination of the polynucleotides (b), (f), (i), and (k);
the combination of the polynucleotides (b), (f), (i), and (l);
the combination of the polynucleotides (b), (f), (j), and (k);
the combination of the polynucleotides (b), (f), (j), and (l);
the combination of the polynucleotides (b), (f), (k), and (l);
the combination of the polynucleotides (b), (g), (h), and (i);
the combination of the polynucleotides (b), (g), (h), and (j);
the combination of the polynucleotides (b), (g), (h), and (k);
the combination of the polynucleotides (b), (g), (h), and (l);
the combination of the polynucleotides (b), (g), (i), and (j);
the combination of the polynucleotides (b), (g), (i), and (k);
the combination of the polynucleotides (b), (g), (i), and (l);
the combination of the polynucleotides (b), (g), (j), and (k);
the combination of the polynucleotides (b), (g), (j), and (l);
the combination of the polynucleotides (b), (g), (k), and (l);
the combination of the polynucleotides (b), (h), (i), and (j);
the combination of the polynucleotides (b), (h), (i), and (k);
the combination of the polynucleotides (b), (h), (i), and (l);
the combination of the polynucleotides (b), (h), (j), and (k);
the combination of the polynucleotides (b), (h), (j), and (l);
the combination of the polynucleotides (b), (h), (k), and (l);
the combination of the polynucleotides (b), (i), (j), and (k);
the combination of the polynucleotides (b), (i), (j), and (l);
the combination of the polynucleotides (b), (i), (k), and (l);
the combination of the polynucleotides (b), (j), (k), and (l);
the combination of the polynucleotides (c), (f), (g), and (h);
the combination of the polynucleotides (c), (f), (g), and (i);
the combination of the polynucleotides (c), (f), (g), and (j);
the combination of the polynucleotides (c), (f), (g), and (k);
the combination of the polynucleotides (c), (f), (g), and (l);
the combination of the polynucleotides (c), (f), (h), and (i);
the combination of the polynucleotides (c), (f), (h), and (j);
the combination of the polynucleotides (c), (f), (h), and (k);
the combination of the polynucleotides (c), (f), (h), and (l);
the combination of the polynucleotides (c), (f), (i), and (j);
the combination of the polynucleotides (c), (f), (i), and (k);
the combination of the polynucleotides (c), (f), (i), and (l);
the combination of the polynucleotides (c), (f), (j), and (k);
the combination of the polynucleotides (c), (f), (j), and (l);
the combination of the polynucleotides (c), (f), (k), and (l);
the combination of the polynucleotides (c), (g), (h), and (i);
the combination of the polynucleotides (c), (g), (h), and (j);
the combination of the polynucleotides (c), (g), (h), and (k);
the combination of the polynucleotides (c), (g), (h), and (l);
the combination of the polynucleotides (c), (g), (i), and (j);
the combination of the polynucleotides (c), (g), (i), and (k);
the combination of the polynucleotides (c), (g), (i), and (l);
the combination of the polynucleotides (c), (g), (j), and (k);
the combination of the polynucleotides (c), (g), (j), and (l);
the combination of the polynucleotides (c), (g), (k), and (l);
the combination of the polynucleotides (c), (h), (i), and (j);
the combination of the polynucleotides (c), (h), (i), and (k);
the combination of the polynucleotides (c), (h), (i), and (l);
the combination of the polynucleotides (c), (h), (j), and (k);
the combination of the polynucleotides (c), (h), (j), and (l);
the combination of the polynucleotides (c), (h), (k), and (l);
the combination of the polynucleotides (c), (i), (j), and (k);
the combination of the polynucleotides (c), (i), (j), and (l);
the combination of the polynucleotides (c), (i), (k), and (l);
the combination of the polynucleotides (c), (j), (k), and (l);
the combination of the polynucleotides (f), (g), (h) and (i);
the combination of the polynucleotides (f), (g), (h), and (j);
the combination of the polynucleotides (f), (g), (h), and (k);
the combination of the polynucleotides (f), (g), (h), and (l);
the combination of the polynucleotides (f), (g), (i), and (j);
the combination of the polynucleotides (f), (g), (i), and (k);
the combination of the polynucleotides (f), (g), (i), and (l);
the combination of the polynucleotides (f), (g), (j), and (k);
the combination of the polynucleotides (f), (g), (j), and (l);
the combination of the polynucleotides (f), (g), (k), and (l);
the combination of the polynucleotides (f), (h), (i), and (j);
the combination of the polynucleotides (f), (h), (i), and (k);
the combination of the polynucleotides (f), (h), (i), and (l);
the combination of the polynucleotides (f), (h), (j), and (k);
the combination of the polynucleotides (f), (h), (j), and (l);
the combination of the polynucleotides (f), (h), (k), and (l);
the combination of the polynucleotides (f), (i), (j), and (k);
the combination of the polynucleotides (f), (i), (j), and (l);
the combination of the polynucleotides (f), (i), (k), and (l);
the combination of the polynucleotides (f), (j), (k), and (l);
the combination of the polynucleotides (g), (h), (i), and (j);
the combination of the polynucleotides (g), (h), (i), and (k);
the combination of the polynucleotides (g), (h), (i), and (l);
the combination of the polynucleotides (g), (h), (j), and (k);
the combination of the polynucleotides (g), (h), (j), and (l);
the combination of the polynucleotides (g), (h), (k), and (l);
the combination of the polynucleotides (g), (i), (j), and (k);
the combination of the polynucleotides (g), (i), (j), and (l);
the combination of the polynucleotides (g), (i), (k), and (l);
the combination of the polynucleotides (g), (j), (k), and (l);
the combination of the polynucleotides (h), (i), (j), and (k);
the combination of the polynucleotides (h), (i), (j), and (l);
the combination of the polynucleotides (h), (i), (k), and (l);
the combination of the polynucleotides (h), (j), (k), and (l);
the combination of the polynucleotides (i), (j), (k), and (l);
the combination of the polynucleotides (b), (c), (f), (g), and (h);
the combination of the polynucleotides (b), (c), (f), (g), and (i);
the combination of the polynucleotides (b), (c), (f), (g), and (j);
the combination of the polynucleotides (b), (c), (f), (g), and (k);
the combination of the polynucleotides (b), (c), (f), (g), and (l);
the combination of the polynucleotides (b), (c), (f), (h), and (i);
the combination of the polynucleotides (b), (c), (f), (h), and (j);
the combination of the polynucleotides (b), (c), (f), (h), and (k);
the combination of the polynucleotides (b), (c), (f), (h), and (l);
the combination of the polynucleotides (b), (c), (f), (i), and (j);
the combination of the polynucleotides (b), (c), (f), (i), and (k);
the combination of the polynucleotides (b), (c), (f), (i), and (l);
the combination of the polynucleotides (b), (c), (f), (j), and (k);
the combination of the polynucleotides (b), (c), (f), (j), and (l);
the combination of the polynucleotides (b), (c), (f), (k), and (l);
the combination of the polynucleotides (b), (c), (g), (h), and (i);
the combination of the polynucleotides (b), (c), (g), (h), and (j);
the combination of the polynucleotides (b), (c), (g), (h), and (k);
the combination of the polynucleotides (b), (c), (g), (h), and (l);
the combination of the polynucleotides (b), (c), (g), (i), and (j);
the combination of the polynucleotides (b), (c), (g), (i), and (k);
the combination of the polynucleotides (b), (c), (g), (i), and (l);
the combination of the polynucleotides (b), (c), (g), (j), and (k);
the combination of the polynucleotides (b), (c), (g), (j), and (l);
the combination of the polynucleotides (b), (c), (g), (k), and (l);
the combination of the polynucleotides (b), (c), (h), (i), and (j);
the combination of the polynucleotides (b), (c), (h), (i), and (k);
the combination of the polynucleotides (b), (c), (h), (i), and (l);
the combination of the polynucleotides (b), (c), (h), (j), and (k);
the combination of the polynucleotides (b), (c), (h), (j), and (l);
the combination of the polynucleotides (b), (c), (h), (k), and (l);
the combination of the polynucleotides (b), (c), (i), (j), and (k);
the combination of the polynucleotides (b), (c), (i), (j), and (l);
the combination of the polynucleotides (b), (c), (i), (k), and (l);
the combination of the polynucleotides (b), (c), (j), (k), and (l);
the combination of the polynucleotides (b), (f), (g), (h), and (i);
the combination of the polynucleotides (b), (f), (g), (h), and (j);
the combination of the polynucleotides (b), (f), (g), (h), and (k);
the combination of the polynucleotides (b), (f), (g), (h), and (l);
the combination of the polynucleotides (b), (f), (g), (i), and (j);
the combination of the polynucleotides (b), (f), (g), (i), and (k);
the combination of the polynucleotides (b), (f), (g), (i), and (l);
the combination of the polynucleotides (b), (f), (g), (j), and (k);
the combination of the polynucleotides (b), (f), (g), (j), and (l);
the combination of the polynucleotides (b), (f), (g), (k), and (l);
the combination of the polynucleotides (b), (f), (h), (i), and (j);
the combination of the polynucleotides (b), (f), (h), (i), and (k);
the combination of the polynucleotides (b), (f), (h), (i), and (l);
the combination of the polynucleotides (b), (f), (h), (j), and (k);
the combination of the polynucleotides (b), (f), (h), (j), and (l);
the combination of the polynucleotides (b), (f), (h), (k), and (l);
the combination of the polynucleotides (b), (f), (i), (j), and (k);
the combination of the polynucleotides (b), (f), (i), (j), and (l);
the combination of the polynucleotides (b), (f), (i), (k), and (l);
the combination of the polynucleotides (b), (f), (j), (k), and (l);
the combination of the polynucleotides (b), (g), (h), (i), and (j);
the combination of the polynucleotides (b), (g), (h), (i), and (k);
the combination of the polynucleotides (b), (g), (h), (i), and (l);
the combination of the polynucleotides (b), (g), (h), (j), and (k);
the combination of the polynucleotides (b), (g), (h), (j), and (l);
the combination of the polynucleotides (b), (g), (h), (k), and (l);
the combination of the polynucleotides (b), (g), (i), (j), and (k);
the combination of the polynucleotides (b), (g), (i), (j), and (l);
the combination of the polynucleotides (b), (g), (i), (k), and (l);
the combination of the polynucleotides (b), (g), (j), (k), and (l);
the combination of the polynucleotides (b), (h), (i), (j), and (k);
the combination of the polynucleotides (b), (h), (i), (j), and (l);
the combination of the polynucleotides (b), (h), (i), (k), and (l);
the combination of the polynucleotides (b), (h), (j), (k), and (l);
the combination of the polynucleotides (b), (i), (j), (k), and (l);
the combination of the polynucleotides (c), (f), (g), (h), and (i);
the combination of the polynucleotides (c), (f), (g), (h), and (j);
the combination of the polynucleotides (c), (f), (g), (h), and (k);
the combination of the polynucleotides (c), (f), (g), (h), and (l);
the combination of the polynucleotides (c), (f), (g), (i), and (j);
the combination of the polynucleotides (c), (f), (g), (i), and (k);
the combination of the polynucleotides (c), (f), (g), (i), and (l);
the combination of the polynucleotides (c), (f), (g), (j), and (k);
the combination of the polynucleotides (c), (f), (g), (j), and (l);
the combination of the polynucleotides (c), (f), (g), (k), and (l);
the combination of the polynucleotides (c), (f), (h), (i), and (j);
the combination of the polynucleotides (c), (f), (h), (i), and (k);
the combination of the polynucleotides (c), (f), (h), (i), and (l);
the combination of the polynucleotides (c), (f), (h), (j), and (k);
the combination of the polynucleotides (c), (f), (h), (j), and (l);
the combination of the polynucleotides (c), (f), (h), (k), and (l);
the combination of the polynucleotides (c), (f), (i), (j), and (k);
the combination of the polynucleotides (c), (f), (i), (j), and (l);
the combination of the polynucleotides (c), (f), (i), (k), and (l);
the combination of the polynucleotides (c), (f), (j), (k), and (l);
the combination of the polynucleotides (c), (g), (h), (i), and (j);
the combination of the polynucleotides (c), (g), (h), (i), and (k);
the combination of the polynucleotides (c), (g), (h), (i), and (l);
the combination of the polynucleotides (c), (g), (h), (j), and (k);
the combination of the polynucleotides (c), (g), (h), (j), and (l);
the combination of the polynucleotides (c), (g), (h), (k), and (l);
the combination of the polynucleotides (c), (g), (i), (j), and (k);
the combination of the polynucleotides (c), (g), (i), (j), and (l);
the combination of the polynucleotides (c), (g), (i), (k), and (l);
the combination of the polynucleotides (c), (g), (j), (k), and (l);
the combination of the polynucleotides (c), (h), (i), (j), and (k);
the combination of the polynucleotides (c), (h), (i), (j), and (l);
the combination of the polynucleotides (c), (h), (i), (k), and (l);
the combination of the polynucleotides (c), (h), (j), (k), and (l);
the combination of the polynucleotides (c), (i), (j), (k), and (l);
the combination of the polynucleotides (f), (g), (h), (i), and (j);
the combination of the polynucleotides (f), (g), (h), (i), and (k);
the combination of the polynucleotides (f), (g), (h), (i), and (l);
the combination of the polynucleotides (f), (g), (h), (j), and (k);
the combination of the polynucleotides (f), (g), (h), (j), and (l);
the combination of the polynucleotides (f), (g), (h), (k), and (l);
the combination of the polynucleotides (f), (g), (i), (j), and (k);
the combination of the polynucleotides (f), (g), (i), (j), and (l);
the combination of the polynucleotides (f), (g), (i), (k), and (l);
the combination of the polynucleotides (f), (g), (j), (k), and (l);
the combination of the polynucleotides (f), (h), (i), (j), and (k);
the combination of the polynucleotides (f), (h), (i), (j), and (l);
the combination of the polynucleotides (f), (h), (i), (k), and (l);
the combination of the polynucleotides (f), (h), (j), (k), and (l);
the combination of the polynucleotides (f), (i), (j), (k), and (l);
the combination of the polynucleotides (g), (h), (i), (j), and (k);
the combination of the polynucleotides (g), (h), (i), (j), and (l);
the combination of the polynucleotides (g), (h), (i), (k), and (l);
the combination of the polynucleotides (g), (h), (j), (k), and (l);
the combination of the polynucleotides (g), (i), (j), (k), and (l);
the combination of the polynucleotides (h), (i), (j), (k), and (l);
the combination of the polynucleotides (b), (c), (f), (g), (h), and (i);
the combination of the polynucleotides (b), (c), (f), (g), (h), and (j);
the combination of the polynucleotides (b), (c), (f), (g), (h), and (k);
the combination of the polynucleotides (b), (c), (f), (g), (h), and (l);
the combination of the polynucleotides (b), (c), (f), (g), (i), and (j);
the combination of the polynucleotides (b), (c), (f), (g), (i), and (k);
the combination of the polynucleotides (b), (c), (f), (g), (i), and (l);
the combination of the polynucleotides (b), (c), (f), (g), (j), and (k);
the combination of the polynucleotides (b), (c), (f), (g), (j), and (l);
the combination of the polynucleotides (b), (c), (f), (g), (k), and (l);
the combination of the polynucleotides (b), (c), (f), (h), (i), and (j);
the combination of the polynucleotides (b), (c), (f), (h), (i), and (k);
the combination of the polynucleotides (b), (c), (f), (h), (i), and (l);
the combination of the polynucleotides (b), (c), (f), (h), (j), and (k);
the combination of the polynucleotides (b), (c), (f), (h), (j), and (l);
the combination of the polynucleotides (b), (c), (f), (h), (k), and (l);
the combination of the polynucleotides (b), (c), (f), (i), (j), and (k);
the combination of the polynucleotides (b), (c), (f), (i), (j), and (l);
the combination of the polynucleotides (b), (c), (f), (i), (k), and (l);
the combination of the polynucleotides (b), (c), (f), (j), (k), and (l);
the combination of the polynucleotides (b), (c), (g), (h), (i), and (j);
the combination of the polynucleotides (b), (c), (g), (h), (i), and (k);
the combination of the polynucleotides (b), (c), (g), (h), (i), and (l);
the combination of the polynucleotides (b), (c), (g), (h), (j), and (k);
the combination of the polynucleotides (b), (c), (g), (h), (j), and (l);
the combination of the polynucleotides (b), (c), (g), (h), (k), and (l);
the combination of the polynucleotides (b), (c), (g), (i), (j), and (k);
the combination of the polynucleotides (b), (c), (g), (i), (j), and (l);
the combination of the polynucleotides (b), (c), (g), (i), (k), and (l);
the combination of the polynucleotides (b), (c), (g), (j), (k), and (l);
the combination of the polynucleotides (b), (c), (h), (i), (j), and (k);
the combination of the polynucleotides (b), (c), (h), (i), (j), and (l);
the combination of the polynucleotides (b), (c), (h), (i), (k), and (l);
the combination of the polynucleotides (b), (c), (h), (j), (k), and (l);
the combination of the polynucleotides (b), (c), (i), (j), (k), and (l);
the combination of the polynucleotides (b), (f), (g), (h), (i), and (j);
the combination of the polynucleotides (b), (f), (g), (h), (i), and (k);
the combination of the polynucleotides (b), (f), (g), (h), (i), and (l);
the combination of the polynucleotides (b), (f), (g), (h), (j), and (k);
the combination of the polynucleotides (b), (f), (g), (h), (j), and (l);
the combination of the polynucleotides (b), (f), (g), (h), (k), and (l);
the combination of the polynucleotides (b), (f), (g), (i), (j), and (k);
the combination of the polynucleotides (b), (f), (g), (i), (j), and (l);
the combination of the polynucleotides (b), (f), (g), (i), (k), and (l);
the combination of the polynucleotides (b), (f), (g), (j), (k), and (l);
the combination of the polynucleotides (b), (f), (h), (i), (j), and (k);
the combination of the polynucleotides (b), (f), (h), (i), (j), and (l);
the combination of the polynucleotides (b), (f), (h), (i), (k), and (l);
the combination of the polynucleotides (b), (f), (h), (j), (k), and (l);
the combination of the polynucleotides (b), (f), (i), (j), (k), and (l);
the combination of the polynucleotides (b), (g), (h), (i), (j), and (k);
the combination of the polynucleotides (b), (g), (h), (i), (j), and (l);
the combination of the polynucleotides (b), (g), (h), (i), (k), and (l);
the combination of the polynucleotides (b), (g), (h), (j), (k), and (l);
the combination of the polynucleotides (b), (g), (i), (j), (k), and (l);
the combination of the polynucleotides (b), (h), (i), (j), (k), and (l);
the combination of the polynucleotides (c), (f), (g), (h), (i), and (j);
the combination of the polynucleotides (c), (f), (g), (h), (i), and (k);
the combination of the polynucleotides (c), (f), (g), (h), (i), and (l);
the combination of the polynucleotides (c), (f), (g), (h), (j), and (k);
the combination of the polynucleotides (c), (f), (g), (h), (j), and (l);
the combination of the polynucleotides (c), (f), (g), (h), (k), and (l);
the combination of the polynucleotides (c), (f), (g), (i), (j), and (k);
the combination of the polynucleotides (c), (f), (g), (i), (j), and (l);
the combination of the polynucleotides (c), (f), (g), (i), (k), and (l);
the combination of the polynucleotides (c), (f), (g), (j), (k), and (l);
the combination of the polynucleotides (c), (f), (h), (i), (j), and (k);
the combination of the polynucleotides (c), (f), (h), (i), (j), and (l);
the combination of the polynucleotides (c), (f), (h), (i), (k), and (l);
the combination of the polynucleotides (c), (f), (h), (j), (k), and (l);
the combination of the polynucleotides (c), (f), (i), (j), (k), and (l);
the combination of the polynucleotides (c), (g), (h), (i), (j), and (k);
the combination of the polynucleotides (c), (g), (h), (i), (j), and (l);
the combination of the polynucleotides (c), (g), (h), (i), (k), and (l);
the combination of the polynucleotides (c), (g), (h), (j), (k), and (l);
the combination of the polynucleotides (c), (g), (i), (j), (k), and (l);
the combination of the polynucleotides (c), (h), (i), (j), (k), and (l);
the combination of the polynucleotides (f), (g), (h), (i), (j), and (k);
the combination of the polynucleotides (f), (g), (h), (i), (j), and (l);
the combination of the polynucleotides (f), (g), (h), (i), (k), and (l);
the combination of the polynucleotides (f), (g), (h), (j), (k), and (l);
the combination of the polynucleotides (f), (g), (i), (j), (k), and (l);
the combination of the polynucleotides (f), (h), (i), (j), (k), and (l);
the combination of the polynucleotides (g), (h), (i), (j), (k), and (l);
the combination of the polynucleotides (b), (c), (f), (g), (h), (i), and (j);
the combination of the polynucleotides (b), (c), (f), (g), (h), (i), and (k);
the combination of the polynucleotides (b), (c), (f), (g), (h), (i), and (l);
the combination of the polynucleotides (b), (c), (f), (g), (h), (j), and (k);
the combination of the polynucleotides (b), (c), (f), (g), (h), (j), and (l);
the combination of the polynucleotides (b), (c), (f), (g), (h), (k), and (l);
the combination of the polynucleotides (b), (c), (f), (g), (i), (j), and (k);
the combination of the polynucleotides (b), (c), (f), (g), (i), (j), and (l);
the combination of the polynucleotides (b), (c), (f), (g), (i), (k), and (l);
the combination of the polynucleotides (b), (c), (f), (g), (j), (k), and (l);
the combination of the polynucleotides (b), (c), (f), (h), (i), (j), and (k);
the combination of the polynucleotides (b), (c), (f), (h), (i), (j), and (l);
the combination of the polynucleotides (b), (c), (f), (h), (i), (k), and (l);
the combination of the polynucleotides (b), (c), (f), (h), (j), (k), and (l);
the combination of the polynucleotides (b), (c), (f), (i), (j), (k), and (l);
the combination of the polynucleotides (b), (c), (g), (h), (i), (j), and (k);
the combination of the polynucleotides (b), (c), (g), (h), (i), (j), and (l);
the combination of the polynucleotides (b), (c), (g), (h), (i), (k), and (l);
the combination of the polynucleotides (b), (c), (g), (h), (j), (k), and (l);
the combination of the polynucleotides (b), (c), (g), (i), (j), (k), and (l);
the combination of the polynucleotides (b), (c), (h), (i), (j), (k), and (l);
the combination of the polynucleotides (b), (f), (g), (h), (i), (j), and (k);
the combination of the polynucleotides (b), (f), (g), (h), (i), (j), and (l);
the combination of the polynucleotides (b), (f), (g), (h), (i), (k), and (l);
the combination of the polynucleotides (b), (f), (g), (h), (j), (k), and (l);
the combination of the polynucleotides (b), (f), (g), (i), (j), (k), and (l);
the combination of the polynucleotides (b), (f), (h), (i), (j), (k), and (l);
the combination of the polynucleotides (b), (g), (h), (i), (j), (k), and (l);
the combination of the polynucleotides (c), (f), (g), (h), (i), (j), and (k);
the combination of the polynucleotides (c), (f), (g), (h), (i), (j), and (l);
the combination of the polynucleotides (c), (f), (g), (h), (i), (k), and (l);
the combination of the polynucleotides (c), (f), (g), (h), (j), (k), and (l);
the combination of the polynucleotides (c), (f), (g), (i), (j), (k), and (l);
the combination of the polynucleotides (c), (f), (h), (i), (j), (k), and (l);
the combination of the polynucleotides (c), (g), (h), (i), (j), (k), and (l);
the combination of the polynucleotides (f), (g), (h), (i), (j), (k), and (l);
the combination of the polynucleotides (b), (c), (f), (g), (h), (i), (j), and (k);
the combination of the polynucleotides (b), (c), (f), (g), (h), (i), (j), and (l);
the combination of the polynucleotides (b), (c), (f), (g), (h), (i), (k), and (l);
the combination of the polynucleotides (b), (c), (f), (g), (h), (j), (k), and (l);
the combination of the polynucleotides (b), (c), (f), (g), (i), (j), (k), and (l);
the combination of the polynucleotides (b), (c), (f), (h), (i), (j), (k), and (l);
the combination of the polynucleotides (b), (c), (g), (h), (i), (j), (k), and (l);
the combination of the polynucleotides (b), (f), (g), (h), (i), (j), (k), and (l);
the combination of the polynucleotides (c), (f), (g), (h), (i), (j), (k), and (l); and
the combination of the polynucleotides (b), (c), (f), (g), (h), (i), (j), (k), and (l).

Of these combinations, for example, the following combinations are preferable because they show higher correlation with the *Eustoma* fusarium wilt resistance:
the combination of the polynucleotides (f) and (k);
the combination of the polynucleotides (a), (b), and (c);
the combination of the polynucleotides (a), (b), (c), and (d); and
the combination of the polynucleotides (b), (c), (f), (g), (h), (i), (j), (k), and (l).

As the base sequence including an SNP marker, a base sequence including Egra20_3656645 (the polynucleotide that consists of the base sequence of SEQ ID NO: 5) may be used, for example. In this case, the above-described polynucleotide may be, for example, a polynucleotide that consists of the base sequence of the above-described polynucleotide in which one or several bases are deleted, substituted, inserted, and/or added with the polymorphism or the underlined base being conserved or a polynucleotide that consists of a base sequence having at least 80% sequence identity to the base sequence of the above-described polynucleotide with the polymorphism or the underlined base being conserved. The above descriptions regarding the term "one or several" and the sequence identity, reference can be made to the descriptions thereon regarding the polynucleotides (a2) and (a3), for example. Each of the polynucleotides may be used, for example, in combination with at least one polynucleotide selected from the group consisting of the polynucleotides (a), (b), (c), (d), (f), (g), (h), (i), (j), (k), and (l).

In the present disclosure, as the base sequence including an SNP marker described in the above item (2), any one or more of polynucleotides including SNPs 1 to 66, respectively, may be used as the base sequence(s) including an SNP marker, in addition to or instead of the above-described polynucleotides (a) to (d) and (f) to (l). In Table 1 and Tables 2A and 2B above, the polynucleotides including SNPs 1 to 66, respectively, are each a polynucleotide consisting of a base sequence including a polymorphism in which the base [N₁/N₂] in brackets is N₂. That is, the polynucleotides including SNPs 1 to 66, respectively, are polynucleotides consisting of the base sequences of SEQ ID NOs: 6 to 71, respectively, each including a polymorphism in which the base [N₁/N₂] in brackets is N₂. The polynucleotides including SNPs 1 to 66, respectively, can be obtained from a *Eustoma* plant deposited under Accession No. FERM BP-22427, as described below, for example.

The polynucleotides including SNPs 1 to 66, respectively, may be, for example, polynucleotides that consist of base sequences corresponding to the base sequences of SEQ ID NOs: 6 to 71, respectively, in which one or several bases are deleted, substituted, inserted, and/or added with the base N₂ being conserved and has a function equivalent to those of the polynucleotides including SNPs 1 to 66, respectively, regarding *Eustoma* fusarium wilt resistance in the *Eustoma* fusarium wilt resistance locus. The term "one or several" means, for example, 1 to 40, 1 to 35, 1 to 30, 1 to 25, 1 to 20, 1 to 18, 1 to 12, 1 to 8, 1 to 4, 1 to 3, or 1 or 2.

The polynucleotides including SNPs 1 to 66, respectively, may be, for example, polynucleotides that consist of base sequences having at least 80% sequence identity to the base sequences of SEQ ID NOs: 6 to 71, respectively, with the base N₂ of the corresponding base sequences being conserved and has a function equivalent to those of the polynucleotides including SNPs 1 to 66, respectively, regarding *Eustoma* fusarium wilt resistance in the *Eustoma* fusarium wilt resistance locus. The sequence identity is, for example, at least 80%, at least 85%, at least 89%, at least 90%, at least 95%, at least 96%, at least 97%, at least 98%, or at least 99%.

### (3) Identification by Region Between Sites of Two SNP Markers

The resistance locus is linked to SNP markers, as described above. Accordingly, the resistance locus may be specified, for example, by a base sequence of a region between sites of two SNP markers, as described in (3) above or by a region specified by two SNP markers (such a region is also referred to as "chromosomal location" or "mapping region"). The base sequence of a region between sites of two SNP markers is not limited to particular base sequences, and is, for example, a base sequence of a region between sites of two SNP markers selected from the group consisting of Egra20_3656645, Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 on the chromosome. Regarding the base sequence of a region between sites of two SNP markers, reference may be made to, for example, the base sequence of a region between sites of the corresponding two SNP markers in a *Eustoma* plant deposited under Accession No. FERM BP-22427 (hereinafter simply referred to as "deposited line"). When compared with the base sequence of the deposited line, the base sequence of the region between the sites of the two SNP markers completely or partially matches the base sequence of the deposited line. In the latter case, the base sequence of the region between the sites of the two SNP markers need only be such that a *Eustoma* plant having a resistance locus identified by the base sequence of the region between the sites of the two SNP markers exhibits *Eustoma* fusarium wilt resistance. The partial match can be specified, for example, by the sequence identity to the base sequence of the deposited line. The sequence identity is, for example, at least 80%, at least 85%, at least 89%, at least 90%, at least 95%, at least 96%, at least 97%, at least 98%, at least 99%, at least 99.1%, at least 99.2%, 99.3%, at least 99.4%, at least 99.5%, at least 99.6%, at least 99.7%, at least 99.8%, or at least 99.9%. When the resistance locus is identified by (3) above, it can also be said that the resistance locus is located in a region between sites of two SNP markers, for example. When the resistance locus is identified by the region in which the resistance locus is located, the resistance locus may be identified by further using an SNP marker or a base sequence including an SNP marker in combination.

The above-described Egra20_3656645 (also referred to as "SNP (e)" hereinafter) is a polymorphism of the underlined base in brackets in SEQ ID NO: 5, for example. In the *Eustoma* plant deposited under Accession No. FERM BP-22427, as described below, SNP (e) is a polymorphism in which the underlined base in brackets is C. However, SNP (e) may be a polymorphism in which the underlined base in brackets is other than C, i.e., A, T, or G, for example. The base sequence of SEQ ID NO: 5 can be obtained from the *Eustoma* plant deposited under Accession No. FERM BP-22427, as described below, for example. SNP (e) also can be identified, for example, on the basis of known information in the database of the above-described web site, and, as a specific example, SNP (e) can be identified as the 3656645th base on chromosome 20 of Egra_v1.0.

SEQ ID NO: 5:
5'-CACAGGTGGTAGGATGGCCTCCAGTTCGATCGTTCCGAAAGAATTCAATGGCAAA
CAATCCTCACAAGGCTGATGAAGAAGCAGATGCAATGACTGGAAG[C]AGTCGTCTTT
ACGTCAAAGTTAGCATGGATGGTGCCCCTTATCTGAGGAAAGTTGATATCAAGACATA
CAACAGCTACAAGGACCTGTCCTCTGCACTTG-3'.

The upstream end and the downstream end of the above-described region can be identified, for example, by the sites of the two SNP markers, as described above. The region is not limited as long as, for example, it extends between the sites of the two SNP markers, and may or may not include both or one of the sites of the two SNP markers, for example. When the region includes the sites of the SNP markers, the upstream end and the downstream end of the region correspond to the sites of the SNP markers. It is to be noted that the base at the upstream end and the base at the downstream end may each be, for example, the above underlined base in the corresponding base sequence or a base other than the underlined base.

The combination of the two SNP markers specifying the region is not limited to particular combinations, and examples thereof include the following combinations:
(1) the SNP at the upstream end is SNP (e) or SNP (a), and the SNP at the downstream end is SNP (c) or SNP (d);
(2) the SNP at the upstream end is SNP (e), and the SNP at the downstream end is SNP (d);
(3) the SNP at the upstream end is SNP (a), and the SNP at the downstream end is SNP (d);
(4) the SNP at the upstream end is SNP (a), and the SNP at the downstream end is SNP (c);
(5) the SNP at the upstream end is SNP (b), and the SNP at the downstream end is SNP (c);
(6) the SNP at the upstream end is SNP (b), and the SNP at the downstream end is SNP (d);
(7) the SNP at the upstream end is SNP (b), and the SNP at the downstream end is SNP (l); and
(8) the SNP at the upstream end is SNP (f), and the SNP at the downstream end is SNP (k).

Of these combinations, for example, the following combinations are preferable because they show higher correlation with the *Eustoma* fusarium wilt resistance:
combinations (2) through (8) above.

Further, of the above-described combinations, for example, the following combinations are preferable because they show still higher correlation with the *Eustoma* fusarium wilt resistance:
the combination (3);
the combination (5);
the combination (7); and
the combination (8).

As the SNPs specifying a region on the chromosome, SNPs 1 to 66 may be used in addition to or instead of SNPs (a) to (d) and (f) to (l).

In the case where the resistance locus is specified by the base sequence of a region between sites of two SNP markers, it is preferable that the resistance locus further includes, in the base sequence of the region, the SNP marker(s) located in the region. Specifically, it is preferable that the resistance locus includes, in the base sequence of the region, at least one SNP marker selected from the group consisting of Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378, for example.

The SNP marker(s) located in the region may be, for example, an SNP marker(s) at one or both of the sites of the two SNP markers specifying the region on the chromosome or an SNP marker(s) located between the sites of the two SNP markers specifying the region. The former SNP marker(s) is also referred to as an SNP marker(s) at the end(s) of the region, and the latter SNP marker(s) is also referred to as an SNP marker(s) inside the region. SNP markers located in the region may include both an SNP marker(s) at the end(s) of the region and an SNP marker(s) inside the region, for example.

The SNP marker(s) inside the region may be, for example, an SNP marker(s) located between the furthest upstream SNP marker and the furthest downstream SNP marker specifying the region, and can be determined as appropriate, for example, on the basis of the locations of the SNP markers shown in FIG. 1. The number of SNP markers between the sites of the two SNP markers need only be, for example, one or more, and as a specific example, the SNP markers between the sites of the two SNP markers may be all the SNP markers located between the sites of the SNP markers specifying the region.

The combination of the base sequence of a region between sites of two SNP markers and an SNP marker(s) included in the base sequence of the region is not limited to particular combinations, and may be, for example, the combination satisfying the following condition (i), (ii), (iii), (iv), or (v).

### Condition (i):

The resistance locus includes the base sequence of a region between sites of SNP (e) and SNP (d) on the chromosome or is located in the region, and
the base sequence of the region includes (is identified by) at least one SNP marker selected from the group consisting of SNP (a), SNP (b), SNP (c), and SNP (d), preferably at least one SNP marker selected from the group consisting of SNP (a), SNP (b), and SNP (c).

### Condition (ii):

The resistance locus includes the base sequence of a region between sites of SNP (a) and SNP (d) on the chromosome or is located in the region, and
the base sequence of the region includes (is identified by) at least one SNP marker selected from the group consisting of SNP (a), SNP (b), SNP (c), and SNP (d), preferably at least one SNP marker selected from the group consisting of SNP (a), SNP (b), and SNP (c).

### Condition (iii):

The resistance locus includes the base sequence of a region between sites of SNP (a) and SNP (c) on the chromosome, and
the base sequence of the region includes (is identified by) at least one SNP marker selected from the group consisting of SNP (a), SNP (b), and SNP (c).

### Condition (iv):

The resistance locus includes the base sequence of a region between sites of SNP (b) and SNP (l) on the chromosome or is located in the region, and
the base sequence of the region includes (is identified by) at least one SNP marker selected from the group consisting of SNP (b), SNP (c), SNP (f), SNP (g), SNP (h), SNP (i), SNP (j), SNP (k), and SNP (l).

### Condition (v):

The resistance locus includes the base sequence of a region between sites of SNP (f) and SNP (k) on the chromosome or is located in the region, and
the base sequence of the region includes (is identified by) at least one SNP marker selected from the group consisting of SNP (c), SNP (f), SNP (g), SNP (h), SNP (i), SNP (j), and SNP (k).

The SNP markers located in the region may be SNPs 1 to 66, in addition to or instead of SNPs (a) to (d) and (f) to (l). As a specific example, the combination of the base sequence of a region between sites of two SNP markers and an SNP marker(s) in the base sequence of the region is not limited to particular combinations, and is, for example, the combination satisfying the following condition (vi) or (vii).

### Condition (vi):

The resistance locus includes the base sequence of a region between sites of SNP (b) and SNP (l) on the chromosome or is located in the region, and
the base sequence of the region includes (is identified by) at least one SNP marker selected from the group consisting of SNPs 14 to 59 and 60.

### Condition (vii):

The resistance locus includes the base sequence of a region between sites of SNP (f) and SNP (k) on the chromosome or is located in the region, and
the base sequence of the region includes (is identified by) at least one SNP marker selected from the group consisting of SNPs 18 to 48 and 49.

The resistance locus is, for example, a *Eustoma* fusarium wilt resistance locus on chromosome 20 of the *Eustoma* plant deposited under Accession No. FERM BP-22427, as described below.

The *Eustoma* fusarium wilt resistance marker according to the present disclosure can confer, for example, *Eustoma* fusarium wilt resistance to a *Eustoma* plant. In the present disclosure, the degree of *Eustoma* fusarium wilt resistance of a *Eustoma* plant can be expressed by evaluating the severity of the disease in the *Eustoma* plant according to the method described in Example 1 below and determining the proportion (S₁/S_{A} × 100 (%)) of individuals (Si) having a severity of 1 in all the surveyed individuals (S_{A}). In the evaluation of *Eustoma* fusarium wilt resistance, the surveyed individuals or surveyed population can be evaluated as being resistant to *Eustoma* fusarium wilt when, for example, the proportion (S₁/S_{A} × 100 (%)) is at least 50%, at least 55%, at least 60%, at least 65%, at least 70%, at least 75%, at least 80%, at least 85%, at least 90%, at least 95%, at least 96%, at least 97%, at least 98%, or at least 99%. The number of *Eustoma* plants (surveyed individuals or surveyed population) used to determine the presence or absence of *Eustoma* fusarium wilt resistance is, for example, the number sufficient for the calculation of the above-described proportion, and specifically is 40 to 50, for example.

### <Eustoma Fusarium Wilt-resistant Eustoma Plant>

In still another aspect, the present disclosure provides a *Eustoma* plant that exhibits resistance to *Eustoma* fusarium wilt. The *Eustoma* plant according to the present disclosure is a *Eustoma* fusarium wilt-resistant *Eustoma* plant including a *Eustoma* fusarium wilt resistance locus on chromosome 20. The *Eustoma* plant of the present disclosure can exhibit resistance to *Eustoma* fusarium wilt owing to the presence of the *Eustoma* fusarium wilt resistance locus. In the description regarding the *Eustoma* plant of the present disclosure, the *Eustoma* fusarium wilt resistance locus on chromosome 20 should be interpreted as interchangeable with, for example, the *Eustoma* fusarium wilt resistance marker of the present disclosure.

In the *Eustoma* plant of the present disclosure, the *Eustoma* fusarium wilt resistance can be conferred by the *Eustoma* fusarium wilt resistance locus on chromosome 20, as described above. Although the *Eustoma* plant of the present disclosure has the resistance locus on chromosome 20, the *Eustoma* fusarium wilt resistance locus on chromosome 20 may be, for example, on any chromosome other than chromosome 20, instead of on chromosome 20. That is, the *Eustoma* plant having the resistance locus may have the resistance locus on chromosome 20 on any of chromosomes 1, 2, 3, 4, 5, 6, 7, 8, 9, 10, 11, 12, 13, 14, 15, 16, 17, 18, 19, 21, 22, 23, 24, 25, 26, 27, 28, 29, 30, 31, 32, 33, 34, 35, and 36.

The *Eustoma* plant of the present disclosure may include, for example, one resistance locus or two or more resistance loci. As a specific example, in a pair of chromosomes, one of the chromosomes may include the resistance locus (heterozygous) or both the chromosomes may each include the resistance locus (homozygous).

Regarding the resistance locus in the *Eustoma* plant of the present disclosure, reference can be made to the description on the *Eustoma* fusarium wilt resistance locus provided above in connection with the *Eustoma* fusarium wilt resistance marker for a *Eustoma* plant according to the present disclosure, for example.

When "(3) Identification by Region Between Sites of Two SNP Markers" described above is used to identify the resistance locus of the *Eustoma* plant of the present disclosure, the resistance locus can also be referred to as, for example, a resistance locus located between two SNP markers. That is, it can be said that the *Eustoma* plant of the present disclosure includes, as the above-described resistance locus, a resistance locus located between sites of two SNP markers selected from the group consisting of Egra20_3656645, Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378. Regarding the combination of the two SNP markers, reference can be made to the description thereon in the item "(3) Identification by Region Between Sites of Two SNP Markers" provided above in connection with the marker of the present disclosure. In this case, for example, the "(1) Identification by SNP Marker" and/or "(2) Identification by Base Sequence Including SNP Marker" described above in connection with the marker of the present disclosure may further be used in combination to identify the resistance locus. In this case, regarding the SNP marker(s) and the base sequence including an SNP marker used to identify the resistance locus, reference can be made to the descriptions thereon in the above items "(1) Identification by SNP Marker" and "(2) Identification by Base Sequence Including SNP Marker" provided above in connection with the marker of the present disclosure.

In one example, the *Eustoma* plant of the present disclosure is the *Eustoma* plant deposited under Accession No. FERM BP-22427 *(Eustoma grandiflorum*) or a progeny line thereof. The progeny line has the resistance locus, for example. Information on the deposit is shown below.
Type of deposit: International deposit
Name of depository institution: National Institute of Technology and Evaluation; NITE-IPOD
Address: 2-5-8-120, Kazusakamatari, Kisarazu-shi, Chiba 292-0818, Japan
Accession Number: FERM BP-22427
Identifying designation: Takii 21
Date of acceptance: September 1, 2021

The *Eustoma* plant of the present disclosure may include the *Eustoma* fusarium wilt resistance locus on chromosome 20 of the *Eustoma* plant deposited under Accession No. FERM BP-22427.

The *Eustoma* plant of the present disclosure also can be produced, for example, by introducing the resistance locus to a *Eustoma* plant. The method for introducing the resistance locus to the *Eustoma* plant is not limited to particular methods, and may be, for example, crossing or embryo culture with the *Eustoma* plant of the present disclosure or a conventionally known genetic engineering procedure. The genetic engineering procedure may be, for example, a method using genetic recombination technology or genome-editing technology. The resistance locus to be introduced may be, for example, the above-described resistance locus. When the resistance locus is introduced by crossing with the Eustoma plant of the present disclosure, the Eustoma plant of the present disclosure to be used in the crossing is preferably homozygous at the resistance locus, for example.

In the *Eustoma* plant of the present disclosure, there is no particular limitation on characteristics other than the *Eustoma* fusarium wilt resistance, such as, for example, morphological characteristics and biological characteristics.

The *Eustoma* plant of the present disclosure may also have other resistance.

### <Part of Eustoma Fusarium Wilt-resistant Eustoma Plant>

In still another aspect, the present disclosure provides a part of a *Eustoma* plant that exhibits resistance to *Eustoma* fusarium wilt. The present disclosure relates to a part of the *Eustoma* fusarium wilt-resistant *Eustoma* plant of the present disclosure. The *Eustoma* plant of the present disclosure includes a *Eustoma* fusarium wilt resistance locus on chromosome 20. The part of the *Eustoma* plant of the present disclosure can exhibit resistance to *Eustoma* fusarium wilt owing to the presence of the *Eustoma* fusarium wilt resistance locus. In the description regarding the *Eustoma* plant of the present disclosure, the *Eustoma* fusarium wilt resistance locus on chromosome 20 should be interpreted as interchangeable with, for example, the *Eustoma* fusarium wilt resistance marker of the present disclosure.

Examples of the part of the plant include plant cells, plant protoplasts, plant cell cultures or tissue cultures from which a plant body can be regenerated, plant calluses, plant clumps, plant cells isolated from the plant or a part of the plant, meristematic cells, flowers, petals, flower buds, pistils, corollas, pollen, leaves, petioles, pith of leaves, cotyledons, ovaries, embryos, ovules, hypocotyls, embryonic sacs, egg cells, microspores, anthers, pistils, flowers, ovaries, cuttings, scions (grafts), rootstocks, roots, tips of roots (root tips), seeds, fruits, trunks, stems, and seedlings. The part of the plant may be an organ, tissue, a cell, or a propagule, for example, and may be any of them. Examples of the organ include petals, corollas, flowers, leaves, seeds, fruits, stems, and roots. The tissue is a part of the organ, for example. The pollen may be mature pollen or immature pollen.

The part of the plant according to the present disclosure can be produced, for example, by obtaining a desired part of the *Eustoma* plant of the present disclosure.

### <Method for Producing Eustoma Fusarium Wilt-resistant Eustoma Plant>

In still another aspect, the present disclosure provides a method for producing a *Eustoma* plant that exhibits resistance to *Eustoma* fusarium wilt. The method for producing a *Eustoma* fusarium wilt-resistant *Eustoma* plant (hereinafter also referred to simply as "production method") according to the present disclosure includes the following steps (a) and (b). Alternatively, the production method of the present disclosure may include only the following step (a).
(a) crossing (crossbreeding) the *Eustoma* fusarium wilt-resistant *Eustoma* plant according to the present disclosure with another *Eustoma* plant.
(b) selecting a *Eustoma* fusarium wilt-resistant *Eustoma* plant from one or more *Eustoma* plants obtained in step (a) or one or more progeny lines thereof

According to the production method of the present disclosure, a *Eustoma* plant that exhibits resistance to *Eustoma* fusarium wilt can be obtained from progeny lines obtained by crossing the *Eustoma* plant of the present disclosure with another *Eustoma* plant. According to the production method of the present disclosure, it is possible to breed a *Eustoma* plant variety that exhibits resistance to *Eustoma* fusarium wilt using any *Eustoma* plants. Accordingly, the production method of the present disclosure can also be referred to as a breeding method or a growing method, for example.

Regarding the resistance locus in the production method of the present disclosure, reference can be made to the description on the resistance locus provided above in connection with the marker of the present disclosure. Also, in the present disclosure, the *Eustoma* fusarium wilt resistance locus should be interpreted as interchangeable with the resistance marker of the present disclosure, and the above description regarding the resistance marker also applies to the *Eustoma* fusarium wilt resistance locus.

In the step (a), the *Eustoma* fusarium wilt-resistant *Eustoma* plant used as a first parent is not limited as long as it is the resistant *Eustoma* plant of the present disclosure. The resistant *Eustoma* plant is preferably the *Eustoma* plant deposited under Accession No. FERM BP-22427 or a progeny line thereof, for example. In step (a), the *Eustoma* fusarium wilt-resistant *Eustoma* plant used as the first parent also can be obtained, for example, by a screening method of the present disclosure as described below. Accordingly, the *Eustoma* fusarium wilt-resistant *Eustoma* plant may be prepared, for example, by selecting it from one or more *Eustoma* plants to be examined in the following step (x), prior to step (a), for example:
(x) selecting the *Eustoma* fusarium wilt-resistant *Eustoma* plant of the present disclosure from one or more *Eustoma* plants to be examined.

In step (x), the *Eustoma* fusarium wilt-resistant *Eustoma* plant can be selected, for example, by directly and/or indirectly evaluating the *Eustoma* fusarium wilt resistance of the one or more *Eustoma* plants to be examined. Regarding the direct evaluation, reference can be made to the above description on the method for evaluating the *Eustoma* fusarium wilt resistance using the severity of the disease.

When the evaluation is performed by indirect evaluation, the selection of the *Eustoma* fusarium wilt-resistant *Eustoma* plant in step (x) can be referred to as a selection of a *Eustoma* plant having the *Eustoma* fusarium wilt resistance locus. Accordingly, step (x) can be performed, for example, by the following steps (x1) and (x2).
(x1) a detection step of detecting the presence or absence of the *Eustoma* fusarium wilt resistance locus on a chromosome of each of the one or more *Eustoma* plants to be examined; and
(x2) a selection step of selecting, from the one or more *Eustoma* plants to be examined, a *Eustoma* plant having the *Eustoma* fusarium wilt resistance locus as a *Eustoma* fusarium wilt-resistant *Eustoma* plant.

As described above, the selection in step (x) is, for example, the selection of a *Eustoma* plant including the resistance locus. Specifically, the resistant *Eustoma* plant can be selected by detecting the resistance locus in each of the one or more *Eustoma* plants to be examined. In step (x2), for example, a *Eustoma* plant to be examined may be selected as the resistant *Eustoma* plant when one of a pair of chromosomes includes the resistance locus or when the pair of chromosomes each include the resistance locus. It is to be noted that the latter is preferable. The detection of the resistance locus in step (x1) can be performed using, for example, as described above in connection with the marker of the present disclosure, (1) an SNP marker, (2) a base sequence including an SNP marker, and (3) a region between sites of two SNP markers, each specifying the *Eustoma* fusarium wilt resistance locus, and any combinations of (1) to (3). As a specific example, in step (x1), for example, a molecular marker such as an SNP associated with the resistance locus on a chromosome of each *Eustoma* plant to be examined is detected, and the presence or absence of the *Eustoma* fusarium wilt resistance locus is detected on the basis of the obtained detection result.

The selection in step (x) will be described with reference to the following specific examples. It is to be noted, however, that the present disclosure is not limited thereto. Regarding the *Eustoma* fusarium wilt resistance locus, reference can be made to the description thereon provided above in connection with the *Eustoma* fusarium wilt resistance marker of the present disclosure.

### (1) Identification by SNP Marker

The selection in step (x) is the selection of a *Eustoma* fusarium wilt-resistant *Eustoma* plant including a *Eustoma* fusarium wilt resistance locus identified by at least one SNP marker selected from the group consisting of Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378. The SNP marker to be selected is not limited to particular SNP markers, and reference can be made to the description on the SNP markers in "(1) Identification by SNP Marker" provided above in connection with the marker of the present disclosure, for example.

### (2) Identification by Base Sequence Including SNP Marker

The selection in step (x) is, for example, the selection of a *Eustoma* plant including a *Eustoma* fusarium wilt resistance locus identified by at least one polynucleotide selected from the group consisting of the polynucleotides (a), (b), (c), (d), (f), (g), (h), (i), (j), (k), and (l). Regarding the polynucleotides (a), (b), (c), (d), (f), (g), (h), (i), (j), (k), and (l), reference can be made to the descriptions thereon in "(2) Identification by Base Sequence Including SNP Marker" provided above in connection with the marker of the present disclosure, for example.

### (3) Identification by Region Between Sites of Two SNP Markers

The selection in step (x) is, for example, the selection of a*Eustoma* plant including a *Eustoma* fusarium wilt resistance locus that includes the base sequence of a region between sites of two SNP markers selected from the group consisting of Egra20_3656645, Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 on the chromosome. Regarding the base sequence of a region between sites of the two SNP markers, reference can be made to the description thereon in "(3) Identification by Region Between Sites of Two SNP Markers" provided above in connection with the *Eustoma* fusarium wilt resistance marker of the present disclosure, for example.

As a specific example, the selection in step (x) is, for example, the selection of a *Eustoma* plant including a *Eustoma* fusarium wilt resistance locus that includes, in the base sequence of the region, at least one SNP marker selected from the group consisting of Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378.

Also, the selection in step (x) may be, for example, the selection of a*Eustoma* fusarium wilt-resistant *Eustoma* plant including a *Eustoma* fusarium wilt resistance locus satisfying the above-described condition (i), (ii), (iii), (iv), (v), (vi), or (vii).

In the case of detection based on the presence or absence of the base sequence of a region between two SNP markers, the base sequence of, for example, the genome (genomic DNA) of the *Eustoma* plant to be examined is decoded in step (x1). Next, in step (x1), detection can be performed by comparing the obtained base sequence with the base sequence of the genome (genomic DNA) of the deposited line and determining whether these base sequences match each other. The decoding of the base sequence can be performed, for example, using a sample containing the genome (genomic DNA) of a *Eustoma* plant to be examined, a reagent for sequencing, and a sequencer. The comparison of the base sequences can be performed, for example, using base sequence analysis software (e.g., the above-described BLAST). In the obtained base sequence, a region to be used for base sequence comparison is the above-described region between sites of two SNP markers. In this manner, in step (x1), a *Eustoma* plant having a base sequence that matches the base sequence of the region between sites of the two SNP markers in the deposited line or satisfies the above-described sequence identity to the same can be specified (identified or detected) as a *Eustoma* plant to be examined including the resistance locus. Then, in step (x2), for example, a *Eustoma* plant to be examined including the resistance locus is selected as the *Eustoma* plant of the present disclosure or a progeny line thereof, i.e., as a *Eustoma* plant that exhibits *Eustoma* fusarium wilt resistance.

The chromosome to be subjected to the detection of the presence or absence of the *Eustoma* fusarium wilt resistance locus is preferably chromosome 20.

In step (a), a *Eustoma* plant to be used as the other parent is not limited to particular *Eustoma* plants, and may be, for example, a known *Eustoma* plant having or not having *Eustoma* fusarium wilt resistance, a *Eustoma* plant having or not having other resistance, or the *Eustoma* fusarium wilt-resistant *Eustoma* plant of the present disclosure.

In step (a), the method for crossing the *Eustoma* fusarium wilt-resistant *Eustoma* plant with another *Eustoma* plant is not limited to particular methods, and known methods can be employed.

In step (b), *Eustoma* plants from which a *Eustoma* fusarium wilt-resistant *Eustoma* plant is selected may be *Eustoma* plants obtained in the step (a) or progeny lines further obtained therefrom, for example. Specifically, the *Eustoma* plants from which the *Eustoma* fusarium wilt-resistant *Eustoma* plant is selected may be, for example, F1 *Eustoma* plants obtained by the crossing in step (a) or progeny lines thereof. The progeny lines may be, for example, selfed progenies or backcross progenies of the F1 *Eustoma* plants obtained by the crossing in step (a) or may be *Eustoma* plants obtained by crossing the F1 *Eustoma* plants with other *Eustoma* plants.

In step (b), the selection of a *Eustoma* fusarium wilt-resistant *Eustoma* plant can be performed, for example, by examining the *Eustoma* fusarium wilt resistance directly or indirectly.

In step (b), the direct examination can be performed by evaluating the *Eustoma* fusarium wilt resistance of the obtained F1 *Eustoma* plants or progeny lines thereof, for example, on the basis of the above-described severity of the disease. Specifically, for example, the direct examination can be performed, for example, by inoculating the F1 *Eustoma* plants or the progeny lines thereof with a *Eustoma* fusarium wilt pathogenic fungus and evaluating the *Eustoma* fusarium wilt resistance on the basis of the proportion of individuals with a severity of 1 in all the surveyed individuals. In this case, for example, the F1 *Eustoma* plants or the progeny lines can be selected as *Eustoma* fusarium wilt-resistant *Eustoma* plants when they satisfy the predetermined evaluation criterion.

In step (b), the selection by the indirect examination can be performed by the following steps (b1) and (b2), for example:
(b1) a detection step of detecting the presence or absence of a *Eustoma* fusarium wilt resistance locus on a chromosome of each of the one or more *Eustoma* plants obtained in step (a) or one or more progeny lines thereof; and
(b2) a selection step of selecting, from the one or more *Eustoma* plants obtained in step (a) or one or more progeny lines thereof, a *Eustoma* plant having the *Eustoma* fusarium wilt resistance locus as a *Eustoma* fusarium wilt-resistant *Eustoma* plant.

The selection of the *Eustoma* fusarium wilt-resistant*Eustoma* plants by the indirect examination in step (b) can be performed, for example, in the same manner as described above regarding step (x), namely, by detecting the presence or absence of the *Eustoma* fusarium wilt resistance locus, and more specifically, by detecting the presence or absence of the *Eustoma* fusarium wilt resistance locus using the above-described molecular marker.

The production method of the present disclosure preferably further includes growing the *Eustoma* fusarium wilt-resistant *Eustoma* plants selected in step (b).

The *Eustoma* plants or progeny lines thereof demonstrated to be *Eustoma* fusarium wilt resistance in the above-described manner can be selected as *Eustoma* fusarium wilt-resistant *Eustoma* plants.

The production method of the present disclosure may further include the step of collecting seeds from the progeny lines obtained by the crossing.

Although the production method of the present disclosure includes steps (a) and (b), the production method of the present disclosure is not limited thereto and may include only step (a). In this case, the *Eustoma* plant of the present disclosure to be used in step (a) is preferably homozygous at the resistance locus.

### <Screening Method for Eustoma Fusarium Wilt-resistant Eustoma Plant>

In still another aspect, the present disclosure provides a screening method for a *Eustoma* plant that exhibits resistance to *Eustoma* fusarium wilt. The screening method of the present disclosure is a screening method for a *Eustoma* fusarium wilt-resistant *Eustoma* plant, including: a selection step of selecting, from one or more *Eustoma* plants to be examined, a *Eustoma* plant that includes a *Eustoma* fusarium wilt resistance locus on chromosome 20 as a *Eustoma* fusarium wilt-resistant *Eustoma* plant. According to the screening method of the present disclosure, a *Eustoma* plant that may exhibit resistance to *Eustoma* fusarium wilt can be selected through screening. According to the screening method of the present disclosure, a parent for producing a *Eustoma* fusarium wilt-resistant *Eustoma* plant by crossing can be selected through screening.

Regarding the selection of the *Eustoma* fusarium wilt-resistant *Eustoma* plant, reference can be made to the description on step (x) provided above in connection with the method for producing a *Eustoma* fusarium wilt-resistant *Eustoma* plant of the present disclosure, for example.

### <Method for Detecting Eustoma Fusarium Wilt Resistance of Eustoma Plant>

In still another aspect, the present disclosure provides a method for detecting *Eustoma* fusarium wilt resistance of a *Eustoma* plant. The detection method of the present disclosure is a method for detecting *Eustoma* fusarium wilt resistance of a *Eustoma* plant, including: a detection step of detecting a *Eustoma* fusarium wilt resistance locus on chromosome 20 in a *Eustoma* plant to be examined. The detection method of the present disclosure can detect whether a *Eustoma* plant of interest exhibits resistance to *Eustoma* fusarium wilt. The detection method of the present disclosure for detecting *Eustoma* fusarium wilt resistance of a *Eustoma* plant also can be referred to as a screening method for *Eustoma* fusarium wilt resistance in a *Eustoma* plant.

Regarding the detection of the *Eustoma* fusarium wilt-resistant *Eustoma* plant, reference can be made to the description on the indirect selection in step (x), i.e., the description on step (x1), provided above in connection with the production method of the present disclosure for producing a *Eustoma* fusarium wilt-resistant *Eustoma* plant, for example. As a specific example, in the detection step, for example, a molecular marker such as an SNP associated with the resistance locus on a chromosome of a *Eustoma* plant to be examined is detected, and the presence or absence of the *Eustoma* fusarium wilt resistance locus is detected on the basis of the obtained detection result. The chromosome to be subjected to the detection is preferably chromosome 20.

### <Method for Conferring Eustoma Fusarium Wilt Resistance to Eustoma Plant>

In still another aspect, the present disclosure provides a method for conferring *Eustoma* fusarium wilt resistance to a *Eustoma* plant. The conferring method of the present disclosure for conferring *Eustoma* fusarium wilt resistance to a *Eustoma* plant includes an introduction step of introducing a *Eustoma* fusarium wilt resistance locus on chromosome 20 to a *Eustoma* plant. The conferring method of the present disclosure can confer *Eustoma* fusarium wilt resistance to a *Eustoma* plant by introducing the *Eustoma* fusarium wilt resistance locus on chromosome 20, i.e., the *Eustoma* fusarium wilt resistance marker of the present disclosure, to the *Eustoma* plant.

In the introduction step, the method for introducing the *Eustoma* fusarium wilt resistance locus on chromosome 20 is not limited to particular methods. The introduction method may be, for example, crossing or embryo culture with the resistant *Eustoma* plant or a conventionally known genetic engineering procedure. The resistance locus to be introduced may be, for example, the above-described resistance locus. When the resistance locus is introduced by crossing with the resistant Eustoma plant, the resistant Eustoma plant is preferably homozygous at the resistance locus, for example.

### <Method for Distinguishing Deposited Line or Progeny Line>

In still another aspect, the present disclosure provides a method capable of distinguishing the deposited line identified by FERM BP-22427 or a progeny line thereof. The distinguishing method of the present disclosure is a method for distinguishing the deposited line identified by FERM BP-22427 or a progeny line thereof, including an SNP detection step of detecting at least one SNP selected from the group consisting of SNPs 1 to 65 and 66 in a *Eustoma* plant to be examined. The distinguishing method of the present disclosure can distinguish whether the *Eustoma* plant to be examined is the deposited line or a progeny line thereof. The term "distinguishing" can also be referred to as, for example, assessment, appraisal, detection, determination, discrimination, or evaluation. Accordingly, the distinguishing method of the present disclosure can also be referred to as, for example, an assessment method, an appraisal method, a detection method, a determination method, a discrimination method, or an evaluation method.

In the SNP detection step, detection of each SNP can be performed by an ordinary method on the basis of the base sequence of a polynucleotide including each SNP. Regarding polynucleotides including SNPs 1 to 66, respectively, reference can be made to Example 2 as described below.

The number of SNPs to be detected in the SNP detection step is not limited to particular values, and for example, any one or any two or more of SNPs 1 to 66 may be detected or all of them may be detected. By detecting a relatively larger number of SNPs in the *Eustoma* plant to be examined in the SNP detection step, the distinguishing method of the present disclosure can distinguish whether the *Eustoma* plant to be examined is the deposited line or a progeny line thereof with higher accuracy.

The distinguishing method of the present disclosure may further include an evaluation step of evaluating whether the *Eustoma* plant to be examined is the deposited line or a progeny line thereof by comparing an SNP(s) of the *Eustoma* plant to be examined with a reference SNP(s) corresponding thereto. Reference SNPs are SNPs 1 to 66 of the deposited line. As a specific example, in the evaluation step, the *Eustoma* plant to be examined can be evaluated as being the deposited line or a progeny line thereof, for example, when the SNP(s) of the *Eustoma* plant to be examined match the reference SNP(s). On the other hand, in the evaluation step, the *Eustoma* plant to be examined can be evaluated as not being the deposited line or a progeny line thereof, for example, when the SNP(s) of the *Eustoma* plant to be examined does not match the reference SNP(s).

In the case where a plurality of SNPs is used for evaluation in the evaluation step, the evaluation may be made on the basis of, for example, the match rate between SNPs (evaluation target SNPs) of the *Eustoma* plant to be examined and the reference SNPs. The match rate can be calculated as the proportion (T/A × 100 (%)) of the number of SNPs (T) of the *Eustoma* plant to be examined that have matched the reference SNPs in the number (A) of evaluation target SNPs. In this case, in the distinguishing method of the present disclosure, the SNP match rate may be calculated from the SNPs (evaluation target SNPs) of the *Eustoma* plant to be examined and the reference SNPs, prior to the evaluation step. Then, in the evaluation step, for example, when the match rate between the evaluation target SNPs of the *Eustoma* plant to be examined and the reference SNPs satisfies a predetermined percentage, the *Eustoma* plant to be examined is evaluated as being the deposited line or a progeny line thereof. On the other hand, in the evaluation step, for example, when the match rate between the evaluation target SNPs in the *Eustoma* plant to be examined and the reference SNPs does not satisfy the predetermined percentage, the *Eustoma* plant to be examined is evaluated as not being the deposited line or a progeny line thereof. The predetermined percentage is, for example, 1% or more, 3% or more, 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, 96% or more, 97% or more, 98% or more, or 99% or more.

In the case where a plurality of SNPs is used for evaluation in the evaluation step, the evaluation may be made on the basis of, for example, the number of SNPs that have matched each other between the SNPs (evaluation target SNPs) of the *Eustoma* plant to be examined and the reference SNPs. In this case, in the distinguishing method of the present disclosure, the number of SNPs that have matched each other may be determined from the SNPs (evaluation target SNPs) of the *Eustoma* plant to be examined and the reference SNPs, prior to the evaluation step. Then, in the evaluation step, for example, when the number of SNPs that have matched each other between the SNPs (evaluation target SNPs) of the *Eustoma* plant to be examined and the reference SNPs satisfies a predetermined value, the *Eustoma* plant to be examined is evaluated as being the deposited line or a progeny line thereof. On the other hand, in the evaluation step, for example, when the number of SNPs that have matched each other between the SNPs (evaluation target SNPs) of the *Eustoma* plant to be examined and the reference SNPs satisfies a predetermined value the *Eustoma* plant to be examined is evaluated as not being the deposited line or a progeny line thereof. The predetermined value is, for example, 2 or more, 3 or more, 4 or more, 5 or more, 6 or more, 7 or more, 8 or more, 9 or more, 10 or more, 15 or more, 20 or more, 25 or more, 30 or more, 35 or more, 40 or more, 45 or more, 50 or more, 55 or more, 60 or more, 61 or more, 62 or more, 63 or more, 64 or more, or 65 or more. SNPs 1 to 66 are SNPs that are haploid near the resistance locus on chromosome 20 in the deposited line. Thus, in general, the *Eustoma* plant to be examined is determined to be the deposited line or a progeny line thereof when two or more of SNPs 1 to 66 match each other. In the evaluation step, a *Eustoma* plant to be examined may be evaluated as being the deposited line or a progeny line thereof when any one or more of evaluation target SNPs 2 to 5 of the *Eustoma* plant to be examined match any one or more of SNPs 2 to 5 in the reference SNPs and the number of SNPs that have matched each other between the evaluation target SNPs of the *Eustoma* plant to be examined and the reference SNPs satisfies a predetermined value. In this case, regarding the predetermined value, reference can be made to the above description thereon, for example. Examples:

The present disclosure will be described specifically below with reference to examples. It is to be noted, however, that the present invention is by no means limited to embodiments described in the following examples.

### Example 1

The present example confirmed that a novel *Eustoma* fusarium wilt-resistant *Eustoma* plant exhibits resistance to a *Eustoma* fusarium wilt pathogenic fungus, and also identified a novel *Eustoma* fusarium wilt resistance locus.

In order to select *Eustoma* plants resistant to the *Eustoma* fusarium wilt pathogenic fungus *(Fusarium oxysporum*), various *Eustoma* plants owned by TAKII & CO., LTD. were subjected to an inoculation test using the fusarium wilt pathogenic fungus according to a method disclosed in Non-Patent Literature 1. Specifically, the inoculation test using the fusarium wilt pathogenic fungus was performed in the following manner.

### (1) Plants Subjected to Test

Seeds of *Eustoma* plants to be subjected to the inoculation test were sown in a 288-well cell tray filled with seeding culture soil manufactured by TAKII & CO., LTD. and were grown for 12 weeks. After the above-described growing process, the *Eustoma* plants were transplanted to a 55-well cell tray filled with seeding culture soil manufactured by TAKII & CO., LTD.

### (2) Inoculation Test

The *Eustoma* fusarium wilt pathogenic fungus (*Fusarium oxysporum*) used in the test was derived from *Eustoma* fusarium wilt-susceptible *Eustoma* plants that had spontaneously developed fusarium wilt in a *Eustoma* cultivation farm in Yamagata, Japan. The following inoculation test confirmed that the fusarium wilt pathogenic fungus was a pathogen capable of infecting the above-described susceptible *Eustoma* plants.

The fusarium wilt pathogenic fungus was shake-cultured in a flask containing a sucrose-added potato decoction liquid medium having the following composition at 25°C for four days. After the culture, the liquid medium was filtered through a double gauze fabric, and the spore concentration was adjusted to 1 × 10⁷ spores/ml. Thereafter, the thus-obtained preparation was used as an inoculum in the inoculation test. *Eustoma* plants to be subjected to the inoculation test were the *Eustoma* plants obtained in the item (1) of Example 1. In the inoculation test, the inoculum was supplied to the *Eustoma* plants (20 ml per individual) by pouring the inoculum to the soil on the same day as the *Eustoma* plants were transplanted in the item (1) of Example 1, and thereafter, the *Eustoma* plants were grown for 3 weeks at 25°C with a day length of 12 hours. After the above-described growing process, the severity of the disease was evaluated.

### (Sucrose-added Potato Decoction Liquid Medium)

| | |
|---|---|
| Potato (300.0 g of decoction) | 1000 mL |
| Ca(NO₃)₂·4H₂O | 0.5 g |
| Na₂HPO₄·12H₂O | 2.0 g |
| Peptone | 5.0 g |
| Sucrose | 20.0 g |
| pH | 6.8 to 7.0 |

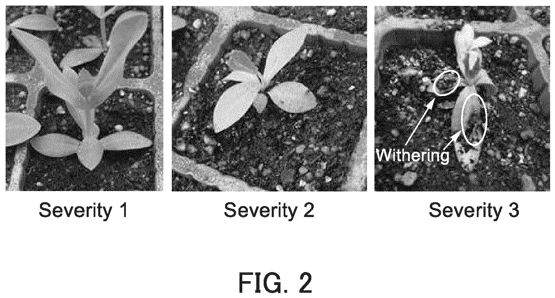

The severity was evaluated according to the following three evaluation criteria. Representative examples of the respective disease indexes are shown in FIG. 2.
Severity 1: No disease symptoms were observed.
Severity 2: The plant body partially wilted.
Severity 3: The plant body entirely wilted or withered.

### (3) Establishment of Deposited Line

As a result of the inoculation test, a novel fusarium wilt-resistant *Eustoma* line that exhibits a high degree of fusarium wilt resistance was obtained. Hereinafter, this fusarium wilt-resistant *Eustoma* plant is referred to as a "parental line". The parental line was deposited under Accession No. FERM BP-22427. Hereinafter, the parental line is also referred to as a "deposited line".

### (4) Identification of Resistance Locus

The deposit line having fusarium wilt resistance (hereinafter also referred to as "PR line") was crossed with a *Eustoma* fusarium wilt-susceptible (sensitive) *Eustoma* plant "Mink" (TAKII & CO., LTD., hereinafter also referred to as "PS line"), thereby obtaining the F1 generation. Next, F2 generation seeds were obtained by selfing the F1 generation. By selfing the F2 generation plants, 192 individuals of F3 generation seeds (hereinafter also referred to as "192 lines") were obtained.

Genomic DNA was extracted from each of the 192 lines using a PureGene DNA extraction kit (Gentra). The extracted genomic DNA was analyzed by the GRAS-Di method. In the above analysis, the above-described Egra_v1.0 was used as a reference sequence to obtain information on single nucleotide polymorphisms (hereafter referred to as SNPs) in the entire genome.

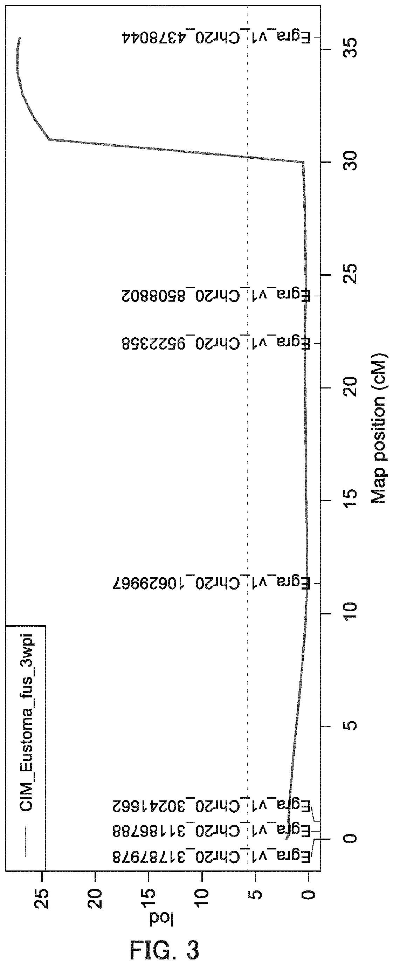

Also, the 192 lines were subjected to an inoculation test. Specifically, the inoculation test was performed in the same manner as the inoculation test described in the item (2) of Example 1, except that the 192 lines were used as the plants to be subjected to the inoculation test. Evaluation of the severity showed that, in 111 lines (10 to 15 individuals per each line), the severity could be evaluated in 10 or more individuals and missing SNP information was not more than 20%. QTL analysis was performed on the basis of the results of the inoculation test for the 111 lines and the whole genome SNP information obtained by the GRAS-Di method. The QTL analysis was performed using R/qtl. The results thereof are shown in FIG 3.

FIG. 3 is a graph showing the LOD score of a chromosome. In FIG. 3, the horizontal axis indicates the location on the chromosome and the SNP marker, and the vertical axis indicates the LOD score. Specifically, in FIG 3, the expression like "Egra_v1_ChrX_Y" means the Yth base (SNP) on chromosome X. The dashed line in FIG. 3 indicates the threshold of the LOD score. As can be seen in FIG. 1, one QTL region with a peak LOD score calculated by composite interval mapping (CIM) being 25 was detected near 3 to 5 Mb on chromosome 20.

In order to obtain SNP information between the PR line and the PS line, the PR line and the PS line were re-sequenced. On the basis of the SNP information obtained by the re-sequencing and the QTL region, five SNP markers shown in 1 above were constructed. SNPs 1 to 5 shown in Table 1 correspond to SNP (e), SNP (a), SNP (b), SNP (c), and SNP (d), respectively. In Table 1, the base [N₁/N₂] in parentheses indicates an SNP, where N₁ is a polymorphism (base) of the PS line, i.e., a susceptible-type polymorphism and N₂ is a polymorphism (base) of the PR line, i.e., an disease resistance-type polymorphism.

Next, 3000 individuals of the F2 generation and 2300 individuals of the F3 generation were subjected to an inoculation test. Specifically, the inoculation test was performed in the same manner as the inoculation test described in the item (2) of Example 1, except that the F2 and F3 generations were used as the plants to be subjected to the inoculation test. Then, the resistance of each individual against the *Eustoma* fusarium wilt pathogenic fungus was evaluated on the basis of the disease index (phenotype). Also, DNA was extracted from each of the plants subjected to the test, and SNP analysis was performed using the above-described SNP markers. The results thereof are shown in Table 3 below.

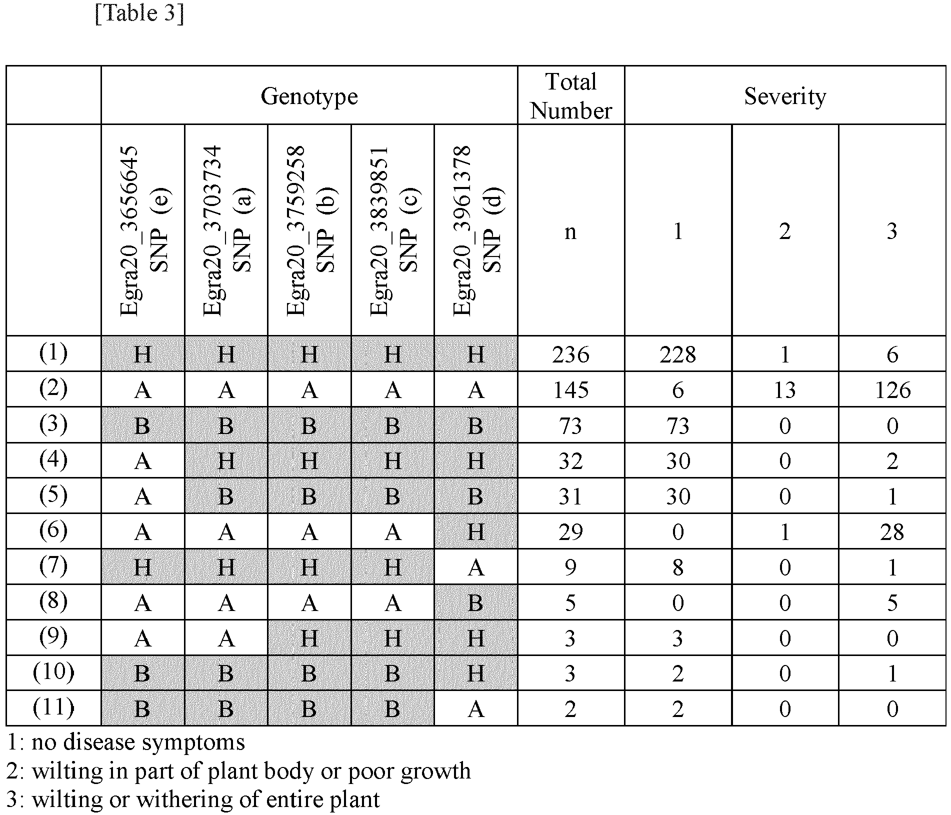

Table 3 above shows the genotype and the severity of the disease in the *Eustoma* plants. In Table 3, "A" indicates that the plant was susceptible homozygous for the SNP marker, "B" indicates that the plant was resistant homozygous for the SNP marker, and "H" indicates that the plant was heterozygous for the SNP marker. In Table 3, "B" and "H" are shaded. As can be seen from Table 3 above, the F2 and F3 generations exhibited resistance to the *Eustoma* fusarium wilt pathogenic fungus when they were resistant homozygous (B) or heterozygous (H) for the SNP markers Egra20_3703734, Egra20_3759258, Egra20_3839851, and Egra20_3961378. These results showed that the resistance genes against the *Eustoma* fusarium wilt pathogenic fungus are located between 3,703,734 bp and 3,961,378 bp on chromosome 20, i.e., this region is a region where the *Eustoma* fusarium wilt resistance locus is located. Further, it was strongly suggested that the resistance genes against the Eustoma fusarium wilt pathogenic fungus are present between 3,703,834 bp and 3,961,278 bp (roughly between SNP (a) and SNP (d)) on chromosome 20, since the plants exhibited resistance when the genotype for Egra20_3961378 was A, i.e., when the genotypes for the SNPs from the upstream was (7) HHHHA or (11) BBBBA, and when genotype for Egra20_3703734 was A, i.e., when the genotypes for the SNPs from the upstream was (9) AAHHH, albeit the number of individuals that support the suggestion was small, and the SNPs were not present in a region extending from 3,703,734 bp to 100 bases downstream therefrom and a region extending from 3,961,378 bp to 100 bases upstream therefrom on chromosome 20. Moreover, the mode of inheritance of the F2 and F3 generations strongly suggested that the resistance genes against the *Eustoma* fusarium wilt pathogenic fungus are single and dominant genes.

### (5) SNP Analysis in Commercially Available Cultivars

The resistance locus of the novel fusarium wilt-resistant *Eustoma* plant was confirmed not to be present in other *Eustoma* plants.

DNA was extracted from each of the *Eustoma* plants listed in Table 4 below, and SNP analysis was performed using SNP markers including Egra20_3759258 and Egra20_3839851, which are strongly linked to the resistance genes against the *Eustoma* fusarium wilt pathogenic fungus. The results thereof are shown in FIG. 4 below. In Table 4 below, "A" indicates that the plant was susceptible homozygous for the SNP marker, and "B" indicates that the plant was resistant homozygous for the SNP marker. In Table 4 below, "B" is shaded. As can be seen from Table 4 below, the PR line was resistant homozygous for all the SNP markers Egra20_3703734, Egra20_3759258, Egra20_3839851, and Egra20_3961378. In contrast, the *Eustoma* plants excluding the PR line were susceptible homozygous for all the SNP markers Egra20_3703734, Egra20_3759258, Egra20_3839851, and Egra20_3961378. These results showed that the above-described SNPs are specific to the PR line.

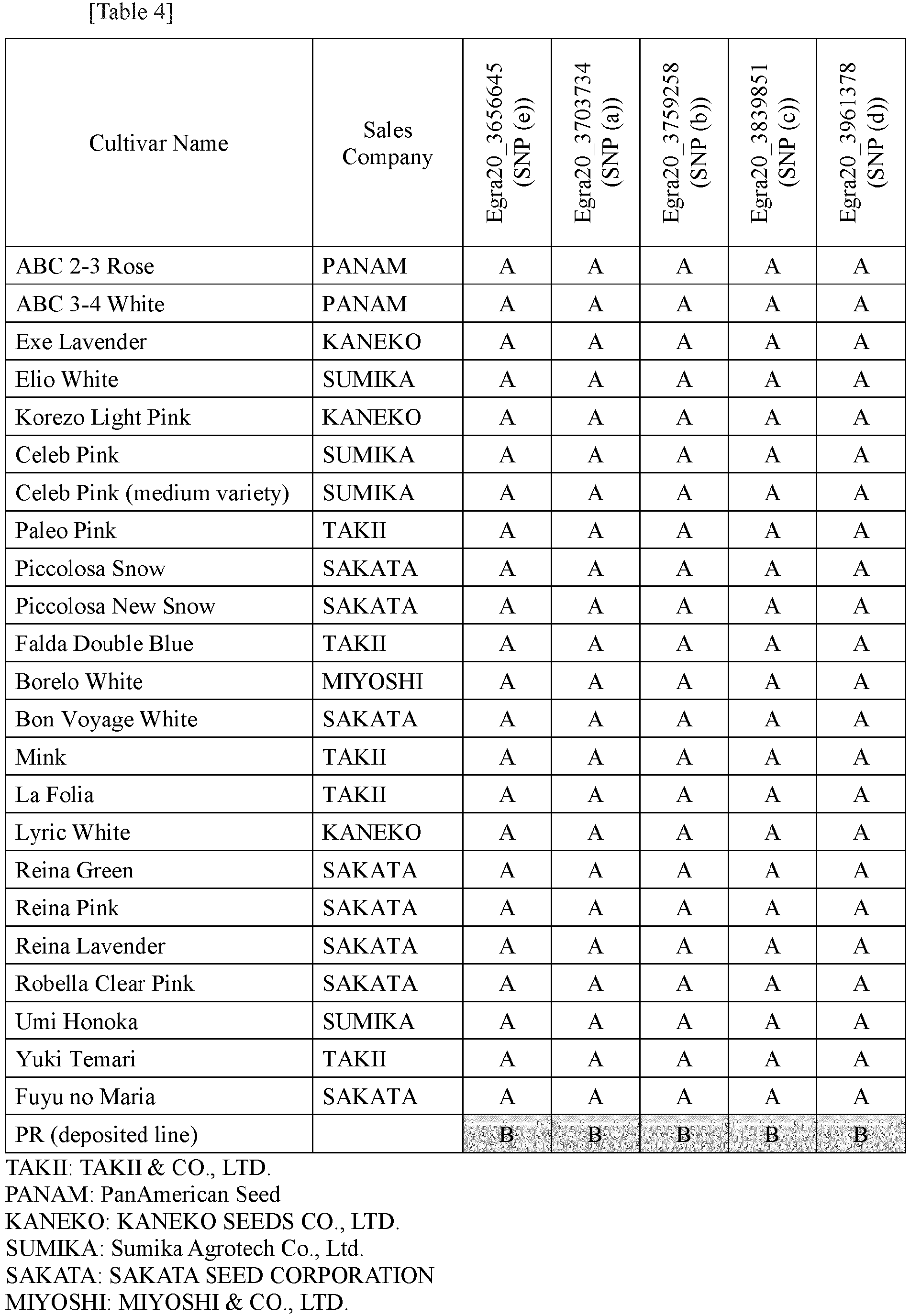

These results showed that a *Eustoma* plant with *Eustoma* fusarium wilt resistance can be obtained by crossbreeding in which the PR line is used as a first parent and a *Eustoma* fusarium wilt susceptible *Eustoma* plant is used as a second parent, and the resistance locus of the present disclosure is inherited dominantly. These results also showed that the *Eustoma* fusarium wilt-resistant *Eustoma* plant of the present disclosure can be selected using the above-described SNP markers.

### Example 2

SNP markers capable of distinguishing the deposited line and progeny lines thereof were identified.

### (1) SNP Markers of Deposited Line

The deposited line (PR line) and the following commercially available *Eustoma* cultivars were used to search for SNP markers specific to the deposited line.
(TAKII & CO., LTD.) Paleo Pink, Falda Double Blue, Mink, La Folia, Yuki Temari, and Maiko
(KANEKO SEEDS CO., LTD.) Exe Lavender and Korezo Light Pink
(Sumika Agrotech Co., Ltd.) Celeb Pink and Umi Honoka
(SAKATA SEED CORPORATION) Robella Clear Pink
(MIYOSHI & CO., LTD.) Borelo White

Specifically, for the deposited line (PR line) and the above-described commercially available *Eustoma* cultivars, SNPs listed in Tables 4A to 4F below were analyzed. In Tables 2A and 2B above, the base [N₁/N₂] in parentheses indicates an SNP, where N₁ is a polymorphism (base) of a line other than the deposited line and N₂ is a polymorphism (base) of the deposited line. The results thereof are shown in Tables 5A to 5C below. In these tables below, "A" indicates that the plant homozygously carried polymorphisms of a line other than the deposited line, and "B" indicates that the plant homozygously carried polymorphisms of the deposited line. As can be seen in Tables 5A to 5C, the results showed that, in the *Eustoma* plants, not only the above-described SNPs 1 to 5 but also SNPs 6 to 66 are SNP markers specific to the deposited line. These results thus strongly suggested that it is possible to distinguish the deposited line and progeny lines thereof by using the detection of SNPs 6 to 66 in combination with the detection of the above-described resistance locus.

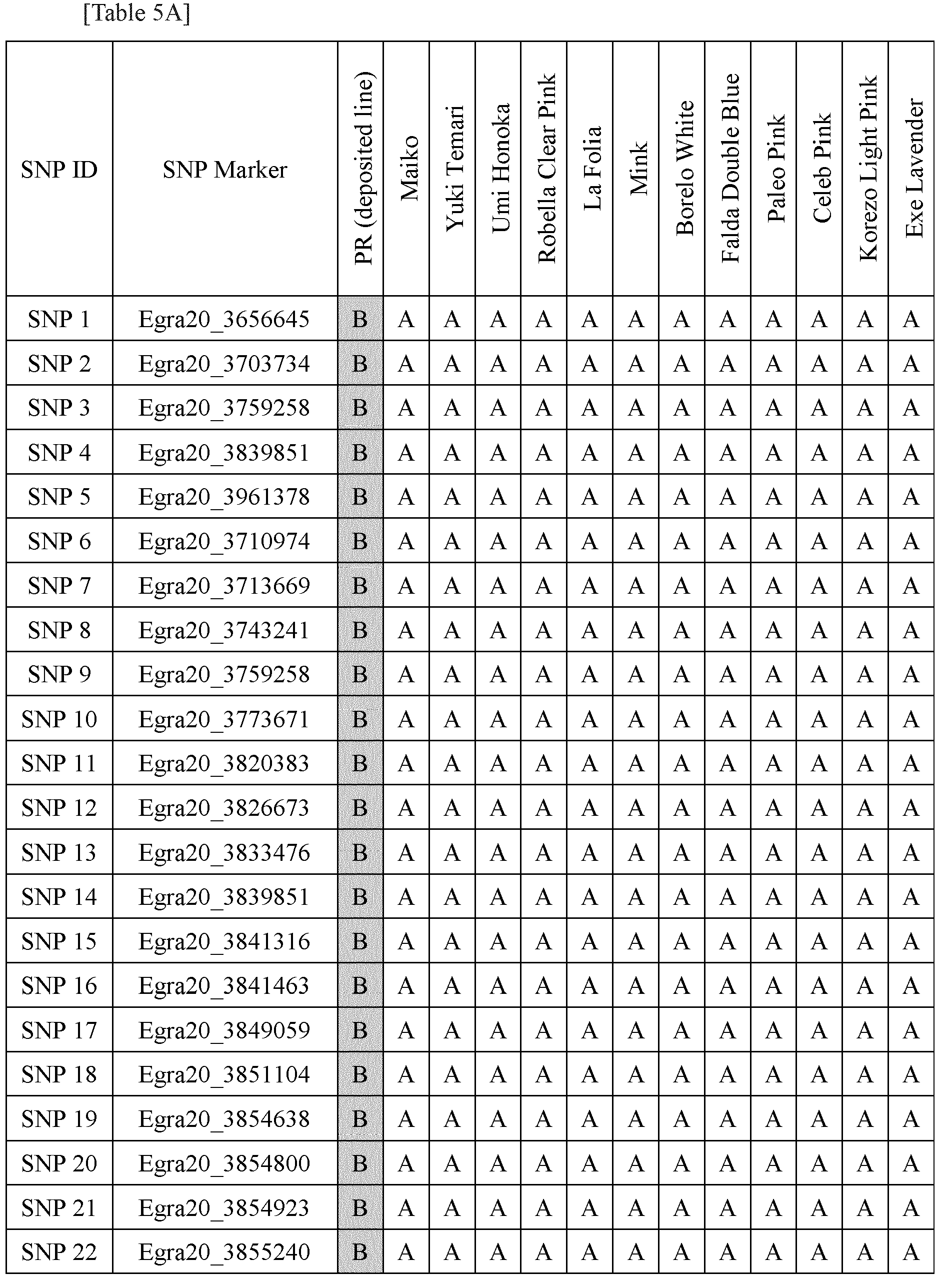

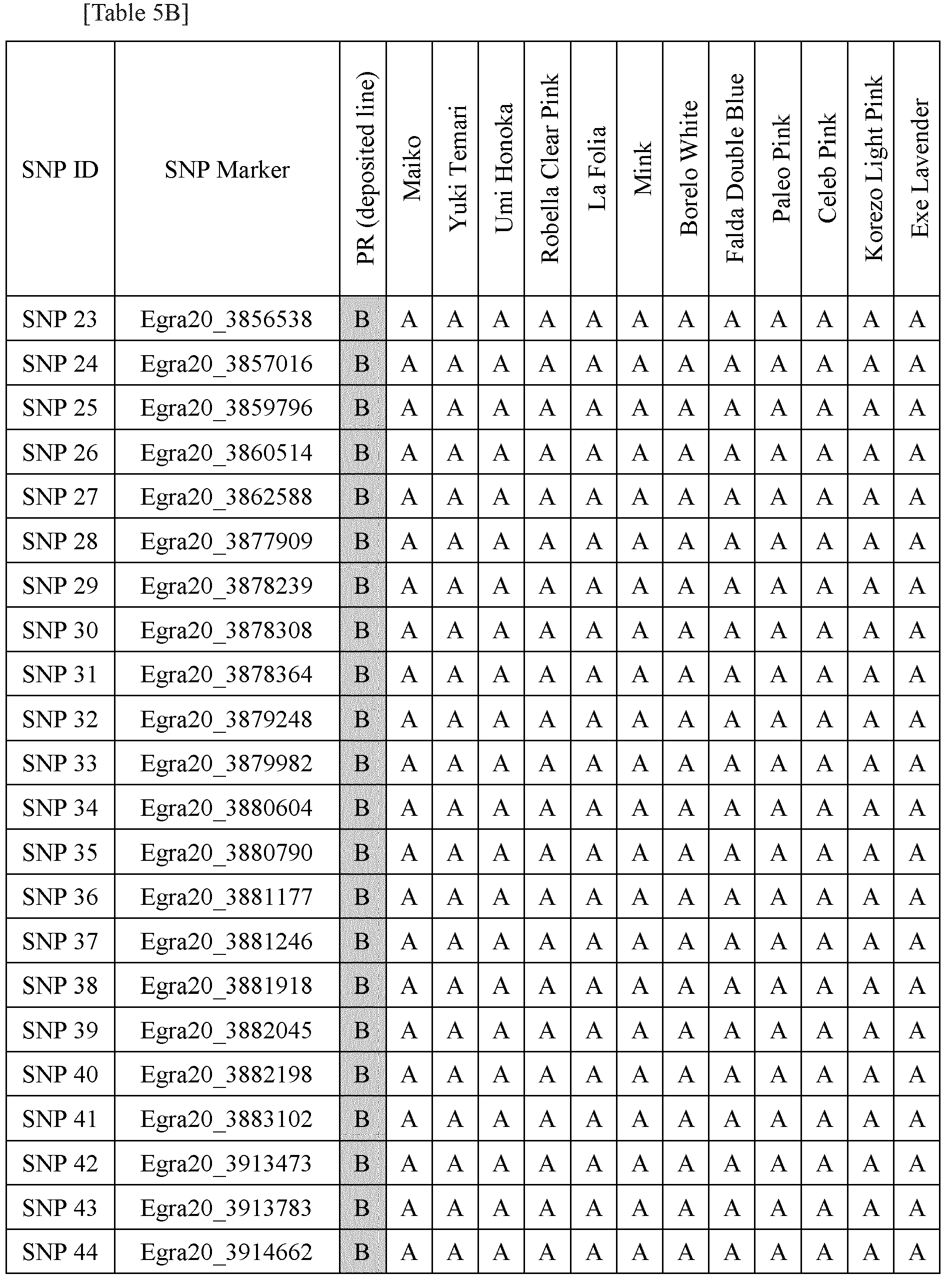

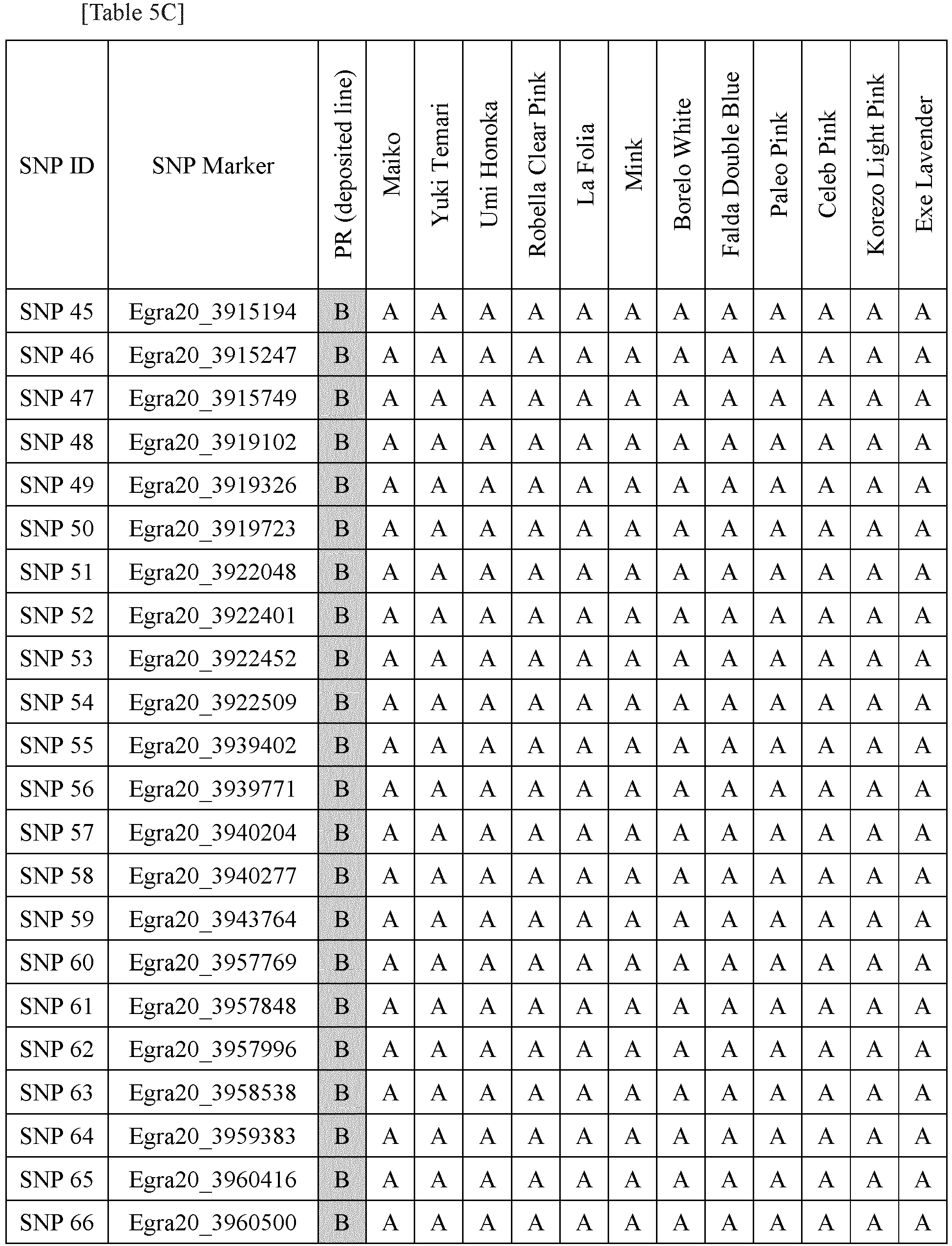

### Example 4

Regarding the novel fusarium wilt-resistant *Eustoma* plant, a specific location of the *Eustoma* fusarium wilt resistance locus was identified.

The deposited line was crossed with the PS line, whereby the F2 generation was obtained (1834 individuals). Regarding the thus-obtained F2 generation, polymorphisms (genotypes) of SNP (b) (Egra20_3759258), SNP (c) (Egra20_3839851), SNP (k) (Egra20_3914662), and SNP (l) (Egra20_3939402) were determined. As a result, 16 recombinant individuals in total were obtained, including 3 recombinant individuals resulting from the recombination between SNP (b) and SNP (f), 1 recombinant individual resulting from the recombination between SNP 4 and SNP 44, and 12 recombinant individuals resulting from the recombination between SNP 44 and SNP 55. From the occurrence ratio of each recombinant individual, the genetic distances between the markers SNP 3 and SNP 4, between the markers SNP 4 and SNP 44, and between the markers SNP 44 and SNP 55 were estimated to be 0.16 cM, 0.06 cM, and 0.65 cM, respectively. Selfed seeds were obtained from these recombinant individuals, and some of the lines were subjected to an inoculation test. For the individuals subjected to the inoculation test, the polymorphisms (genotypes) of SNP (b), SNP (c), SNP (f), SNP (g), SNP (h), SNP (i), SNP (j), SNP (k), and SNP (l) were determined. The results thereof are shown in Table 6 below. In the table below, "A" indicates that the plant homozygously carried polymorphisms of a line other than the deposited line, "B" indicates that the plant homozygously carried polymorphisms of the deposited line, and "H" indicates that the plant heterozygously carried a polymorphism of a line other than the deposited line and the polymorphism of the deposited line.

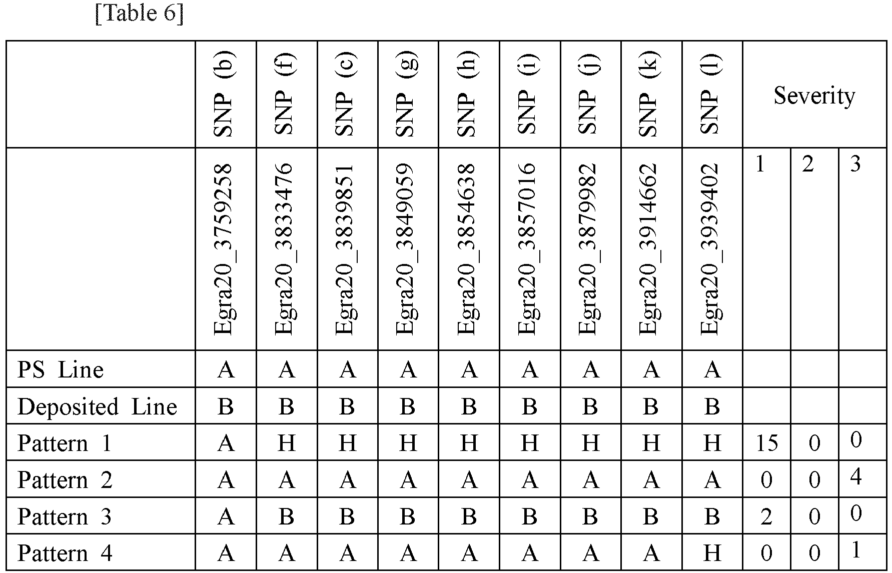

As can be seen from Table 6 above, it was found that the *Eustoma* fusarium wilt resistance locus was located between SNP (b) and SNP (l), in particular, between SNP (f) and SNP (k). Also, the fact that the genetic distance between SNP (b) and SNP (l) is 0.87 cM demonstrates that SNP (b), SNP (c), SNP (f), SNP (g), SNP (h), SNP (i), SNP (j), SNP (k), and SNP (l) are all strongly linked to the *Eustoma* fusarium wilt resistance locus, and, accordingly, the *Eustoma* fusarium wilt resistance locus of the present disclosure can be detected by detecting any one or more of them.

While the present disclosure has been described above with reference to exemplary embodiments and examples, the present disclosure is by no means limited thereto. Various changes and modifications that may become apparent to those skilled in the art may be made in the configuration and specifics of the present disclosure without departing from the scope of the present disclosure.

The present application is based upon and claims the benefit of priority from Japanese patent application No. 2021-173508 filed on October 22, 2021, the disclosure of which is incorporated herein in its entirety by reference.
locus on chromosome 20.

### <Supplementary Notes>

The whole or part of the exemplary embodiments and examples disclosed above can be described as, but not limited to, the following Supplementary Notes.

### <Eustoma Fusarium Wilt-resistant Eustoma Plant>

### (Supplementary Note 1)

A *Eustoma* fusarium wilt-resistant *Eustoma* plant including:
a *Eustoma* fusarium wilt resistance locus on chromosome 20.

### (Supplementary Note 2)

The *Eustoma* fusarium wilt-resistant *Eustoma* plant according to Supplementary Note 1, wherein;
the *Eustoma* fusarium wilt resistance locus is identified by an SNP marker selected from the group consisting of Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 and/or an SNP marker selected from the group consisting of SNPs 1 to 65 and 66.

### (Supplementary Note 3)

The *Eustoma* fusarium wilt-resistant *Eustoma* plant according to Supplementary Note 2, wherein;
the *Eustoma* fusarium wilt resistance locus is identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402.

### (Supplementary Note 4)

The *Eustoma* plant according to any one of Supplementary Notes 1 to 3, wherein;
the *Eustoma* fusarium wilt resistance locus is identified by a polynucleotide selected from the group consisting of polynucleotides (a) to (d) and (f) to (l) below and/or a polynucleotide selected from the group consisting of polynucleotides including SNPs 1 to 65 and 66:
(a) a polynucleotide (a1), (a2), or (a3) below:
   (a1) a polynucleotide that consists of a base sequence of SEQ ID NO: 1;
   (a2) a polynucleotide that consists of a base sequence corresponding to the base sequence of the polynucleotide (a1) in which 1 to 20 bases are deleted, substituted, inserted, and/or added with a 101st base (T) being conserved and has a function equivalent to that of the polynucleotide (a1) regarding *Eustoma* fusarium wilt resistance in the *Eustoma* fusarium wilt resistance locus; or
   (a3) a polynucleotide that consists of a base sequence having at least 90% identity to the base sequence of the polynucleotide (a1) with the 101st base (T) of the polynucleotide (a1) being conserved and has a function equivalent to that of the polynucleotide (a1) regarding *Eustoma* fusarium wilt resistance in the *Eustoma* fusarium wilt resistance locus;
(b) a polynucleotide (b1), (b2), or (b3) below:
   (b1) a polynucleotide that consists of a base sequence of SEQ ID NO: 2;
   (b2) a polynucleotide that consists of a base sequence corresponding to the base sequence of the polynucleotide (b1) in which 1 to 20 bases are deleted, substituted, inserted, and/or added with a 101st base (A) being conserved and has a function equivalent to that of the polynucleotide (b1) regarding *Eustoma* fusarium wilt resistance in the *Eustoma* fusarium wilt resistance locus; or
   (b3) a polynucleotide that consists of a base sequence having at least 90% identity to the base sequence of the polynucleotide (b1) with the 101st base (A) of the polynucleotide (b1) being conserved and has a function equivalent to that of the polynucleotide (b1) regarding *Eustoma* fusarium wilt resistance in the *Eustoma* fusarium wilt resistance locus;
(c) a polynucleotide (c1), (c2), or (c3) below:
   (c1) a polynucleotide that consists of a base sequence of SEQ ID NO: 3;
   (c2) a polynucleotide that consists of a base sequence corresponding to the base sequence of the polynucleotide (c1) in which 1 to 20 bases are deleted, substituted, inserted, and/or added with a 101st base (A) being conserved and has a function equivalent to that of the polynucleotide (c1) regarding *Eustoma* fusarium wilt resistance in the *Eustoma* fusarium wilt resistance locus; or
   (c3) a polynucleotide that consists of a base sequence having at least 90% identity to the base sequence of the polynucleotide (c1) with the 101st base (A) of the polynucleotide (c1) being conserved and has a function equivalent to that of the polynucleotide (c1) regarding *Eustoma* fusarium wilt resistance in the *Eustoma* fusarium wilt resistance locus;
(d) a polynucleotide (d1), (d2), or (d3) below:
   (d1) a polynucleotide that consists of a base sequence of SEQ ID NO: 4;
   (d2) a polynucleotide that consists of a base sequence corresponding to the base sequence of the polynucleotide (d1) in which 1 to 15 bases are deleted, substituted, inserted, and/or added with a 76th base (G) being conserved and has a function equivalent to that of the polynucleotide (d1) regarding *Eustoma* fusarium wilt resistance in the *Eustoma* fusarium wilt resistance locus; or
   (d3) a polynucleotide that consists of a base sequence having at least 90% identity to the base sequence of the polynucleotide (d1) with the 76th base (G) of the polynucleotide (d1) being conserved and has a function equivalent to that of the polynucleotide (d1) regarding *Eustoma* fusarium wilt resistance in the *Eustoma* fusarium wilt resistance locus;
(f) a polynucleotide (f1), (f2), or (f3) below:
   (f1) a polynucleotide that consists of a base sequence of SEQ ID NO: 72;
   (f2) a polynucleotide that consists of a base sequence corresponding to the base sequence of the polynucleotide (f1) in which 1 to 15 bases are deleted, substituted, inserted, and/or added with a 51st base (A) being conserved and has a function equivalent to that of the polynucleotide (f1) regarding *Eustoma* fusarium wilt resistance in the *Eustoma* fusarium wilt resistance locus; or
   (f3) a polynucleotide that consists of a base sequence having at least 90% identity to the base sequence of the polynucleotide (f1) with the 51st base (A) of the polynucleotide (f1) being conserved and has a function equivalent to that of the polynucleotide (f1) regarding *Eustoma* fusarium wilt resistance in the *Eustoma* fusarium wilt resistance locus;
(g) a polynucleotide (g1), (g2), or (g3) below:
   (g1) a polynucleotide that consists of a base sequence of SEQ ID NO: 73;
   (g2) a polynucleotide that consists of a base sequence corresponding to the base sequence of the polynucleotide (g1) in which 1 to 15 bases are deleted, substituted, inserted, and/or added with a 51st base (T) being conserved and has a function equivalent to that of the polynucleotide (g1) regarding *Eustoma* fusarium wilt resistance in the *Eustoma* fusarium wilt resistance locus; or
   (g3) a polynucleotide that consists of a base sequence having at least 90% identity to the base sequence of the polynucleotide (g1) with the 51st base (T) of the polynucleotide (g1) being conserved and has a function equivalent to that of the polynucleotide (g1) regarding *Eustoma* fusarium wilt resistance in the *Eustoma* fusarium wilt resistance locus;
(h) a polynucleotide (h1), (h2), or (h3) below:
   (h1) a polynucleotide that consists of a base sequence of SEQ ID NO: 74;
   (h2) a polynucleotide that consists of a base sequence corresponding to the base sequence of the polynucleotide (h1) in which 1 to 15 bases are deleted, substituted, inserted, and/or added with a 51st base (T) being conserved and has a function equivalent to that of the polynucleotide (h1) regarding *Eustoma* fusarium wilt resistance in the *Eustoma* fusarium wilt resistance locus; or
   (h3) a polynucleotide that consists of a base sequence having at least 90% identity to the base sequence of the polynucleotide (h1) with the 51st base (T) of the polynucleotide (h1) being conserved and has a function equivalent to that of the polynucleotide (h1) regarding *Eustoma* fusarium wilt resistance in the *Eustoma* fusarium wilt resistance locus;
(i) a polynucleotide (i1), (i2), or (i3) below:
   (i1) a polynucleotide that consists of a base sequence of SEQ ID NO: 75;
   (i2) a polynucleotide that consists of a base sequence corresponding to the base sequence of the polynucleotide (i1) in which 1 to 15 bases are deleted, substituted, inserted, and/or added with a 51st base (A) being conserved and has a function equivalent to that of the polynucleotide (i1) regarding *Eustoma* fusarium wilt resistance in the *Eustoma* fusarium wilt resistance locus; or
   (i3) a polynucleotide that consists of a base sequence having at least 90% identity to the base sequence of the polynucleotide (i1) with the 51st base (A) of the polynucleotide (i 1) being conserved and has a function equivalent to that of the polynucleotide (i 1) regarding *Eustoma* fusarium wilt resistance in the *Eustoma* fusarium wilt resistance locus;
(j) a polynucleotide (j 1), (j2) or (j3) below:
   (j 1) a polynucleotide that consists of a base sequence of SEQ ID NO: 76;
   (j2) a polynucleotide that consists of a base sequence corresponding to the base sequence of the polynucleotide (j 1) in which 1 to 15 bases are deleted, substituted, inserted, and/or added with a 51st base (G) being conserved and has a function equivalent to that of the polynucleotide (j 1) regarding *Eustoma* fusarium wilt resistance in the *Eustoma* fusarium wilt resistance locus; or
   (j3) a polynucleotide that consists of a base sequence having at least 90% identity to the base sequence of the polynucleotide (j 1) with the 51st base (G) in the polynucleotide (j 1) being conserved and has a function equivalent to that of the polynucleotide (j 1) regarding *Eustoma* fusarium wilt resistance in the *Eustoma* fusarium wilt resistance locus;
(k) a polynucleotide (k1), (k2) or (k3) below:
   (k1) a polynucleotide that consists of a base sequence of SEQ ID NO: 77;
   (k2) a polynucleotide that consists of a base sequence corresponding to the base sequence of the polynucleotide (k1) in which 1 to 15 bases are deleted, substituted, inserted, and/or added with a 51st base (T) being conserved and has a function equivalent to that of the polynucleotide (k1) regarding *Eustoma* fusarium wilt resistance in the *Eustoma* fusarium wilt resistance locus; or
   (k3) a polynucleotide that consists of a base sequence having at least 90% identity to the base sequence of the polynucleotide (k1) with the 51st base (T) of the polynucleotide (k1) being conserved and has a function equivalent to that of the polynucleotide (k1) regarding *Eustoma* fusarium wilt resistance in the *Eustoma* fusarium wilt resistance locus; and
(l) a polynucleotide (l1), (l2) or (l3) below:
   (11) a polynucleotide that consists of a base sequence of SEQ ID NO: 78;
   (l2) a polynucleotide that consists of a base sequence corresponding to the base sequence of the polynucleotide (11) in which 1 to 15 bases are deleted, substituted, inserted, and/or added with a 51st base (A) being conserved and has a function equivalent to that of the polynucleotide (11) regarding *Eustoma* fusarium wilt resistance in the *Eustoma* fusarium wilt resistance locus; or
   (13) a polynucleotide that consists of a base sequence having at least 90% identity to the base sequence of the polynucleotide (11) with the 51st base (A) of the polynucleotide (11) being conserved and has a function equivalent to that of the polynucleotide (11) regarding *Eustoma* fusarium wilt resistance in the *Eustoma* fusarium wilt resistance locus.

### (Supplementary Note 5)

The *Eustoma* plant according to Supplementary Note 4, wherein;
the *Eustoma* fusarium wilt resistance locus is identified by a polynucleotide selected from the group consisting of the polynucleotides (b), (c), and (f) to (l).

### (Supplementary Note 6)

The *Eustoma* plant according to any one of Supplementary Notes 1 to 5, wherein;
the *Eustoma* fusarium wilt resistance locus is located in a region between sites of two SNP markers selected from the group consisting of Egra20_3656645, Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 and/or the group consisting of SNPs 1 to 66 on the chromosome.

### (Supplementary Note 7)

The *Eustoma* plant according to Supplementary Note 6, wherein;
the *Eustoma* fusarium wilt resistance locus is located in a region between sites of the SNP markers Egra20_3759258 and Egra20_3939402 or a region between sites of the SNP markers Egra20_3833476 and Egra20_3914662 on the chromosome.

### (Supplementary Note 8)

The *Eustoma* plant according to Supplementary Note 7, wherein;
the *Eustoma* fusarium wilt resistance locus is:
located in a region between sites of the SNP markers Egra20_3759258 and Egra20_3939402 on the chromosome and identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402;
located in a region between sites of the SNP markers Egra20_3833476 and Egra20_3914662 on the chromosome and identified by an SNP marker selected from the group consisting of Egra20_3833476, Egra20_3839851, Egra20_3849059, Egra20_3854638, Egra20_3857016, Egra20_3879982, and Egra20_3914662;
located in a region between sites of the SNP markers Egra20_3759258 and Egra20_3939402 on the chromosome and is identified by an SNP marker selected from the group consisting of SNPs 14 to 59 and 60; or
located in a region between sites of the SNP markers Egra20_3833476 and Egra20_3914662 on the chromosome and identified by an SNP marker selected from the group consisting of SNPs 18 to 48 and 49.

### (Supplementary Note 9)

The *Eustoma* plant according to any one of Supplementary Notes 1 to 8, which is a *Eustoma* plant identified by Accession No. FERM BP-22427 or a progeny line thereof.

### (Supplementary Note 10)

The *Eustoma* plant according to any one of Supplementary Notes 1 to 9, wherein;
the *Eustoma* fusarium wilt resistance locus is derived from a *Eustoma* fusarium wilt resistance locus of a *Eustoma* plant identified by Accession No. FERM BP-22427.

### <Part of Eustoma Fusarium Wilt-resistant Eustoma Plant>

### (Supplementary Note 11)

A part of the *Eustoma* fusarium wilt-resistant *Eustoma* plant according to any one of Supplementary Notes 1 to 10.

### (Supplementary Note 12)

The part of the plant according to Supplementary Note 11, wherein the part of the plant is a seed.

### <Production Method>

### (Supplementary Note 13)

A method for producing a *Eustoma* fusarium wilt-resistant *Eustoma* plant, the method including the following steps (a) and (b):
(a) crossing the *Eustoma* fusarium wilt-resistant *Eustoma* plant according to any one of Supplementary Notes 1 to 10 with another *Eustoma* plant; and
(b) selecting a *Eustoma* fusarium wilt-resistant *Eustoma* plant from one or more *Eustoma* plants obtained in the step (a) or one or more progeny lines thereof.

### (Supplementary Note 14)

The production method according to Supplementary Note 13, further including the following step (x) prior to the step (a):
(x) selecting the *Eustoma* fusarium wilt-resistant *Eustoma* plant according to any one of Supplementary Notes 1 to 10 from one or more *Eustoma* plants to be examined.

### (Supplementary Note 15)

The production method according to Supplementary Note 14, wherein;
the selection in the (x) is the selection of a *Eustoma* fusarium wilt-resistant *Eustoma* plant including a *Eustoma* fusarium wilt resistance locus on chromosome 20.

### (Supplementary Note 16)

The production method according to Supplementary Note 15, wherein;
the selection in step (x) is the selection of a *Eustoma* fusarium wilt-resistant *Eustoma* plant including a *Eustoma* fusarium wilt resistance locus identified by an SNP marker selected from the group consisting of Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 and/or an SNP marker selected from the group consisting of SNPs 1 to 65 and 66.

### (Supplementary Note 17)

The production method according to Supplementary Note 16, wherein;
the selection in the step (x) is selection of a *Eustoma* fusarium wilt-resistant *Eustoma* plant including a *Eustoma* fusarium wilt resistance locus identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402.

### (Supplementary Note 18)

The production method according to Supplementary Notes 15 to 17, wherein;
the selection in step (x) is the selection of a *Eustoma* fusarium wilt-resistant *Eustoma* plant including a *Eustoma* fusarium wilt resistance locus identified by a polynucleotide selected from the group consisting of polynucleotides (a) to (d) and (f) to (l) below and/or a polynucleotide selected from the group consisting of polynucleotides including SNPs 1 to 65 and 66:
(a) a polynucleotide (a1), (a2), or (a3) below:
   (a1) a polynucleotide that consists of a base sequence of SEQ ID NO: 1;
   (a2) a polynucleotide that consists of a base sequence corresponding to the base sequence of the polynucleotide (a1) in which 1 to 20 bases are deleted, substituted, inserted, and/or added with a 101st base (T) being conserved and has a function equivalent to that of the polynucleotide (a1) regarding *Eustoma* fusarium wilt resistance in the *Eustoma* fusarium wilt resistance locus; or
   (a3) a polynucleotide that consists of a base sequence having at least 90% identity to the base sequence of the polynucleotide (a1) with the 101st base (T) of the polynucleotide (a1) being conserved and has a function equivalent to that of the polynucleotide (a1) regarding *Eustoma* fusarium wilt resistance in the *Eustoma* fusarium wilt resistance locus;
(b) a polynucleotide (b1), (b2), or (b3) below:
   (b1) a polynucleotide that consists of a base sequence of SEQ ID NO: 2;
   (b2) a polynucleotide that consists of a base sequence corresponding to the base sequence of the polynucleotide (b1) in which 1 to 20 bases are deleted, substituted, inserted, and/or added with a 101st base (A) being conserved and has a function equivalent to that of the polynucleotide (b1) regarding *Eustoma* fusarium wilt resistance in the *Eustoma* fusarium wilt resistance locus; or
   (b3) a polynucleotide that consists of a base sequence having at least 90% identity to the base sequence of the polynucleotide (b1) with the 101st base (A) of the polynucleotide (b1) being conserved and has a function equivalent to that of the polynucleotide (b1) regarding *Eustoma* fusarium wilt resistance in the *Eustoma* fusarium wilt resistance locus;
(c) a polynucleotide (c1), (c2), or (c3) below:
   (c1) a polynucleotide that consists of a base sequence of SEQ ID NO: 3;
   (c2) a polynucleotide that consists of a base sequence corresponding to the base sequence of the polynucleotide (c1) in which 1 to 20 bases are deleted, substituted, inserted, and/or added with a 101st base (A) being conserved and has a function equivalent to that of the polynucleotide (c1) regarding *Eustoma* fusarium wilt resistance in the *Eustoma* fusarium wilt resistance locus; or
   (c3) a polynucleotide that consists of a base sequence having at least 90% identity to the base sequence of the polynucleotide (c1) with the 101st base (A) of the polynucleotide (c1) being conserved and has a function equivalent to that of the polynucleotide (c1) regarding *Eustoma* fusarium wilt resistance in the *Eustoma* fusarium wilt resistance locus;
(d) a polynucleotide (d1), (d2), or (d3) below:
   (d1) a polynucleotide that consists of a base sequence of SEQ ID NO: 4;
   (d2) a polynucleotide that consists of a base sequence corresponding to the base sequence of the polynucleotide (d1) in which 1 to 15 bases are deleted, substituted, inserted, and/or added with a 76th base (G) being conserved and has a function equivalent to that of the polynucleotide (d1) regarding *Eustoma* fusarium wilt resistance in the *Eustoma* fusarium wilt resistance locus; or
   (d3) a polynucleotide that consists of a base sequence having at least 90% identity to the base sequence of the polynucleotide (d1) with the 76th base (G) of the polynucleotide (d1) being conserved and has a function equivalent to that of the polynucleotide (d1) regarding *Eustoma* fusarium wilt resistance in the *Eustoma* fusarium wilt resistance locus;
(f) a polynucleotide (f1), (f2), or (f3) below:
   (f1) a polynucleotide that consists of a base sequence of SEQ ID NO: 72;
   (f2) a polynucleotide that consists of a base sequence corresponding to the base sequence of the polynucleotide (f1) in which 1 to 15 bases are deleted, substituted, inserted, and/or added with a 51st base (A) being conserved and has a function equivalent to that of the polynucleotide (f1) regarding *Eustoma* fusarium wilt resistance in the *Eustoma* fusarium wilt resistance locus; or
   (f3) a polynucleotide that consists of a base sequence having at least 90% identity to the base sequence of the polynucleotide (f1) with the 51st base (A) of the polynucleotide (f1) being conserved and has a function equivalent to that of the polynucleotide (f1) regarding *Eustoma* fusarium wilt resistance in the *Eustoma* fusarium wilt resistance locus;
(g) a polynucleotide (g1), (g2), or (g3) below:
   (g1) a polynucleotide that consists of a base sequence of SEQ ID NO: 73;
   (g2) a polynucleotide that consists of a base sequence corresponding to the base sequence of the polynucleotide (g1) in which 1 to 15 bases are deleted, substituted, inserted, and/or added with a 51st base (T) being conserved and has a function equivalent to that of the polynucleotide (g1) regarding *Eustoma* fusarium wilt resistance in the *Eustoma* fusarium wilt resistance locus; or
   (g3) a polynucleotide that consists of a base sequence having at least 90% identity to the base sequence of the polynucleotide (g1) with the 51st base (T) of the polynucleotide (g1) being conserved and has a function equivalent to that of the polynucleotide (g1) regarding *Eustoma* fusarium wilt resistance in the *Eustoma* fusarium wilt resistance locus;
(h) a polynucleotide (h1), (h2), or (h3) below:
   (h1) a polynucleotide that consists of a base sequence of SEQ ID NO: 74;
   (h2) a polynucleotide that consists of a base sequence corresponding to the base sequence of the polynucleotide (h1) in which 1 to 15 bases are deleted, substituted, inserted, and/or added with a 51st base (T) being conserved and has a function equivalent to that of the polynucleotide (h1) regarding *Eustoma* fusarium wilt resistance in the *Eustoma* fusarium wilt resistance locus; or
   (h3) a polynucleotide that consists of a base sequence having at least 90% identity to the base sequence of the polynucleotide (h1) with the 51st base (T) of the polynucleotide (h1) being conserved and has a function equivalent to that of the polynucleotide (h1) regarding *Eustoma* fusarium wilt resistance in the *Eustoma* fusarium wilt resistance locus;
(i) a polynucleotide (i1), (i2), or (i3) below:
   (i1) a polynucleotide that consists of a base sequence of SEQ ID NO: 75;
   (i2) a polynucleotide that consists of a base sequence corresponding to the base sequence of the polynucleotide (i1) in which 1 to 15 bases are deleted, substituted, inserted, and/or added with a 51st base (A) being conserved and has a function equivalent to that of the polynucleotide (i1) regarding *Eustoma* fusarium wilt resistance in the *Eustoma* fusarium wilt resistance locus; or
   (i3) a polynucleotide that consists of a base sequence having at least 90% identity to the base sequence of the polynucleotide (i 1) with the 51st base (A) of the polynucleotide (i 1) being conserved and has a function equivalent to that of the polynucleotide (i 1) regarding *Eustoma* fusarium wilt resistance in the *Eustoma* fusarium wilt resistance locus;
(j) a polynucleotide (j 1), (j2) or (j3) below:
   (j 1) a polynucleotide that consists of a base sequence of SEQ ID NO: 76;
   (j2) a polynucleotide that consists of a base sequence corresponding to the base sequence of the polynucleotide (j 1) in which 1 to 15 bases are deleted, substituted, inserted, and/or added with a 51st base (G) being conserved and has a function equivalent to that of the polynucleotide (j 1) regarding *Eustoma* fusarium wilt resistance in the *Eustoma* fusarium wilt resistance locus; or
   (j3) a polynucleotide that consists of a base sequence having at least 90% identity to the base sequence of the polynucleotide (j 1) with the 51st base (G) in the polynucleotide (j 1) being conserved and has a function equivalent to that of the polynucleotide (j 1) regarding *Eustoma* fusarium wilt resistance in the *Eustoma* fusarium wilt resistance locus;
(k) a polynucleotide (k1), (k2) or (k3) below:
   (k1) a polynucleotide that consists of a base sequence of SEQ ID NO: 77;
   (k2) a polynucleotide that consists of a base sequence corresponding to the base sequence of the polynucleotide (k1) in which 1 to 15 bases are deleted, substituted, inserted, and/or added with a 51st base (T) being conserved and has a function equivalent to that of the polynucleotide (k1) regarding *Eustoma* fusarium wilt resistance in the *Eustoma* fusarium wilt resistance locus; or
   (k3) a polynucleotide that consists of a base sequence having at least 90% identity to the base sequence of the polynucleotide (k1) with the 51st base (T) of the polynucleotide (k1) being conserved and has a function equivalent to that of the polynucleotide (k1) regarding *Eustoma* fusarium wilt resistance in the *Eustoma* fusarium wilt resistance locus; and
(l) a polynucleotide (l1), (l2) or (l3) below:
   (11) a polynucleotide that consists of a base sequence of SEQ ID NO: 78;
   (l2) a polynucleotide that consists of a base sequence corresponding to the base sequence of the polynucleotide (11) in which 1 to 15 bases are deleted, substituted, inserted, and/or added with a 51st base (A) being conserved and has a function equivalent to that of the polynucleotide (11) regarding *Eustoma* fusarium wilt resistance in the *Eustoma* fusarium wilt resistance locus; or
   (13) a polynucleotide that consists of a base sequence having at least 90% identity to the base sequence of the polynucleotide (11) with the 51st base (A) of the polynucleotide (11) being conserved and has a function equivalent to that of the polynucleotide (11) regarding *Eustoma* fusarium wilt resistance in the *Eustoma* fusarium wilt resistance locus.

### (Supplementary Note 19)

The production method according to Supplementary Note 18, wherein;
the selection in step (x) is the selection of a *Eustoma* fusarium wilt-resistant *Eustoma* plant including a *Eustoma* fusarium wilt resistance locus identified by a polynucleotide selected from the group consisting of the polynucleotides (b), (c), and (f) to (l).

### (Supplementary Note 20)

The production method according to any one of Supplementary Notes 15 to 19, wherein;
the selection in step (x) is the selection of a *Eustoma* fusarium wilt-resistant *Eustoma* plant including a *Eustoma* fusarium wilt resistance locus located in a region between sites of two SNP markers selected from the group consisting of Egra20_3656645, Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 and/or the group consisting of SNPs 1 to 66 on the chromosome.

### (Supplementary Note 21)

The production method according to Supplementary Note 20, wherein;
the selection in step (x) is the selection of a *Eustoma* fusarium wilt-resistant *Eustoma* plant including a *Eustoma* fusarium wilt resistance locus located in a region between sites of the SNP markers Egra20_3759258 and Egra20_3939402 or a region between sites of the SNP markers Egra20_3833476 and Egra20_3914662 on the chromosome.

### (Supplementary Note 22)

The production method according to Supplementary Note 21, wherein;
the selection in step (x) is the selection of a *Eustoma* fusarium wilt-resistant *Eustoma* plant including a *Eustoma* fusarium wilt resistance locus that is:
located in a region between sites of the SNP markers Egra20_3759258 and Egra20_3939402 on the chromosome and identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402;
located in a region between sites of the SNP markers Egra20_3833476 and Egra20_3914662 on the chromosome and identified by an SNP marker selected from the group consisting of Egra20_3833476, Egra20_3839851, Egra20_3849059, Egra20_3854638, Egra20_3857016, Egra20_3879982, and Egra20_3914662;
located in a region between sites of the SNP markers Egra20_3759258 and Egra20_3939402 on the chromosome and is identified by an SNP marker selected from the group consisting of SNPs 14 to 59 and 60; or
located in a region between sites of the SNP markers Egra20_3833476 and Egra20_3914662 on the chromosome and identified by an SNP marker selected from the group consisting of SNPs 18 to 48 and 49.

### (Supplementary Note 23)

The production method according to any one of Supplementary Notes 13 to 22, wherein;
the selection in the step (b) is selection of a *Eustoma* fusarium wilt-resistant *Eustoma* plant including a *Eustoma* fusarium wilt resistance locus on chromosome 20.

### (Supplementary Note 24)

The production method according to any one of Supplementary Notes 13 to 23, wherein;
the selection in the step (b) is selection of a *Eustoma* fusarium wilt-resistant *Eustoma* plant including a *Eustoma* fusarium wilt resistance locus identified by an SNP marker selected from the group consisting of Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 and/or an SNP marker selected from the group consisting of SNPs 1 to 65 and 66.

### (Supplementary Note 25)

The production method according to Supplementary Note 24, wherein;
the selection in the step (b) is selection of a *Eustoma* fusarium wilt-resistant *Eustoma* plant including a *Eustoma* fusarium wilt resistance locus identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402.

### (Supplementary Note 26)

The production method according to any one of Supplementary Notes 13 to 25, wherein;
the selection in the step (b) is selection of a *Eustoma* fusarium wilt-resistant *Eustoma* plant including a *Eustoma* fusarium wilt resistance locus identified by a polynucleotide selected from the group consisting of polynucleotides (a) to (d) and (f) to (l) below and/or a polynucleotide selected from the group consisting of polynucleotides including SNPs 1 to 65 and 66:
(a) a polynucleotide (a1), (a2), or (a3) below:
   (a1) a polynucleotide that consists of a base sequence of SEQ ID NO: 1;
   (a2) a polynucleotide that consists of a base sequence corresponding to the base sequence of the polynucleotide (a1) in which 1 to 20 bases are deleted, substituted, inserted, and/or added with a 101st base (T) being conserved and has a function equivalent to that of the polynucleotide (a1) regarding *Eustoma* fusarium wilt resistance in the *Eustoma* fusarium wilt resistance locus; or
   (a3) a polynucleotide that consists of a base sequence having at least 90% identity to the base sequence of the polynucleotide (a1) with the 101st base (T) of the polynucleotide (a1) being conserved and has a function equivalent to that of the polynucleotide (a1) regarding *Eustoma* fusarium wilt resistance in the *Eustoma* fusarium wilt resistance locus;
(b) a polynucleotide (b1), (b2), or (b3) below:
   (b1) a polynucleotide that consists of a base sequence of SEQ ID NO: 2;
   (b2) a polynucleotide that consists of a base sequence corresponding to the base sequence of the polynucleotide (b1) in which 1 to 20 bases are deleted, substituted, inserted, and/or added with a 101st base (A) being conserved and has a function equivalent to that of the polynucleotide (b1) regarding *Eustoma* fusarium wilt resistance in the *Eustoma* fusarium wilt resistance locus; or
   (b3) a polynucleotide that consists of a base sequence having at least 90% identity to the base sequence of the polynucleotide (b1) with the 101st base (A) of the polynucleotide (b1) being conserved and has a function equivalent to that of the polynucleotide (b1) regarding *Eustoma* fusarium wilt resistance in the *Eustoma* fusarium wilt resistance locus;
(c) a polynucleotide (c1), (c2), or (c3) below:
   (c1) a polynucleotide that consists of a base sequence of SEQ ID NO: 3;
   (c2) a polynucleotide that consists of a base sequence corresponding to the base sequence of the polynucleotide (c1) in which 1 to 20 bases are deleted, substituted, inserted, and/or added with a 101st base (A) being conserved and has a function equivalent to that of the polynucleotide (c1) regarding *Eustoma* fusarium wilt resistance in the *Eustoma* fusarium wilt resistance locus; or
   (c3) a polynucleotide that consists of a base sequence having at least 90% identity to the base sequence of the polynucleotide (c1) with the 101st base (A) of the polynucleotide (c1) being conserved and has a function equivalent to that of the polynucleotide (c1) regarding *Eustoma* fusarium wilt resistance in the *Eustoma* fusarium wilt resistance locus;
(d) a polynucleotide (d1), (d2), or (d3) below:
   (d1) a polynucleotide that consists of a base sequence of SEQ ID NO: 4;
   (d2) a polynucleotide that consists of a base sequence corresponding to the base sequence of the polynucleotide (d1) in which 1 to 15 bases are deleted, substituted, inserted, and/or added with a 76th base (G) being conserved and has a function equivalent to that of the polynucleotide (d1) regarding *Eustoma* fusarium wilt resistance in the *Eustoma* fusarium wilt resistance locus; or
   (d3) a polynucleotide that consists of a base sequence having at least 90% identity to the base sequence of the polynucleotide (d1) with the 76th base (G) of the polynucleotide (d1) being conserved and has a function equivalent to that of the polynucleotide (d1) regarding *Eustoma* fusarium wilt resistance in the *Eustoma* fusarium wilt resistance locus;
(f) a polynucleotide (f1), (f2), or (f3) below:
   (f1) a polynucleotide that consists of a base sequence of SEQ ID NO: 72;
   (f2) a polynucleotide that consists of a base sequence corresponding to the base sequence of the polynucleotide (f1) in which 1 to 15 bases are deleted, substituted, inserted, and/or added with a 51st base (A) being conserved and has a function equivalent to that of the polynucleotide (f1) regarding *Eustoma* fusarium wilt resistance in the *Eustoma* fusarium wilt resistance locus; or
   (f3) a polynucleotide that consists of a base sequence having at least 90% identity to the base sequence of the polynucleotide (f1) with the 51st base (A) of the polynucleotide (f1) being conserved and has a function equivalent to that of the polynucleotide (f1) regarding *Eustoma* fusarium wilt resistance in the *Eustoma* fusarium wilt resistance locus;
(g) a polynucleotide (g1), (g2), or (g3) below:
   (g1) a polynucleotide that consists of a base sequence of SEQ ID NO: 73;
   (g2) a polynucleotide that consists of a base sequence corresponding to the base sequence of the polynucleotide (g1) in which 1 to 15 bases are deleted, substituted, inserted, and/or added with a 51st base (T) being conserved and has a function equivalent to that of the polynucleotide (g1) regarding *Eustoma* fusarium wilt resistance in the *Eustoma* fusarium wilt resistance locus; or
   (g3) a polynucleotide that consists of a base sequence having at least 90% identity to the base sequence of the polynucleotide (g1) with the 51st base (T) of the polynucleotide (g1) being conserved and has a function equivalent to that of the polynucleotide (g1) regarding *Eustoma* fusarium wilt resistance in the *Eustoma* fusarium wilt resistance locus;
(h) a polynucleotide (h1), (h2), or (h3) below:
   (h1) a polynucleotide that consists of a base sequence of SEQ ID NO: 74;
   (h2) a polynucleotide that consists of a base sequence corresponding to the base sequence of the polynucleotide (h1) in which 1 to 15 bases are deleted, substituted, inserted, and/or added with a 51st base (T) being conserved and has a function equivalent to that of the polynucleotide (h1) regarding *Eustoma* fusarium wilt resistance in the *Eustoma* fusarium wilt resistance locus; or
   (h3) a polynucleotide that consists of a base sequence having at least 90% identity to the base sequence of the polynucleotide (h1) with the 51st base (T) of the polynucleotide (h1) being conserved and has a function equivalent to that of the polynucleotide (h1) regarding *Eustoma* fusarium wilt resistance in the *Eustoma* fusarium wilt resistance locus;
(i) a polynucleotide (i1), (i2), or (i3) below:
   (i1) a polynucleotide that consists of a base sequence of SEQ ID NO: 75;
   (i2) a polynucleotide that consists of a base sequence corresponding to the base sequence of the polynucleotide (i1) in which 1 to 15 bases are deleted, substituted, inserted, and/or added with a 51st base (A) being conserved and has a function equivalent to that of the polynucleotide (i1) regarding *Eustoma* fusarium wilt resistance in the *Eustoma* fusarium wilt resistance locus; or
   (i3) a polynucleotide that consists of a base sequence having at least 90% identity to the base sequence of the polynucleotide (i 1) with the 51st base (A) of the polynucleotide (i 1) being conserved and has a function equivalent to that of the polynucleotide (i 1) regarding *Eustoma* fusarium wilt resistance in the *Eustoma* fusarium wilt resistance locus;
(j) a polynucleotide (j 1), (j2) or (j3) below:
   (j 1) a polynucleotide that consists of a base sequence of SEQ ID NO: 76;
   (j2) a polynucleotide that consists of a base sequence corresponding to the base sequence of the polynucleotide (j 1) in which 1 to 15 bases are deleted, substituted, inserted, and/or added with a 51st base (G) being conserved and has a function equivalent to that of the polynucleotide (j 1) regarding *Eustoma* fusarium wilt resistance in the *Eustoma* fusarium wilt resistance locus; or
   (j3) a polynucleotide that consists of a base sequence having at least 90% identity to the base sequence of the polynucleotide (j 1) with the 51st base (G) in the polynucleotide (j 1) being conserved and has a function equivalent to that of the polynucleotide (j 1) regarding *Eustoma* fusarium wilt resistance in the *Eustoma* fusarium wilt resistance locus;
(k) a polynucleotide (k1), (k2) or (k3) below:
   (k1) a polynucleotide that consists of a base sequence of SEQ ID NO: 77;
   (k2) a polynucleotide that consists of a base sequence corresponding to the base sequence of the polynucleotide (k1) in which 1 to 15 bases are deleted, substituted, inserted, and/or added with a 51st base (T) being conserved and has a function equivalent to that of the polynucleotide (k1) regarding *Eustoma* fusarium wilt resistance in the *Eustoma* fusarium wilt resistance locus; or
   (k3) a polynucleotide that consists of a base sequence having at least 90% identity to the base sequence of the polynucleotide (k1) with the 51st base (T) of the polynucleotide (k1) being conserved and has a function equivalent to that of the polynucleotide (k1) regarding *Eustoma* fusarium wilt resistance in the *Eustoma* fusarium wilt resistance locus; and
(l) a polynucleotide (l1), (l2) or (l3) below:
   (l1) a polynucleotide that consists of a base sequence of SEQ ID NO: 78;
   (l2) a polynucleotide that consists of a base sequence corresponding to the base sequence of the polynucleotide (11) in which 1 to 15 bases are deleted, substituted, inserted, and/or added with a 51st base (A) being conserved and has a function equivalent to that of the polynucleotide (11) regarding *Eustoma* fusarium wilt resistance in the *Eustoma* fusarium wilt resistance locus; or
   (13) a polynucleotide that consists of a base sequence having at least 90% identity to the base sequence of the polynucleotide (11) with the 51st base (A) of the polynucleotide (11) being conserved and has a function equivalent to that of the polynucleotide (11) regarding *Eustoma* fusarium wilt resistance in the *Eustoma* fusarium wilt resistance locus.

### (Supplementary Note 27)

The production method according to Supplementary Note 26, wherein;
the selection in the step (b) is selection of a *Eustoma* fusarium wilt-resistant *Eustoma* plant including a *Eustoma* fusarium wilt resistance locus identified by a polynucleotide selected from the group consisting of the polynucleotides (b), (c), and (f) to (l).

### (Supplementary Note 28)

The production method according to any one of Supplementary Notes 13 to 27, wherein;
the selection in the step (b) is selection of a *Eustoma* fusarium wilt-resistant *Eustoma* plant including a *Eustoma* fusarium wilt resistance locus located in a region between sites of two SNP markers selected from the group consisting of Egra20_3656645, Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 and/or the group consisting of SNPs 1 to 66 on the chromosome.

### (Supplementary Note 29)

The production method according to Supplementary Note 28, wherein;
the selection in the step (b) is selection of a *Eustoma* fusarium wilt-resistant *Eustoma* plant including a *Eustoma* fusarium wilt resistance locus located in a region between sites of the SNP markers Egra20_3759258 and Egra20_3939402 or a region between sites of the SNP markers Egra20_3833476 and Egra20_3914662 on the chromosome.

### (Supplementary Note 30)

The production method according to Supplementary Note 29, wherein;
the selection in the step (b) is selection of a *Eustoma* fusarium wilt-resistant *Eustoma* plant including a *Eustoma* fusarium wilt resistance locus that is:
located in a region between sites of the SNP markers Egra20_3759258 and Egra20_3939402 on the chromosome and identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402;
located in a region between sites of the SNP markers Egra20_3833476 and Egra20_3914662 on the chromosome and identified by an SNP marker selected from the group consisting of Egra20_3833476, Egra20_3839851, Egra20_3849059, Egra20_3854638, Egra20_3857016, Egra20_3879982, and Egra20_3914662;
located in a region between sites of the SNP markers Egra20_3759258 and Egra20_3939402 on the chromosome and is identified by an SNP marker selected from the group consisting of SNPs 14 to 59 and 60; or
located in a region between sites of the SNP markers Egra20_3833476 and Egra20_3914662 on the chromosome and identified by an SNP marker selected from the group consisting of SNPs 18 to 48 and 49.

### <Conferring Method>

### (Supplementary Note 31)

A method for conferring *Eustoma* fusarium wilt resistance to a *Eustoma* plant, the method including the step of:
introducing a *Eustoma* fusarium wilt resistance locus on chromosome 20 to a *Eustoma* plant.

### (Supplementary Note 32)

The conferring method according to Supplementary Note 31, wherein;
the *Eustoma* fusarium wilt resistance locus is identified by an SNP marker selected from the group consisting of Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 and/or an SNP marker selected from the group consisting of SNPs 1 to 65 and 66.

### (Supplementary Note 33)

The conferring method according to Supplementary Note 32, wherein
the *Eustoma* fusarium wilt resistance locus is identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402.

### (Supplementary Note 34)

The conferring method according to any one of Supplementary Notes 31 to 33, wherein;
the *Eustoma* fusarium wilt resistance locus is identified by a polynucleotide selected from the group consisting of polynucleotides (a) to (d) and (f) to (l) below and/or a polynucleotide selected from the group consisting of polynucleotides including SNPs 1 to 65 and 66:
(a) a polynucleotide (a1), (a2), or (a3) below:
   (a1) a polynucleotide that consists of a base sequence of SEQ ID NO: 1;
   (a2) a polynucleotide that consists of a base sequence corresponding to the base sequence of the polynucleotide (a1) in which 1 to 20 bases are deleted, substituted, inserted, and/or added with a 101st base (T) being conserved and has a function equivalent to that of the polynucleotide (a1) regarding *Eustoma* fusarium wilt resistance in the *Eustoma* fusarium wilt resistance locus; or
   (a3) a polynucleotide that consists of a base sequence having at least 90% identity to the base sequence of the polynucleotide (a1) with the 101st base (T) of the polynucleotide (a1) being conserved and has a function equivalent to that of the polynucleotide (a1) regarding *Eustoma* fusarium wilt resistance in the *Eustoma* fusarium wilt resistance locus;
(b) a polynucleotide (b1), (b2), or (b3) below:
   (b1) a polynucleotide that consists of a base sequence of SEQ ID NO: 2;
   (b2) a polynucleotide that consists of a base sequence corresponding to the base sequence of the polynucleotide (b1) in which 1 to 20 bases are deleted, substituted, inserted, and/or added with a 101st base (A) being conserved and has a function equivalent to that of the polynucleotide (b1) regarding *Eustoma* fusarium wilt resistance in the *Eustoma* fusarium wilt resistance locus; or
   (b3) a polynucleotide that consists of a base sequence having at least 90% identity to the base sequence of the polynucleotide (b1) with the 101st base (A) of the polynucleotide (b1) being conserved and has a function equivalent to that of the polynucleotide (b1) regarding *Eustoma* fusarium wilt resistance in the *Eustoma* fusarium wilt resistance locus;
(c) a polynucleotide (c1), (c2), or (c3) below:
   (c1) a polynucleotide that consists of a base sequence of SEQ ID NO: 3;
   (c2) a polynucleotide that consists of a base sequence corresponding to the base sequence of the polynucleotide (c1) in which 1 to 20 bases are deleted, substituted, inserted, and/or added with a 101st base (A) being conserved and has a function equivalent to that of the polynucleotide (c1) regarding *Eustoma* fusarium wilt resistance in the *Eustoma* fusarium wilt resistance locus; or
   (c3) a polynucleotide that consists of a base sequence having at least 90% identity to the base sequence of the polynucleotide (c1) with the 101st base (A) of the polynucleotide (c1) being conserved and has a function equivalent to that of the polynucleotide (c1) regarding *Eustoma* fusarium wilt resistance in the *Eustoma* fusarium wilt resistance locus;
(d) a polynucleotide (d1), (d2), or (d3) below:
   (d1) a polynucleotide that consists of a base sequence of SEQ ID NO: 4;
   (d2) a polynucleotide that consists of a base sequence corresponding to the base sequence of the polynucleotide (d1) in which 1 to 15 bases are deleted, substituted, inserted, and/or added with a 76th base (G) being conserved and has a function equivalent to that of the polynucleotide (d1) regarding *Eustoma* fusarium wilt resistance in the *Eustoma* fusarium wilt resistance locus; or
   (d3) a polynucleotide that consists of a base sequence having at least 90% identity to the base sequence of the polynucleotide (d1) with the 76th base (G) of the polynucleotide (d1) being conserved and has a function equivalent to that of the polynucleotide (d1) regarding *Eustoma* fusarium wilt resistance in the *Eustoma* fusarium wilt resistance locus;
(f) a polynucleotide (f1), (f2), or (f3) below:
   (f1) a polynucleotide that consists of a base sequence of SEQ ID NO: 72;
   (f2) a polynucleotide that consists of a base sequence corresponding to the base sequence of the polynucleotide (f1) in which 1 to 15 bases are deleted, substituted, inserted, and/or added with a 51st base (A) being conserved and has a function equivalent to that of the polynucleotide (f1) regarding *Eustoma* fusarium wilt resistance in the *Eustoma* fusarium wilt resistance locus; or
   (f3) a polynucleotide that consists of a base sequence having at least 90% identity to the base sequence of the polynucleotide (f1) with the 51st base (A) of the polynucleotide (f1) being conserved and has a function equivalent to that of the polynucleotide (f1) regarding *Eustoma* fusarium wilt resistance in the *Eustoma* fusarium wilt resistance locus;
(g) a polynucleotide (g1), (g2), or (g3) below:
   (g1) a polynucleotide that consists of a base sequence of SEQ ID NO: 73;
   (g2) a polynucleotide that consists of a base sequence corresponding to the base sequence of the polynucleotide (g1) in which 1 to 15 bases are deleted, substituted, inserted, and/or added with a 51st base (T) being conserved and has a function equivalent to that of the polynucleotide (g1) regarding *Eustoma* fusarium wilt resistance in the *Eustoma* fusarium wilt resistance locus; or
   (g3) a polynucleotide that consists of a base sequence having at least 90% identity to the base sequence of the polynucleotide (g1) with the 51st base (T) of the polynucleotide (g1) being conserved and has a function equivalent to that of the polynucleotide (g1) regarding *Eustoma* fusarium wilt resistance in the *Eustoma* fusarium wilt resistance locus;
(h) a polynucleotide (h1), (h2), or (h3) below:
   (h1) a polynucleotide that consists of a base sequence of SEQ ID NO: 74;
   (h2) a polynucleotide that consists of a base sequence corresponding to the base sequence of the polynucleotide (h1) in which 1 to 15 bases are deleted, substituted, inserted, and/or added with a 51st base (T) being conserved and has a function equivalent to that of the polynucleotide (h1) regarding *Eustoma* fusarium wilt resistance in the *Eustoma* fusarium wilt resistance locus; or
   (h3) a polynucleotide that consists of a base sequence having at least 90% identity to the base sequence of the polynucleotide (h1) with the 51st base (T) of the polynucleotide (h1) being conserved and has a function equivalent to that of the polynucleotide (h1) regarding *Eustoma* fusarium wilt resistance in the *Eustoma* fusarium wilt resistance locus;
(i) a polynucleotide (i1), (i2), or (i3) below:
   (i1) a polynucleotide that consists of a base sequence of SEQ ID NO: 75;
   (i2) a polynucleotide that consists of a base sequence corresponding to the base sequence of the polynucleotide (i1) in which 1 to 15 bases are deleted, substituted, inserted, and/or added with a 51st base (A) being conserved and has a function equivalent to that of the polynucleotide (i1) regarding *Eustoma* fusarium wilt resistance in the *Eustoma* fusarium wilt resistance locus; or
   (i3) a polynucleotide that consists of a base sequence having at least 90% identity to the base sequence of the polynucleotide (i1) with the 51st base (A) of the polynucleotide (i1) being conserved and has a function equivalent to that of the polynucleotide (i1) regarding *Eustoma* fusarium wilt resistance in the *Eustoma* fusarium wilt resistance locus;
(j) a polynucleotide (j 1), (j2) or (j3) below:
   (j 1) a polynucleotide that consists of a base sequence of SEQ ID NO: 76;
   (j2) a polynucleotide that consists of a base sequence corresponding to the base sequence of the polynucleotide (j 1) in which 1 to 15 bases are deleted, substituted, inserted, and/or added with a 51st base (G) being conserved and has a function equivalent to that of the polynucleotide (j 1) regarding *Eustoma* fusarium wilt resistance in the *Eustoma* fusarium wilt resistance locus; or
   (j3) a polynucleotide that consists of a base sequence having at least 90% identity to the base sequence of the polynucleotide (j 1) with the 51st base (G) in the polynucleotide (j 1) being conserved and has a function equivalent to that of the polynucleotide (j 1) regarding *Eustoma* fusarium wilt resistance in the *Eustoma* fusarium wilt resistance locus;
(k) a polynucleotide (k1), (k2) or (k3) below:
   (k1) a polynucleotide that consists of a base sequence of SEQ ID NO: 77;
   (k2) a polynucleotide that consists of a base sequence corresponding to the base sequence of the polynucleotide (k1) in which 1 to 15 bases are deleted, substituted, inserted, and/or added with a 51st base (T) being conserved and has a function equivalent to that of the polynucleotide (k1) regarding *Eustoma* fusarium wilt resistance in the *Eustoma* fusarium wilt resistance locus; or
   (k3) a polynucleotide that consists of a base sequence having at least 90% identity to the base sequence of the polynucleotide (k1) with the 51st base (T) of the polynucleotide (k1) being conserved and has a function equivalent to that of the polynucleotide (k1) regarding *Eustoma* fusarium wilt resistance in the *Eustoma* fusarium wilt resistance locus; and
(l) a polynucleotide (l1), (l2) or (l3) below:
   (l1) a polynucleotide that consists of a base sequence of SEQ ID NO: 78;
   (l2) a polynucleotide that consists of a base sequence corresponding to the base sequence of the polynucleotide (11) in which 1 to 15 bases are deleted, substituted, inserted, and/or added with a 51st base (A) being conserved and has a function equivalent to that of the polynucleotide (11) regarding *Eustoma* fusarium wilt resistance in the *Eustoma* fusarium wilt resistance locus; or
   (l3) a polynucleotide that consists of a base sequence having at least 90% identity to the base sequence of the polynucleotide (11) with the 51st base (A) of the polynucleotide (l1) being conserved and has a function equivalent to that of the polynucleotide (11) regarding *Eustoma* fusarium wilt resistance in the *Eustoma* fusarium wilt resistance locus.

### (Supplementary Note 35)

The conferring method according to Supplementary Note 34, wherein;
the *Eustoma* fusarium wilt resistance locus is identified by a polynucleotide selected from the group consisting of the polynucleotides (b), (c), and (f) to (l).

### (Supplementary Note 36)

The conferring method according to any one of Supplementary Notes 31 to 35, wherein;
the *Eustoma* fusarium wilt resistance locus is located in a region between sites of two SNP markers selected from the group consisting of Egra20_3656645, Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 and/or the group consisting of SNPs 1 to 66 on the chromosome.

### (Supplementary Note 37)

The conferring method according to Supplementary Note 36, wherein;
the *Eustoma* fusarium wilt resistance locus is located in a region between sites of the SNP markers Egra20_3759258 and Egra20_3939402 or a region between sites of the SNP markers Egra20_3833476 and Egra20_3914662 on the chromosome.

### (Supplementary Note 38)

The conferring method according to Supplementary Note 37, wherein;
the *Eustoma* fusarium wilt resistance locus is:
located in a region between sites of the SNP markers Egra20_3759258 and Egra20_3939402 on the chromosome and identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402;
located in a region between sites of the SNP markers Egra20_3833476 and Egra20_3914662 on the chromosome and identified by an SNP marker selected from the group consisting of Egra20_3833476, Egra20_3839851, Egra20_3849059, Egra20_3854638, Egra20_3857016, Egra20_3879982, and Egra20_3914662;
located in a region between sites of the SNP markers Egra20_3759258 and Egra20_3939402 on the chromosome and is identified by an SNP marker selected from the group consisting of SNPs 14 to 59 and 60; or
located in a region between sites of the SNP markers Egra20_3833476 and Egra20_3914662 on the chromosome and identified by an SNP marker selected from the group consisting of SNPs 18 to 48 and 49.

### (Supplementary Note 39)

The conferring method according to any one of Supplementary Notes 31 to 38, wherein;
the *Eustoma* fusarium wilt resistance locus is derived from a *Eustoma* fusarium wilt resistance locus of a *Eustoma* plant identified by Accession No. FERM BP-22427.

### (Supplementary Note 40)

The conferring method according to any one of Supplementary Notes 31 to 39, wherein;
the *Eustoma* fusarium wilt resistance locus on chromosome 20 is introduced by crossing with the *Eustoma* fusarium wilt-resistant *Eustoma* plant according to any one of Supplementary Notes 1 to 10.

### (Supplementary Note 41)

The conferring method according to Supplementary Note 40, wherein;
the *Eustoma* fusarium wilt-resistant *Eustoma* plant is a *Eustoma* plant identified by Accession No. FERM BP-22427 or a progeny line thereof.

### <Eustoma Fusarium Wilt Resistance Marker for Eustoma Plants>

### (Supplementary Note 42)

A *Eustoma* fusarium wilt resistance marker for *Eustoma* plant, including:
a *Eustoma* fusarium wilt resistance locus on chromosome 20.

### (Supplementary Note 43)

The marker according to Supplementary Note 42, wherein;
the *Eustoma* fusarium wilt resistance locus is identified by an SNP marker selected from the group consisting of Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 and/or an SNP marker selected from the group consisting of SNPs 1 to 65 and 66.

### (Supplementary Note 44)

The marker according to Supplementary Note 43, wherein;
the *Eustoma* fusarium wilt resistance locus is identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402.

### (Supplementary Note 45)

The marker according to any one of Supplementary Notes 42 to 44, wherein;
the *Eustoma* fusarium wilt resistance locus is identified by a polynucleotide selected from the group consisting of polynucleotides (a) to (d) and (f) to (l) below and/or a polynucleotide selected from the group consisting of polynucleotides including SNPs 1 to 65 and 66:
(a) a polynucleotide (a1), (a2), or (a3) below:
   (a1) a polynucleotide that consists of a base sequence of SEQ ID NO: 1;
   (a2) a polynucleotide that consists of a base sequence corresponding to the base sequence of the polynucleotide (a1) in which 1 to 20 bases are deleted, substituted, inserted, and/or added with a 101st base (T) being conserved and has a function equivalent to that of the polynucleotide (a1) regarding *Eustoma* fusarium wilt resistance in the *Eustoma* fusarium wilt resistance locus; or
   (a3) a polynucleotide that consists of a base sequence having at least 90% identity to the base sequence of the polynucleotide (a1) with the 101st base (T) of the polynucleotide (a1) being conserved and has a function equivalent to that of the polynucleotide (a1) regarding *Eustoma* fusarium wilt resistance in the *Eustoma* fusarium wilt resistance locus;
(b) a polynucleotide (b1), (b2), or (b3) below:
   (b1) a polynucleotide that consists of a base sequence of SEQ ID NO: 2;
   (b2) a polynucleotide that consists of a base sequence corresponding to the base sequence of the polynucleotide (b1) in which 1 to 20 bases are deleted, substituted, inserted, and/or added with a 101st base (A) being conserved and has a function equivalent to that of the polynucleotide (b1) regarding *Eustoma* fusarium wilt resistance in the *Eustoma* fusarium wilt resistance locus; or
   (b3) a polynucleotide that consists of a base sequence having at least 90% identity to the base sequence of the polynucleotide (b1) with the 101st base (A) of the polynucleotide (b1) being conserved and has a function equivalent to that of the polynucleotide (b1) regarding *Eustoma* fusarium wilt resistance in the *Eustoma* fusarium wilt resistance locus;
(c) a polynucleotide (c1), (c2), or (c3) below:
   (c1) a polynucleotide that consists of a base sequence of SEQ ID NO: 3;
   (c2) a polynucleotide that consists of a base sequence corresponding to the base sequence of the polynucleotide (c1) in which 1 to 20 bases are deleted, substituted, inserted, and/or added with a 101st base (A) being conserved and has a function equivalent to that of the polynucleotide (c1) regarding *Eustoma* fusarium wilt resistance in the *Eustoma* fusarium wilt resistance locus; or
   (c3) a polynucleotide that consists of a base sequence having at least 90% identity to the base sequence of the polynucleotide (c1) with the 101st base (A) of the polynucleotide (c1) being conserved and has a function equivalent to that of the polynucleotide (c1) regarding *Eustoma* fusarium wilt resistance in the *Eustoma* fusarium wilt resistance locus;
(d) a polynucleotide (d1), (d2), or (d3) below:
   (d1) a polynucleotide that consists of a base sequence of SEQ ID NO: 4;
   (d2) a polynucleotide that consists of a base sequence corresponding to the base sequence of the polynucleotide (d1) in which 1 to 15 bases are deleted, substituted, inserted, and/or added with a 76th base (G) being conserved and has a function equivalent to that of the polynucleotide (d1) regarding *Eustoma* fusarium wilt resistance in the *Eustoma* fusarium wilt resistance locus; or
   (d3) a polynucleotide that consists of a base sequence having at least 90% identity to the base sequence of the polynucleotide (d1) with the 76th base (G) of the polynucleotide (d1) being conserved and has a function equivalent to that of the polynucleotide (d1) regarding *Eustoma* fusarium wilt resistance in the *Eustoma* fusarium wilt resistance locus;
(f) a polynucleotide (f1), (f2), or (f3) below:
   (f1) a polynucleotide that consists of a base sequence of SEQ ID NO: 72;
   (f2) a polynucleotide that consists of a base sequence corresponding to the base sequence of the polynucleotide (f1) in which 1 to 15 bases are deleted, substituted, inserted, and/or added with a 51st base (A) being conserved and has a function equivalent to that of the polynucleotide (f1) regarding *Eustoma* fusarium wilt resistance in the *Eustoma* fusarium wilt resistance locus; or
   (f3) a polynucleotide that consists of a base sequence having at least 90% identity to the base sequence of the polynucleotide (f1) with the 51st base (A) of the polynucleotide (f1) being conserved and has a function equivalent to that of the polynucleotide (f1) regarding *Eustoma* fusarium wilt resistance in the *Eustoma* fusarium wilt resistance locus;
(g) a polynucleotide (g1), (g2), or (g3) below:
   (g1) a polynucleotide that consists of a base sequence of SEQ ID NO: 73;
   (g2) a polynucleotide that consists of a base sequence corresponding to the base sequence of the polynucleotide (g1) in which 1 to 15 bases are deleted, substituted, inserted, and/or added with a 51st base (T) being conserved and has a function equivalent to that of the polynucleotide (g1) regarding *Eustoma* fusarium wilt resistance in the *Eustoma* fusarium wilt resistance locus; or
   (g3) a polynucleotide that consists of a base sequence having at least 90% identity to the base sequence of the polynucleotide (g1) with the 51st base (T) of the polynucleotide (g1) being conserved and has a function equivalent to that of the polynucleotide (g1) regarding *Eustoma* fusarium wilt resistance in the *Eustoma* fusarium wilt resistance locus;
(h) a polynucleotide (h1), (h2), or (h3) below:
   (h1) a polynucleotide that consists of a base sequence of SEQ ID NO: 74;
   (h2) a polynucleotide that consists of a base sequence corresponding to the base sequence of the polynucleotide (h1) in which 1 to 15 bases are deleted, substituted, inserted, and/or added with a 51st base (T) being conserved and has a function equivalent to that of the polynucleotide (h1) regarding *Eustoma* fusarium wilt resistance in the *Eustoma* fusarium wilt resistance locus; or
   (h3) a polynucleotide that consists of a base sequence having at least 90% identity to the base sequence of the polynucleotide (h1) with the 51st base (T) of the polynucleotide (h1) being conserved and has a function equivalent to that of the polynucleotide (h1) regarding *Eustoma* fusarium wilt resistance in the *Eustoma* fusarium wilt resistance locus;
(i) a polynucleotide (i1), (i2), or (i3) below:
   (i1) a polynucleotide that consists of a base sequence of SEQ ID NO: 75;
   (i2) a polynucleotide that consists of a base sequence corresponding to the base sequence of the polynucleotide (i1) in which 1 to 15 bases are deleted, substituted, inserted, and/or added with a 51st base (A) being conserved and has a function equivalent to that of the polynucleotide (i1) regarding *Eustoma* fusarium wilt resistance in the *Eustoma* fusarium wilt resistance locus; or
   (i3) a polynucleotide that consists of a base sequence having at least 90% identity to the base sequence of the polynucleotide (i 1) with the 51st base (A) of the polynucleotide (i 1) being conserved and has a function equivalent to that of the polynucleotide (i 1) regarding *Eustoma* fusarium wilt resistance in the *Eustoma* fusarium wilt resistance locus;
(j) a polynucleotide (j 1), (j2) or (j3) below:
   (j 1) a polynucleotide that consists of a base sequence of SEQ ID NO: 76;
   (j2) a polynucleotide that consists of a base sequence corresponding to the base sequence of the polynucleotide (j 1) in which 1 to 15 bases are deleted, substituted, inserted, and/or added with a 51st base (G) being conserved and has a function equivalent to that of the polynucleotide (j 1) regarding *Eustoma* fusarium wilt resistance in the *Eustoma* fusarium wilt resistance locus; or
   (j3) a polynucleotide that consists of a base sequence having at least 90% identity to the base sequence of the polynucleotide (j 1) with the 51st base (G) in the polynucleotide (j1) being conserved and has a function equivalent to that of the polynucleotide (j1) regarding *Eustoma* fusarium wilt resistance in the *Eustoma* fusarium wilt resistance locus;
(k) a polynucleotide (k1), (k2) or (k3) below:
   (k1) a polynucleotide that consists of a base sequence of SEQ ID NO: 77;
   (k2) a polynucleotide that consists of a base sequence corresponding to the base sequence of the polynucleotide (k1) in which 1 to 15 bases are deleted, substituted, inserted, and/or added with a 51st base (T) being conserved and has a function equivalent to that of the polynucleotide (k1) regarding *Eustoma* fusarium wilt resistance in the *Eustoma* fusarium wilt resistance locus; or
   (k3) a polynucleotide that consists of a base sequence having at least 90% identity to the base sequence of the polynucleotide (k1) with the 51st base (T) of the polynucleotide (k1) being conserved and has a function equivalent to that of the polynucleotide (k1) regarding *Eustoma* fusarium wilt resistance in the *Eustoma* fusarium wilt resistance locus; and
(l) a polynucleotide (l1), (l2) or (l3) below:
   (l1) a polynucleotide that consists of a base sequence of SEQ ID NO: 78;
   (l2) a polynucleotide that consists of a base sequence corresponding to the base sequence of the polynucleotide (l1) in which 1 to 15 bases are deleted, substituted, inserted, and/or added with a 51st base (A) being conserved and has a function equivalent to that of the polynucleotide (l1) regarding *Eustoma* fusarium wilt resistance in the *Eustoma* fusarium wilt resistance locus; or
   (l3) a polynucleotide that consists of a base sequence having at least 90% identity to the base sequence of the polynucleotide (l1) with the 51st base (A) of the polynucleotide (l1) being conserved and has a function equivalent to that of the polynucleotide (11) regarding *Eustoma* fusarium wilt resistance in the *Eustoma* fusarium wilt resistance locus.

### (Supplementary Note 46)

The marker according to Supplementary Note 45, wherein;
the *Eustoma* fusarium wilt resistance locus is identified by a polynucleotide selected from the group consisting of the polynucleotides (b), (c), and (f) to (l).

### (Supplementary Note 47)

The marker according to any one of Supplementary Notes 42 to 46, wherein;
the *Eustoma* fusarium wilt resistance locus is located in a region between sites of two SNP markers selected from the group consisting of Egra20_3656645, Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 and/or the group consisting of SNs 1 to 66 on the chromosome.

### (Supplementary Note 48)

The marker according to Supplementary Note 47, wherein;
the *Eustoma* fusarium wilt resistance locus is located in a region between sites of the SNP markers Egra20_3759258 and Egra20_3939402 or a region between sites of the SNP markers Egra20_3833476 and Egra20_3914662 on the chromosome.

### (Supplementary Note 49)

The marker according to Supplementary Note 48, wherein;
the *Eustoma* fusarium wilt resistance locus is:
located in a region between sites of the SNP markers Egra20_3759258 and Egra20_3939402 on the chromosome and identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402;
located in a region between sites of the SNP markers Egra20_3833476 and Egra20_3914662 on the chromosome and identified by an SNP marker selected from the group consisting of Egra20_3833476, Egra20_3839851, Egra20_3849059, Egra20_3854638, Egra20_3857016, Egra20_3879982, and Egra20_3914662;
located in a region between sites of the SNP markers Egra20_3759258 and Egra20_3939402 on the chromosome and is identified by an SNP marker selected from the group consisting of SNPs 14 to 59 and 60; or
located in a region between sites of the SNP markers Egra20_3833476 and Egra20_3914662 on the chromosome and identified by an SNP marker selected from the group consisting of SNPs 18 to 48 and 49.

### <Method for Detecting Eustoma Fusarium Wilt Resistance of a Eustoma Plant>

### (Supplementary Note 50)

A method for detecting *Eustoma* fusarium wilt resistance of a *Eustoma* plant, the method including the step of:
detecting a *Eustoma* fusarium wilt resistance locus on chromosome 20 in a *Eustoma* plant to be examined.

### (Supplementary Note 51)

The detection method according to Supplementary Note 50, wherein;
in the detecting, the *Eustoma* fusarium wilt resistance locus is detected by detecting an SNP marker selected from the group consisting of Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 and/or an SNP marker selected from the group consisting of SNPs 1 to 65 and 66.

### (Supplementary Note 52)

The detection method according to Supplementary Note 51, wherein;
in the detecting, the *Eustoma* fusarium wilt resistance locus is detected by detecting an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402.

### (Supplementary Note 53)

The detection method according to any one of Supplementary Notes 50 to 52, wherein;
in the detecting, the *Eustoma* fusarium wilt resistance locus is detected by detecting a polynucleotide selected from the group consisting of polynucleotides (a) to (d) and (f) to (l) below and/or a polynucleotide selected from the group consisting of polynucleotides including SNPs 1 to 65 and 66:
(a) a polynucleotide (a1), (a2), or (a3) below:
   (a1) a polynucleotide that consists of a base sequence of SEQ ID NO: 1;
   (a2) a polynucleotide that consists of a base sequence corresponding to the base sequence of the polynucleotide (a1) in which 1 to 20 bases are deleted, substituted, inserted, and/or added with a 101st base (T) being conserved and has a function equivalent to that of the polynucleotide (a1) regarding *Eustoma* fusarium wilt resistance in the *Eustoma* fusarium wilt resistance locus; or
   (a3) a polynucleotide that consists of a base sequence having at least 90% identity to the base sequence of the polynucleotide (a1) with the 101st base (T) of the polynucleotide (a1) being conserved and has a function equivalent to that of the polynucleotide (a1) regarding *Eustoma* fusarium wilt resistance in the *Eustoma* fusarium wilt resistance locus;
(b) a polynucleotide (b1), (b2), or (b3) below:
   (b1) a polynucleotide that consists of a base sequence of SEQ ID NO: 2;
   (b2) a polynucleotide that consists of a base sequence corresponding to the base sequence of the polynucleotide (b1) in which 1 to 20 bases are deleted, substituted, inserted, and/or added with a 101st base (A) being conserved and has a function equivalent to that of the polynucleotide (b1) regarding *Eustoma* fusarium wilt resistance in the *Eustoma* fusarium wilt resistance locus; or
   (b3) a polynucleotide that consists of a base sequence having at least 90% identity to the base sequence of the polynucleotide (b1) with the 101st base (A) of the polynucleotide (b1) being conserved and has a function equivalent to that of the polynucleotide (b1) regarding *Eustoma* fusarium wilt resistance in the *Eustoma* fusarium wilt resistance locus;
(c) a polynucleotide (c1), (c2), or (c3) below:
   (c1) a polynucleotide that consists of a base sequence of SEQ ID NO: 3;
   (c2) a polynucleotide that consists of a base sequence corresponding to the base sequence of the polynucleotide (c1) in which 1 to 20 bases are deleted, substituted, inserted, and/or added with a 101st base (A) being conserved and has a function equivalent to that of the polynucleotide (c1) regarding *Eustoma* fusarium wilt resistance in the *Eustoma* fusarium wilt resistance locus; or
   (c3) a polynucleotide that consists of a base sequence having at least 90% identity to the base sequence of the polynucleotide (c1) with the 101st base (A) of the polynucleotide (c1) being conserved and has a function equivalent to that of the polynucleotide (c1) regarding *Eustoma* fusarium wilt resistance in the *Eustoma* fusarium wilt resistance locus;
(d) a polynucleotide (d1), (d2), or (d3) below:
   (d1) a polynucleotide that consists of a base sequence of SEQ ID NO: 4;
   (d2) a polynucleotide that consists of a base sequence corresponding to the base sequence of the polynucleotide (d1) in which 1 to 15 bases are deleted, substituted, inserted, and/or added with a 76th base (G) being conserved and has a function equivalent to that of the polynucleotide (d1) regarding *Eustoma* fusarium wilt resistance in the *Eustoma* fusarium wilt resistance locus; or
   (d3) a polynucleotide that consists of a base sequence having at least 90% identity to the base sequence of the polynucleotide (d1) with the 76th base (G) of the polynucleotide (d1) being conserved and has a function equivalent to that of the polynucleotide (d1) regarding *Eustoma* fusarium wilt resistance in the *Eustoma* fusarium wilt resistance locus;
(f) a polynucleotide (f1), (f2), or (f3) below:
   (f1) a polynucleotide that consists of a base sequence of SEQ ID NO: 72;
   (f2) a polynucleotide that consists of a base sequence corresponding to the base sequence of the polynucleotide (f1) in which 1 to 15 bases are deleted, substituted, inserted, and/or added with a 51st base (A) being conserved and has a function equivalent to that of the polynucleotide (f1) regarding *Eustoma* fusarium wilt resistance in the *Eustoma* fusarium wilt resistance locus; or
   (f3) a polynucleotide that consists of a base sequence having at least 90% identity to the base sequence of the polynucleotide (f1) with the 51st base (A) of the polynucleotide (f1) being conserved and has a function equivalent to that of the polynucleotide (f1) regarding *Eustoma* fusarium wilt resistance in the *Eustoma* fusarium wilt resistance locus;
(g) a polynucleotide (g1), (g2), or (g3) below:
   (g1) a polynucleotide that consists of a base sequence of SEQ ID NO: 73;
   (g2) a polynucleotide that consists of a base sequence corresponding to the base sequence of the polynucleotide (g1) in which 1 to 15 bases are deleted, substituted, inserted, and/or added with a 51st base (T) being conserved and has a function equivalent to that of the polynucleotide (g1) regarding *Eustoma* fusarium wilt resistance in the *Eustoma* fusarium wilt resistance locus; or
   (g3) a polynucleotide that consists of a base sequence having at least 90% identity to the base sequence of the polynucleotide (g1) with the 51st base (T) of the polynucleotide (g1) being conserved and has a function equivalent to that of the polynucleotide (g1) regarding *Eustoma* fusarium wilt resistance in the *Eustoma* fusarium wilt resistance locus;
(h) a polynucleotide (h1), (h2), or (h3) below:
   (h1) a polynucleotide that consists of a base sequence of SEQ ID NO: 74;
   (h2) a polynucleotide that consists of a base sequence corresponding to the base sequence of the polynucleotide (h1) in which 1 to 15 bases are deleted, substituted, inserted, and/or added with a 51st base (T) being conserved and has a function equivalent to that of the polynucleotide (h1) regarding *Eustoma* fusarium wilt resistance in the *Eustoma* fusarium wilt resistance locus; or
   (h3) a polynucleotide that consists of a base sequence having at least 90% identity to the base sequence of the polynucleotide (h1) with the 51st base (T) of the polynucleotide (h1) being conserved and has a function equivalent to that of the polynucleotide (h1) regarding *Eustoma* fusarium wilt resistance in the *Eustoma* fusarium wilt resistance locus;
(i) a polynucleotide (i1), (i2), or (i3) below:
   (i1) a polynucleotide that consists of a base sequence of SEQ ID NO: 75;
   (i2) a polynucleotide that consists of a base sequence corresponding to the base sequence of the polynucleotide (i1) in which 1 to 15 bases are deleted, substituted, inserted, and/or added with a 51st base (A) being conserved and has a function equivalent to that of the polynucleotide (i1) regarding *Eustoma* fusarium wilt resistance in the *Eustoma* fusarium wilt resistance locus; or
   (i3) a polynucleotide that consists of a base sequence having at least 90% identity to the base sequence of the polynucleotide (i1) with the 51st base (A) of the polynucleotide (i1) being conserved and has a function equivalent to that of the polynucleotide (i1) regarding *Eustoma* fusarium wilt resistance in the *Eustoma* fusarium wilt resistance locus;
(j) a polynucleotide (j1), (j2) or (j3) below:
   (j1) a polynucleotide that consists of a base sequence of SEQ ID NO: 76;
   (j2) a polynucleotide that consists of a base sequence corresponding to the base sequence of the polynucleotide (j1) in which 1 to 15 bases are deleted, substituted, inserted, and/or added with a 51st base (G) being conserved and has a function equivalent to that of the polynucleotide (j1) regarding *Eustoma* fusarium wilt resistance in the *Eustoma* fusarium wilt resistance locus; or
   (j3) a polynucleotide that consists of a base sequence having at least 90% identity to the base sequence of the polynucleotide (j1) with the 51st base (G) in the polynucleotide (j1) being conserved and has a function equivalent to that of the polynucleotide (j1) regarding *Eustoma* fusarium wilt resistance in the *Eustoma* fusarium wilt resistance locus;
(k) a polynucleotide (k1), (k2) or (k3) below:
   (k1) a polynucleotide that consists of a base sequence of SEQ ID NO: 77;
   (k2) a polynucleotide that consists of a base sequence corresponding to the base sequence of the polynucleotide (k1) in which 1 to 15 bases are deleted, substituted, inserted, and/or added with a 51st base (T) being conserved and has a function equivalent to that of the polynucleotide (k1) regarding *Eustoma* fusarium wilt resistance in the *Eustoma* fusarium wilt resistance locus; or
   (k3) a polynucleotide that consists of a base sequence having at least 90% identity to the base sequence of the polynucleotide (k1) with the 51st base (T) of the polynucleotide (k1) being conserved and has a function equivalent to that of the polynucleotide (k1) regarding *Eustoma* fusarium wilt resistance in the *Eustoma* fusarium wilt resistance locus; and
(l) a polynucleotide (11), (l2) or (l3) below:
   (l1) a polynucleotide that consists of a base sequence of SEQ ID NO: 78;
   (l2) a polynucleotide that consists of a base sequence corresponding to the base sequence of the polynucleotide (11) in which 1 to 15 bases are deleted, substituted, inserted, and/or added with a 51st base (A) being conserved and has a function equivalent to that of the polynucleotide (l1) regarding *Eustoma* fusarium wilt resistance in the *Eustoma* fusarium wilt resistance locus; or
   (l3) a polynucleotide that consists of a base sequence having at least 90% identity to the base sequence of the polynucleotide (l1) with the 51st base (A) of the polynucleotide (l1) being conserved and has a function equivalent to that of the polynucleotide (l1) regarding *Eustoma* fusarium wilt resistance in the *Eustoma* fusarium wilt resistance locus.

### (Supplementary Note 54)

The detection method according to Supplementary Note 53, wherein;
in the detecting, the *Eustoma* fusarium wilt resistance locus is detected by detecting a polynucleotide selected from the group consisting of the polynucleotides (b), (c), and (f) to (l).

### (Supplementary Note 55)

The detection method according to any one of Supplementary Notes 50 to 54, wherein;
the *Eustoma* fusarium wilt resistance locus detected in the detecting is a *Eustoma* fusarium wilt resistance locus located in a region between sites of two SNP markers selected from the group consisting of Egra20_3656645, Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 and/or the group consisting of SNPs 1 to 66 on the chromosome.

### (Supplementary Note 56)

The detection method according to Supplementary Note 55, wherein;
the *Eustoma* fusarium wilt resistance locus detected in the detecting is a *Eustoma* fusarium wilt resistance locus located in a region between sites of the SNP markers Egra20_3759258 and Egra20_3939402 or a region between sites of the SNP markers Egra20_3833476 and Egra20_3914662 on the chromosome.

### (Supplementary Note 57)

The detection method according to Supplementary Note 56, wherein;
the *Eustoma* fusarium wilt resistance locus detected in the detecting is a *Eustoma* fusarium wilt resistance locus that is:
located in a region between sites of the SNP markers Egra20_3759258 and Egra20_3939402 on the chromosome and identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402;
located in a region between sites of the SNP markers Egra20_3833476 and Egra20_3914662 on the chromosome and identified by an SNP marker selected from the group consisting of Egra20_3833476, Egra20_3839851, Egra20_3849059, Egra20_3854638, Egra20_3857016, Egra20_3879982, and Egra20_3914662;
located in a region between sites of the SNP markers Egra20_3759258 and Egra20_3939402 on the chromosome and is identified by an SNP marker selected from the group consisting of SNPs 14 to 59 and 60; or
located in a region between sites of the SNP markers Egra20_3833476 and Egra20_3914662 on the chromosome and identified by an SNP marker selected from the group consisting of SNPs 18 to 48 and 49.

### <Screening Method for Eustoma Fusarium Wilt-resistant Eustoma Plant>

### (Supplementary Note 58)

A screening method for a *Eustoma* fusarium wilt-resistant *Eustoma* plant, the screening method including:
a selection step of selecting a *Eustoma* plant that includes a *Eustoma* fusarium wilt resistance locus on chromosome 20 as a *Eustoma* fusarium wilt-resistant *Eustoma* plantfrom one or more *Eustoma* plants to be examined.

### (Supplementary Note 59)

The screening method according to Supplementary Note 58, wherein;
the *Eustoma* plant selected as the *Eustoma* fusarium wilt-resistant *Eustoma* plant in the selection step is a *Eustoma* fusarium wilt-resistant *Eustoma* plant including a *Eustoma* fusarium wilt resistance locus identified by an SNP marker selected from the group consisting of Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 and/or an SNP marker selected from the group consisting of SNPs 1 to 65 and 66.

### (Supplementary Note 60)

The screening method according to Supplementary Note 59, wherein;
the *Eustoma* plant selected as the *Eustoma* fusarium wilt-resistant *Eustoma* plant in the selection step is a *Eustoma* fusarium wilt-resistant *Eustoma* plant including a *Eustoma* fusarium wilt resistance locus identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402.

### (Supplementary Note 61)

The screening method according to any one of Supplementary Notes 58 to 60, wherein;
the *Eustoma* plant selected as the *Eustoma* fusarium wilt-resistant *Eustoma* plant in the selection step is a *Eustoma* fusarium wilt-resistant *Eustoma* plant including a *Eustoma* fusarium wilt resistance locus identified by a polynucleotide selected from the group consisting of polynucleotides (a) to (d) and (f) to (l) below and/or a polynucleotide selected from the group consisting of polynucleotides including SNPs 1 to 65 and 66:
(a) a polynucleotide (a1), (a2), or (a3) below:
   (a1) a polynucleotide that consists of a base sequence of SEQ ID NO: 1;
   (a2) a polynucleotide that consists of a base sequence corresponding to the base sequence of the polynucleotide (a1) in which 1 to 20 bases are deleted, substituted, inserted, and/or added with a 101st base (T) being conserved and has a function equivalent to that of the polynucleotide (a1) regarding *Eustoma* fusarium wilt resistance in the *Eustoma* fusarium wilt resistance locus; or
   (a3) a polynucleotide that consists of a base sequence having at least 90% identity to the base sequence of the polynucleotide (a1) with the 101st base (T) of the polynucleotide (a1) being conserved and has a function equivalent to that of the polynucleotide (a1) regarding *Eustoma* fusarium wilt resistance in the *Eustoma* fusarium wilt resistance locus;
(b) a polynucleotide (b1), (b2), or (b3) below:
   (b1) a polynucleotide that consists of a base sequence of SEQ ID NO: 2;
   (b2) a polynucleotide that consists of a base sequence corresponding to the base sequence of the polynucleotide (b1) in which 1 to 20 bases are deleted, substituted, inserted, and/or added with a 101st base (A) being conserved and has a function equivalent to that of the polynucleotide (b1) regarding *Eustoma* fusarium wilt resistance in the *Eustoma* fusarium wilt resistance locus; or
   (b3) a polynucleotide that consists of a base sequence having at least 90% identity to the base sequence of the polynucleotide (b1) with the 101st base (A) of the polynucleotide (b1) being conserved and has a function equivalent to that of the polynucleotide (b1) regarding *Eustoma* fusarium wilt resistance in the *Eustoma* fusarium wilt resistance locus;
(c) a polynucleotide (c1), (c2), or (c3) below:
   (c1) a polynucleotide that consists of a base sequence of SEQ ID NO: 3;
   (c2) a polynucleotide that consists of a base sequence corresponding to the base sequence of the polynucleotide (c1) in which 1 to 20 bases are deleted, substituted, inserted, and/or added with a 101st base (A) being conserved and has a function equivalent to that of the polynucleotide (c1) regarding *Eustoma* fusarium wilt resistance in the *Eustoma* fusarium wilt resistance locus; or
   (c3) a polynucleotide that consists of a base sequence having at least 90% identity to the base sequence of the polynucleotide (c1) with the 101st base (A) of the polynucleotide (c1) being conserved and has a function equivalent to that of the polynucleotide (c1) regarding *Eustoma* fusarium wilt resistance in the *Eustoma* fusarium wilt resistance locus;
(d) a polynucleotide (d1), (d2), or (d3) below:
   (d1) a polynucleotide that consists of a base sequence of SEQ ID NO: 4;
   (d2) a polynucleotide that consists of a base sequence corresponding to the base sequence of the polynucleotide (d1) in which 1 to 15 bases are deleted, substituted, inserted, and/or added with a 76th base (G) being conserved and has a function equivalent to that of the polynucleotide (d1) regarding *Eustoma* fusarium wilt resistance in the *Eustoma* fusarium wilt resistance locus; or
   (d3) a polynucleotide that consists of a base sequence having at least 90% identity to the base sequence of the polynucleotide (d1) with the 76th base (G) of the polynucleotide (d1) being conserved and has a function equivalent to that of the polynucleotide (d1) regarding *Eustoma* fusarium wilt resistance in the *Eustoma* fusarium wilt resistance locus;
(f) a polynucleotide (f1), (f2), or (f3) below:
   (f1) a polynucleotide that consists of a base sequence of SEQ ID NO: 72;
   (f2) a polynucleotide that consists of a base sequence corresponding to the base sequence of the polynucleotide (f1) in which 1 to 15 bases are deleted, substituted, inserted, and/or added with a 51st base (A) being conserved and has a function equivalent to that of the polynucleotide (f1) regarding *Eustoma* fusarium wilt resistance in the *Eustoma* fusarium wilt resistance locus; or
   (f3) a polynucleotide that consists of a base sequence having at least 90% identity to the base sequence of the polynucleotide (f1) with the 51st base (A) of the polynucleotide (f1) being conserved and has a function equivalent to that of the polynucleotide (f1) regarding *Eustoma* fusarium wilt resistance in the *Eustoma* fusarium wilt resistance locus;
(g) a polynucleotide (g1), (g2), or (g3) below:
   (g1) a polynucleotide that consists of a base sequence of SEQ ID NO: 73;
   (g2) a polynucleotide that consists of a base sequence corresponding to the base sequence of the polynucleotide (g1) in which 1 to 15 bases are deleted, substituted, inserted, and/or added with a 51st base (T) being conserved and has a function equivalent to that of the polynucleotide (g1) regarding *Eustoma* fusarium wilt resistance in the *Eustoma* fusarium wilt resistance locus; or
   (g3) a polynucleotide that consists of a base sequence having at least 90% identity to the base sequence of the polynucleotide (g1) with the 51st base (T) of the polynucleotide (g1) being conserved and has a function equivalent to that of the polynucleotide (g1) regarding *Eustoma* fusarium wilt resistance in the *Eustoma* fusarium wilt resistance locus;
(h) a polynucleotide (h1), (h2), or (h3) below:
   (h1) a polynucleotide that consists of a base sequence of SEQ ID NO: 74;
   (h2) a polynucleotide that consists of a base sequence corresponding to the base sequence of the polynucleotide (h1) in which 1 to 15 bases are deleted, substituted, inserted, and/or added with a 51st base (T) being conserved and has a function equivalent to that of the polynucleotide (h1) regarding *Eustoma* fusarium wilt resistance in the *Eustoma* fusarium wilt resistance locus; or
   (h3) a polynucleotide that consists of a base sequence having at least 90% identity to the base sequence of the polynucleotide (h1) with the 51st base (T) of the polynucleotide (h1) being conserved and has a function equivalent to that of the polynucleotide (h1) regarding *Eustoma* fusarium wilt resistance in the *Eustoma* fusarium wilt resistance locus;
(i) a polynucleotide (i1), (i2), or (i3) below:
   (i1) a polynucleotide that consists of a base sequence of SEQ ID NO: 75;
   (i2) a polynucleotide that consists of a base sequence corresponding to the base sequence of the polynucleotide (i1) in which 1 to 15 bases are deleted, substituted, inserted, and/or added with a 51st base (A) being conserved and has a function equivalent to that of the polynucleotide (i1) regarding *Eustoma* fusarium wilt resistance in the *Eustoma* fusarium wilt resistance locus; or
   (i3) a polynucleotide that consists of a base sequence having at least 90% identity to the base sequence of the polynucleotide (i1) with the 51st base (A) of the polynucleotide (i1) being conserved and has a function equivalent to that of the polynucleotide (i1) regarding *Eustoma* fusarium wilt resistance in the *Eustoma* fusarium wilt resistance locus;
(j) a polynucleotide (j1), (j2) or (j3) below:
   (j1) a polynucleotide that consists of a base sequence of SEQ ID NO: 76;
   (j2) a polynucleotide that consists of a base sequence corresponding to the base sequence of the polynucleotide (j1) in which 1 to 15 bases are deleted, substituted, inserted, and/or added with a 51st base (G) being conserved and has a function equivalent to that of the polynucleotide (j1) regarding *Eustoma* fusarium wilt resistance in the *Eustoma* fusarium wilt resistance locus; or
   (j3) a polynucleotide that consists of a base sequence having at least 90% identity to the base sequence of the polynucleotide (j1) with the 51st base (G) in the polynucleotide (j 1) being conserved and has a function equivalent to that of the polynucleotide (j1) regarding *Eustoma* fusarium wilt resistance in the *Eustoma* fusarium wilt resistance locus;
(k) a polynucleotide (k1), (k2) or (k3) below:
   (k1) a polynucleotide that consists of a base sequence of SEQ ID NO: 77;
   (k2) a polynucleotide that consists of a base sequence corresponding to the base sequence of the polynucleotide (k1) in which 1 to 15 bases are deleted, substituted, inserted, and/or added with a 51st base (T) being conserved and has a function equivalent to that of the polynucleotide (k1) regarding *Eustoma* fusarium wilt resistance in the *Eustoma* fusarium wilt resistance locus; or
   (k3) a polynucleotide that consists of a base sequence having at least 90% identity to the base sequence of the polynucleotide (k1) with the 51st base (T) of the polynucleotide (k1) being conserved and has a function equivalent to that of the polynucleotide (k1) regarding *Eustoma* fusarium wilt resistance in the *Eustoma* fusarium wilt resistance locus; and
(l) a polynucleotide (l1), (l2) or (l3) below:
   (l1) a polynucleotide that consists of a base sequence of SEQ ID NO: 78;
   (l2) a polynucleotide that consists of a base sequence corresponding to the base sequence of the polynucleotide (l1) in which 1 to 15 bases are deleted, substituted, inserted, and/or added with a 51st base (A) being conserved and has a function equivalent to that of the polynucleotide (l1) regarding *Eustoma* fusarium wilt resistance in the *Eustoma* fusarium wilt resistance locus; or
   (l3) a polynucleotide that consists of a base sequence having at least 90% identity to the base sequence of the polynucleotide (l1) with the 51st base (A) of the polynucleotide (l1) being conserved and has a function equivalent to that of the polynucleotide (l1) regarding *Eustoma* fusarium wilt resistance in the *Eustoma* fusarium wilt resistance locus.

### (Supplementary Note 62)

The screening method according to Supplementary Note 61, wherein;
the *Eustoma* plant selected as the *Eustoma* fusarium wilt-resistant *Eustoma* plant in the selection step is a *Eustoma* fusarium wilt-resistant *Eustoma* plant including a *Eustoma* fusarium wilt resistance locus identified by a polynucleotide selected from the group consisting of the polynucleotides (b), (c), and (f) to (l).

### (Supplementary Note 63)

The screening method according to any one of Supplementary Notes 58 to 62, wherein;
the *Eustoma* plant selected as the *Eustoma* fusarium wilt-resistant *Eustoma* plant in the selection step is a *Eustoma* fusarium wilt-resistant *Eustoma* plant including a *Eustoma* fusarium wilt resistance locus located in a region between sites of two SNP markers selected from the group consisting of Egra20_3656645, Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 and/or the group consisting of SNPs 1 to 66 on the chromosome.

### (Supplementary Note 64)

The screening method according to Supplementary Note 63, wherein;
the *Eustoma* plant selected as the *Eustoma* fusarium wilt-resistant *Eustoma* plant in the selection step is a *Eustoma* fusarium wilt-resistant *Eustoma* plant including a *Eustoma* fusarium wilt resistance locus located in a region between sites of the SNP markers Egra20_3759258 and Egra20_3939402 or a region between sites of the SNP markers Egra20_3833476 and Egra20_3914662 on the chromosome.

### (Supplementary Note 65)

The screening method according to Supplementary Note 64, wherein;
the *Eustoma* plant selected as the *Eustoma* fusarium wilt-resistant *Eustoma* plant in the selection step is a *Eustoma* fusarium wilt-resistant *Eustoma* plant including a *Eustoma* fusarium wilt resistance locus that is:
located in a region between sites of the SNP markers Egra20_3759258 and Egra20_3939402 on the chromosome and identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402;
located in a region between sites of the SNP markers Egra20_3833476 and Egra20_3914662 on the chromosome and identified by an SNP marker selected from the group consisting of Egra20_3833476, Egra20_3839851, Egra20_3849059, Egra20_3854638, Egra20_3857016, Egra20_3879982, and Egra20_3914662;
located in a region between sites of the SNP markers Egra20_3759258 and Egra20_3939402 on the chromosome and is identified by an SNP marker selected from the group consisting of SNPs 14 to 59 and 60; or
located in a region between sites of the SNP markers Egra20_3833476 and Egra20_3914662 on the chromosome and identified by an SNP marker selected from the group consisting of SNPs 18 to 48 and 49.

### <Method for Distinguishing Deposited Line or Progeny Line>

### (Supplementary Note 66)

A method for distinguishing a deposited line identified by FERM BP-22427 or a progeny line thereof, the method including:
an SNP detection step of detecting at least one SNP selected from the group consisting of SNPs 1 to 65 and 66 in a *Eustoma* plant to be examined.

### (Supplementary Note 67)

The distinguishing method according to Supplementary Note 66, further including an evaluation step of evaluating whether the *Eustoma* plant to be examined is the deposited line or a progeny line thereof by comparing an SNP of the *Eustoma* plant to be examined with a corresponding reference SNP.

### <Industrial Applicability>

As specifically described above, the present disclosure can provide a *Eustoma* plant that exhibits resistance to *Eustoma* fusarium wilt. Therefore, the present disclosure is very useful in the fields of agriculture and breeding, for example.

## Claims

1. A *Eustoma* fusarium wilt-resistant *Eustoma* plant comprising:
a *Eustoma* fusarium wilt resistance locus on chromosome 20.

2. The *Eustoma* fusarium wilt-resistant *Eustoma* plant according to claim 1, wherein;
the *Eustoma* fusarium wilt resistance locus is identified by an SNP marker selected from the group consisting of Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378.

3. The *Eustoma* fusarium wilt-resistant *Eustoma* plant according to claim 2, wherein;
the *Eustoma* fusarium wilt resistance locus is identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402.

4. The *Eustoma* plant according to any one of claims 1 to 3, wherein;
the *Eustoma* fusarium wilt resistance locus is identified by a polynucleotide selected from the group consisting of polynucleotides (a) to (d) and (f) to (l) below:
(a) a polynucleotide (a1), (a2), or (a3) below:
(a1) a polynucleotide that consists of a base sequence of SEQ ID NO: 1;
(a2) a polynucleotide that consists of a base sequence corresponding to the base sequence of the polynucleotide (a1) in which 1 to 20 bases are deleted, substituted, inserted, and/or added with a 101st base (T) being conserved and has a function equivalent to that of the polynucleotide (a1) regarding *Eustoma* fusarium wilt resistance in the *Eustoma* fusarium wilt resistance locus; or
(a3) a polynucleotide that consists of a base sequence having at least 90% identity to the base sequence of the polynucleotide (a1) with the 101st base (T) of the polynucleotide (a1) being conserved and has a function equivalent to that of the polynucleotide (a1) regarding *Eustoma* fusarium wilt resistance in the *Eustoma* fusarium wilt resistance locus;
(b) a polynucleotide (b1), (b2), or (b3) below:
(b1) a polynucleotide that consists of a base sequence of SEQ ID NO: 2;
(b2) a polynucleotide that consists of a base sequence corresponding to the base sequence of the polynucleotide (b1) in which 1 to 20 bases are deleted, substituted, inserted, and/or added with a 101st base (A) being conserved and has a function equivalent to that of the polynucleotide (b1) regarding *Eustoma* fusarium wilt resistance in the *Eustoma* fusarium wilt resistance locus; or
(b3) a polynucleotide that consists of a base sequence having at least 90% identity to the base sequence of the polynucleotide (b1) with the 101st base (A) of the polynucleotide (b1) being conserved and has a function equivalent to that of the polynucleotide (b1) regarding *Eustoma* fusarium wilt resistance in the *Eustoma* fusarium wilt resistance locus;
(c) a polynucleotide (c1), (c2), or (c3) below:
(c1) a polynucleotide that consists of a base sequence of SEQ ID NO: 3;
(c2) a polynucleotide that consists of a base sequence corresponding to the base sequence of the polynucleotide (c1) in which 1 to 20 bases are deleted, substituted, inserted, and/or added with a 101st base (A) being conserved and has a function equivalent to that of the polynucleotide (c1) regarding *Eustoma* fusarium wilt resistance in the *Eustoma* fusarium wilt resistance locus; or
(c3) a polynucleotide that consists of a base sequence having at least 90% identity to the base sequence of the polynucleotide (c1) with the 101st base (A) of the polynucleotide (c1) being conserved and has a function equivalent to that of the polynucleotide (c1) regarding *Eustoma* fusarium wilt resistance in the *Eustoma* fusarium wilt resistance locus;
(d) a polynucleotide (d1), (d2), or (d3) below:
(d1) a polynucleotide that consists of a base sequence of SEQ ID NO: 4;
(d2) a polynucleotide that consists of a base sequence corresponding to the base sequence of the polynucleotide (d1) in which 1 to 15 bases are deleted, substituted, inserted, and/or added with a 76th base (G) being conserved and has a function equivalent to that of the polynucleotide (d1) regarding *Eustoma* fusarium wilt resistance in the *Eustoma* fusarium wilt resistance locus; or
(d3) a polynucleotide that consists of a base sequence having at least 90% identity to the base sequence of the polynucleotide (d1) with the 76th base (G) of the polynucleotide (d1) being conserved and has a function equivalent to that of the polynucleotide (d1) regarding *Eustoma* fusarium wilt resistance in the *Eustoma* fusarium wilt resistance locus;
(f) a polynucleotide (f1), (f2), or (f3) below:
(f1) a polynucleotide that consists of a base sequence of SEQ ID NO: 72;
(f2) a polynucleotide that consists of a base sequence corresponding to the base sequence of the polynucleotide (f1) in which 1 to 15 bases are deleted, substituted, inserted, and/or added with a 51st base (A) being conserved and has a function equivalent to that of the polynucleotide (f1) regarding *Eustoma* fusarium wilt resistance in the *Eustoma* fusarium wilt resistance locus; or
(f3) a polynucleotide that consists of a base sequence having at least 90% identity to the base sequence of the polynucleotide (f1) with the 51st base (A) of the polynucleotide (f1) being conserved and has a function equivalent to that of the polynucleotide (f1) regarding *Eustoma* fusarium wilt resistance in the *Eustoma* fusarium wilt resistance locus;
(g) a polynucleotide (g1), (g2), or (g3) below:
(g1) a polynucleotide that consists of a base sequence of SEQ ID NO: 73;
(g2) a polynucleotide that consists of a base sequence corresponding to the base sequence of the polynucleotide (g1) in which 1 to 15 bases are deleted, substituted, inserted, and/or added with a 51st base (T) being conserved and has a function equivalent to that of the polynucleotide (g1) regarding *Eustoma* fusarium wilt resistance in the *Eustoma* fusarium wilt resistance locus; or
(g3) a polynucleotide that consists of a base sequence having at least 90% identity to the base sequence of the polynucleotide (g1) with the 51st base (T) of the polynucleotide (g1) being conserved and has a function equivalent to that of the polynucleotide (g1) regarding *Eustoma* fusarium wilt resistance in the *Eustoma* fusarium wilt resistance locus;
(h) a polynucleotide (h1), (h2), or (h3) below:
(h1) a polynucleotide that consists of a base sequence of SEQ ID NO: 74;
(h2) a polynucleotide that consists of a base sequence corresponding to the base sequence of the polynucleotide (h1) in which 1 to 15 bases are deleted, substituted, inserted, and/or added with a 51st base (T) being conserved and has a function equivalent to that of the polynucleotide (h1) regarding *Eustoma* fusarium wilt resistance in the *Eustoma* fusarium wilt resistance locus; or
(h3) a polynucleotide that consists of a base sequence having at least 90% identity to the base sequence of the polynucleotide (h1) with the 51st base (T) of the polynucleotide (h1) being conserved and has a function equivalent to that of the polynucleotide (h1) regarding *Eustoma* fusarium wilt resistance in the *Eustoma* fusarium wilt resistance locus;
(i) a polynucleotide (i1), (i2), or (i3) below:
(i1) a polynucleotide that consists of a base sequence of SEQ ID NO: 75;
(i2) a polynucleotide that consists of a base sequence corresponding to the base sequence of the polynucleotide (i1) in which 1 to 15 bases are deleted, substituted, inserted, and/or added with a 51st base (A) being conserved and has a function equivalent to that of the polynucleotide (i1) regarding *Eustoma* fusarium wilt resistance in the *Eustoma* fusarium wilt resistance locus; or
(i3) a polynucleotide that consists of a base sequence having at least 90% identity to the base sequence of the polynucleotide (i 1) with the 51st base (A) of the polynucleotide (i 1) being conserved and has a function equivalent to that of the polynucleotide (i 1) regarding *Eustoma* fusarium wilt resistance in the *Eustoma* fusarium wilt resistance locus;
(j) a polynucleotide (j1), (j2) or (j3) below:
(j1) a polynucleotide that consists of a base sequence of SEQ ID NO: 76;
(j2) a polynucleotide that consists of a base sequence corresponding to the base sequence of the polynucleotide (j1) in which 1 to 15 bases are deleted, substituted, inserted, and/or added with a 51st base (G) being conserved and has a function equivalent to that of the polynucleotide (j1) regarding *Eustoma* fusarium wilt resistance in the *Eustoma* fusarium wilt resistance locus; or
(j3) a polynucleotide that consists of a base sequence having at least 90% identity to the base sequence of the polynucleotide (j1) with the 51st base (G) in the polynucleotide (j1) being conserved and has a function equivalent to that of the polynucleotide (j1) regarding *Eustoma* fusarium wilt resistance in the *Eustoma* fusarium wilt resistance locus;
(k) a polynucleotide (k1), (k2) or (k3) below:
(k1) a polynucleotide that consists of a base sequence of SEQ ID NO: 77;
(k2) a polynucleotide that consists of a base sequence corresponding to the base sequence of the polynucleotide (k1) in which 1 to 15 bases are deleted, substituted, inserted, and/or added with a 51st base (T) being conserved and has a function equivalent to that of the polynucleotide (k1) regarding *Eustoma* fusarium wilt resistance in the *Eustoma* fusarium wilt resistance locus; or
(k3) a polynucleotide that consists of a base sequence having at least 90% identity to the base sequence of the polynucleotide (k1) with the 51st base (T) of the polynucleotide (k1) being conserved and has a function equivalent to that of the polynucleotide (k1) regarding *Eustoma* fusarium wilt resistance in the *Eustoma* fusarium wilt resistance locus; and
(l) a polynucleotide (l1), (l2) or (l3) below:
(l1) a polynucleotide that consists of a base sequence of SEQ ID NO: 78;
(l2) a polynucleotide that consists of a base sequence corresponding to the base sequence of the polynucleotide (l1) in which 1 to 15 bases are deleted, substituted, inserted, and/or added with a 51st base (A) being conserved and has a function equivalent to that of the polynucleotide (l1) regarding *Eustoma* fusarium wilt resistance in the *Eustoma* fusarium wilt resistance locus; or
(l3) a polynucleotide that consists of a base sequence having at least 90% identity to the base sequence of the polynucleotide (l1) with the 51st base (A) of the polynucleotide (l1) being conserved and has a function equivalent to that of the polynucleotide (l1) regarding *Eustoma* fusarium wilt resistance in the *Eustoma* fusarium wilt resistance locus.

5. The *Eustoma* plant according to claim 4, wherein;
the *Eustoma* fusarium wilt resistance locus is identified by a polynucleotide selected from the group consisting of the polynucleotides (b), (c), and (f) to (l).

6. The *Eustoma* plant according to any one of claims 1 to 4, wherein;
the *Eustoma* fusarium wilt resistance locus is located in a region between sites of two SNP markers selected from the group consisting of Egra20_3656645, Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 on the chromosome.

7. The *Eustoma* plant according to claim 6, wherein;
the *Eustoma* fusarium wilt resistance locus is located in a region between sites of the SNP markers Egra20_3759258 and Egra20_3939402 or a region between sites of the SNP markers Egra20_3833476 and Egra20_3914662 on the chromosome.

8. The *Eustoma* plant according to claim 7, wherein;
the *Eustoma* fusarium wilt resistance locus is:
located in a region between sites of the SNP markers Egra20_3759258 and Egra20_3939402 on the chromosome and identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402; or
located in a region between sites of the SNP markers Egra20_3833476 and Egra20_3914662 on the chromosome and identified by an SNP marker selected from the group consisting of Egra20_3833476, Egra20_3839851, Egra20_3849059, Egra20_3854638, Egra20_3857016, Egra20_3879982, and Egra20_3914662.

9. The *Eustoma* plant according to any one of claims 1 to 8, which is a *Eustoma* plant identified by Accession No. FERM BP-22427 or a progeny line thereof.

10. The *Eustoma* plant according to any one of claims 1 to 9, wherein;
the *Eustoma* fusarium wilt resistance locus is derived from a *Eustoma* fusarium wilt resistance locus of a *Eustoma* plant identified by Accession No. FERM BP-22427.

11. A part of the *Eustoma* fusarium wilt-resistant *Eustoma* plant according to any one of claims 1 to 10.

12. The part of the plant according to claim 11, wherein the part of the plant is a seed.

13. A method for producing a *Eustoma* fusarium wilt-resistant *Eustoma* plant, the method comprising the following steps (a) and (b):
(a) crossing the *Eustoma* fusarium wilt-resistant *Eustoma* plant according to any one of claims 1 to 10 with another *Eustoma* plant; and
(b) selecting a *Eustoma* fusarium wilt-resistant *Eustoma* plant from one or more *Eustoma* plants obtained in the step (a) or one or more progeny lines thereof.

14. The production method according to claim 13, further comprising the following step (x) prior to the step (a):
(x) selecting the *Eustoma* fusarium wilt-resistant *Eustoma* plant according to any one of claims 1 to 10 from one or more *Eustoma* plants to be examined.

15. The production method according to claim 14, wherein;
the selection in step (x) is the selection of a *Eustoma* fusarium wilt-resistant *Eustoma* plant comprising a *Eustoma* fusarium wilt resistance locus on chromosome 20.

16. The production method according to claim 15, wherein;
the selection in step (x) is the selection of a *Eustoma* fusarium wilt-resistant *Eustoma* plant comprising a *Eustoma* fusarium wilt resistance locus identified by an SNP marker selected from the group consisting of Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378.

17. The production method according to claim 16, wherein;
the selection in step (x) is the selection of a *Eustoma* fusarium wilt-resistant *Eustoma* plant comprising a *Eustoma* fusarium wilt resistance locus identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402.

18. The production method according to any one of claims 15 to 17, wherein;
the selection in the step (x) is selection of a *Eustoma* fusarium wilt-resistant *Eustoma* plant comprising a *Eustoma* fusarium wilt resistance locus identified by a polynucleotide selected from the group consisting of polynucleotides (a) to (d) and (f) to (l) below:
(a) a polynucleotide (a1), (a2), or (a3) below:
(a1) a polynucleotide that consists of a base sequence of SEQ ID NO: 1;
(a2) a polynucleotide that consists of a base sequence corresponding to the base sequence of the polynucleotide (a1) in which 1 to 20 bases are deleted, substituted, inserted, and/or added with a 101st base (T) being conserved and has a function equivalent to that of the polynucleotide (a1) regarding *Eustoma* fusarium wilt resistance in the *Eustoma* fusarium wilt resistance locus; or
(a3) a polynucleotide that consists of a base sequence having at least 90% identity to the base sequence of the polynucleotide (a1) with the 101st base (T) of the polynucleotide (a1) being conserved and has a function equivalent to that of the polynucleotide (a1) regarding *Eustoma* fusarium wilt resistance in the *Eustoma* fusarium wilt resistance locus;
(b) a polynucleotide (b1), (b2), or (b3) below:
(b1) a polynucleotide that consists of a base sequence of SEQ ID NO: 2;
(b2) a polynucleotide that consists of a base sequence corresponding to the base sequence of the polynucleotide (b1) in which 1 to 20 bases are deleted, substituted, inserted, and/or added with a 101st base (A) being conserved and has a function equivalent to that of the polynucleotide (b1) regarding *Eustoma* fusarium wilt resistance in the *Eustoma* fusarium wilt resistance locus; or
(b3) a polynucleotide that consists of a base sequence having at least 90% identity to the base sequence of the polynucleotide (b1) with the 101st base (A) of the polynucleotide (b1) being conserved and has a function equivalent to that of the polynucleotide (b1) regarding *Eustoma* fusarium wilt resistance in the *Eustoma* fusarium wilt resistance locus;
(c) a polynucleotide (c1), (c2), or (c3) below:
(c1) a polynucleotide that consists of a base sequence of SEQ ID NO: 3;
(c2) a polynucleotide that consists of a base sequence corresponding to the base sequence of the polynucleotide (c1) in which 1 to 20 bases are deleted, substituted, inserted, and/or added with a 101st base (A) being conserved and has a function equivalent to that of the polynucleotide (c1) regarding *Eustoma* fusarium wilt resistance in the *Eustoma* fusarium wilt resistance locus; or
(c3) a polynucleotide that consists of a base sequence having at least 90% identity to the base sequence of the polynucleotide (c1) with the 101st base (A) of the polynucleotide (c1) being conserved and has a function equivalent to that of the polynucleotide (c1) regarding *Eustoma* fusarium wilt resistance in the *Eustoma* fusarium wilt resistance locus;
(d) a polynucleotide (d1), (d2), or (d3) below:
(d1) a polynucleotide that consists of a base sequence of SEQ ID NO: 4;
(d2) a polynucleotide that consists of a base sequence corresponding to the base sequence of the polynucleotide (d1) in which 1 to 15 bases are deleted, substituted, inserted, and/or added with a 76th base (G) being conserved and has a function equivalent to that of the polynucleotide (d1) regarding *Eustoma* fusarium wilt resistance in the *Eustoma* fusarium wilt resistance locus; or
(d3) a polynucleotide that consists of a base sequence having at least 90% identity to the base sequence of the polynucleotide (d1) with the 76th base (G) of the polynucleotide (d1) being conserved and has a function equivalent to that of the polynucleotide (d1) regarding *Eustoma* fusarium wilt resistance in the *Eustoma* fusarium wilt resistance locus;
(f) a polynucleotide (f1), (f2), or (f3) below:
(f1) a polynucleotide that consists of a base sequence of SEQ ID NO: 72;
(f2) a polynucleotide that consists of a base sequence corresponding to the base sequence of the polynucleotide (f1) in which 1 to 15 bases are deleted, substituted, inserted, and/or added with a 51st base (A) being conserved and has a function equivalent to that of the polynucleotide (f1) regarding *Eustoma* fusarium wilt resistance in the *Eustoma* fusarium wilt resistance locus; or
(f3) a polynucleotide that consists of a base sequence having at least 90% identity to the base sequence of the polynucleotide (f1) with the 51st base (A) of the polynucleotide (f1) being conserved and has a function equivalent to that of the polynucleotide (f1) regarding *Eustoma* fusarium wilt resistance in the *Eustoma* fusarium wilt resistance locus;
(g) a polynucleotide (g1), (g2), or (g3) below:
(g1) a polynucleotide that consists of a base sequence of SEQ ID NO: 73;
(g2) a polynucleotide that consists of a base sequence corresponding to the base sequence of the polynucleotide (g1) in which 1 to 15 bases are deleted, substituted, inserted, and/or added with a 51st base (T) being conserved and has a function equivalent to that of the polynucleotide (g1) regarding *Eustoma* fusarium wilt resistance in the *Eustoma* fusarium wilt resistance locus; or
(g3) a polynucleotide that consists of a base sequence having at least 90% identity to the base sequence of the polynucleotide (g1) with the 51st base (T) of the polynucleotide (g1) being conserved and has a function equivalent to that of the polynucleotide (g1) regarding *Eustoma* fusarium wilt resistance in the *Eustoma* fusarium wilt resistance locus;
(h) a polynucleotide (h1), (h2), or (h3) below:
(h1) a polynucleotide that consists of a base sequence of SEQ ID NO: 74;
(h2) a polynucleotide that consists of a base sequence corresponding to the base sequence of the polynucleotide (h1) in which 1 to 15 bases are deleted, substituted, inserted, and/or added with a 51st base (T) being conserved and has a function equivalent to that of the polynucleotide (h1) regarding *Eustoma* fusarium wilt resistance in the *Eustoma* fusarium wilt resistance locus; or
(h3) a polynucleotide that consists of a base sequence having at least 90% identity to the base sequence of the polynucleotide (h1) with the 51st base (T) of the polynucleotide (h1) being conserved and has a function equivalent to that of the polynucleotide (h1) regarding *Eustoma* fusarium wilt resistance in the *Eustoma* fusarium wilt resistance locus;
(i) a polynucleotide (i1), (i2), or (i3) below:
(i1) a polynucleotide that consists of a base sequence of SEQ ID NO: 75;
(i2) a polynucleotide that consists of a base sequence corresponding to the base sequence of the polynucleotide (i1) in which 1 to 15 bases are deleted, substituted, inserted, and/or added with a 51st base (A) being conserved and has a function equivalent to that of the polynucleotide (i1) regarding *Eustoma* fusarium wilt resistance in the *Eustoma* fusarium wilt resistance locus; or
(i3) a polynucleotide that consists of a base sequence having at least 90% identity to the base sequence of the polynucleotide (i 1) with the 51st base (A) of the polynucleotide (i 1) being conserved and has a function equivalent to that of the polynucleotide (i 1) regarding *Eustoma* fusarium wilt resistance in the *Eustoma* fusarium wilt resistance locus;
(j) a polynucleotide (j1), (j2) or (j3) below:
(j1) a polynucleotide that consists of a base sequence of SEQ ID NO: 76;
(j2) a polynucleotide that consists of a base sequence corresponding to the base sequence of the polynucleotide (j1) in which 1 to 15 bases are deleted, substituted, inserted, and/or added with a 51st base (G) being conserved and has a function equivalent to that of the polynucleotide (j1) regarding *Eustoma* fusarium wilt resistance in the *Eustoma* fusarium wilt resistance locus; or
(j3) a polynucleotide that consists of a base sequence having at least 90% identity to the base sequence of the polynucleotide (j1) with the 51st base (G) in the polynucleotide (j1) being conserved and has a function equivalent to that of the polynucleotide (j1) regarding *Eustoma* fusarium wilt resistance in the *Eustoma* fusarium wilt resistance locus;
(k) a polynucleotide (k1), (k2) or (k3) below:
(k1) a polynucleotide that consists of a base sequence of SEQ ID NO: 77;
(k2) a polynucleotide that consists of a base sequence corresponding to the base sequence of the polynucleotide (k1) in which 1 to 15 bases are deleted, substituted, inserted, and/or added with a 51st base (T) being conserved and has a function equivalent to that of the polynucleotide (k1) regarding *Eustoma* fusarium wilt resistance in the *Eustoma* fusarium wilt resistance locus; or
(k3) a polynucleotide that consists of a base sequence having at least 90% identity to the base sequence of the polynucleotide (k1) with the 51st base (T) of the polynucleotide (k1) being conserved and has a function equivalent to that of the polynucleotide (k1) regarding *Eustoma* fusarium wilt resistance in the *Eustoma* fusarium wilt resistance locus; and
(l) a polynucleotide (l1), (l2) or (l3) below:
(l1) a polynucleotide that consists of a base sequence of SEQ ID NO: 78;
(l2) a polynucleotide that consists of a base sequence corresponding to the base sequence of the polynucleotide (l1) in which 1 to 15 bases are deleted, substituted, inserted, and/or added with a 51st base (A) being conserved and has a function equivalent to that of the polynucleotide (l1) regarding *Eustoma* fusarium wilt resistance in the *Eustoma* fusarium wilt resistance locus; or
(l3) a polynucleotide that consists of a base sequence having at least 90% identity to the base sequence of the polynucleotide (l1) with the 51st base (A) of the polynucleotide (l1) being conserved and has a function equivalent to that of the polynucleotide (l1) regarding *Eustoma* fusarium wilt resistance in the *Eustoma* fusarium wilt resistance locus.

19. The production method according to claim 18, wherein;
the selection in step (x) is the selection of a *Eustoma* fusarium wilt-resistant *Eustoma* plant comprising a *Eustoma* fusarium wilt resistance locus identified by a polynucleotide selected from the group consisting of the polynucleotides (b), (c), and (f) to (l).

20. The production method according to any one of claims 15 to 19, wherein;
the selection in step (x) is the selection of a *Eustoma* fusarium wilt-resistant *Eustoma* plant comprising a *Eustoma* fusarium wilt resistance locus located in a region between sites of two SNP markers selected from the group consisting of Egra20_3656645, Egra20_3703734, Egra20_3759258, Egra20_3833476, Egra20_3839851, Egra20_3849059, Egra20_3854638, Egra20_3857016, Egra20_3879982, Egra20_3914662, Egra20_3939402, and Egra20_3961378 on the chromosome.

21. The production method according to claim 20, wherein;
the selection in step (x) is the selection of a *Eustoma* fusarium wilt-resistant *Eustoma* plant comprising a *Eustoma* fusarium wilt resistance locus located in a region between sites of the SNP markers Egra20_3759258 and Egra20_3939402 or a region between sites of the SNP markers Egra20_3833476 and Egra20_3914662 on the chromosome.

22. The production method according to claim 21, wherein;
the selection in step (x) is the selection of a *Eustoma* fusarium wilt-resistant *Eustoma* plant comprising a *Eustoma* fusarium wilt resistance locus that is:
located in a region between sites of the SNP markers Egra20_3759258 and Egra20_3939402 on the chromosome and identified by an SNP marker selected from the group consisting of Egra20_3759258, Egra20_3833476, Egra20_3839851, Egra20_3849059, Egra20_3854638, Egra20_3857016, Egra20_3879982, Egra20_3914662, and Egra20_3939402; or
located in a region between sites of the SNP markers Egra20_3833476 and Egra20_3914662 on the chromosome and identified by an SNP marker selected from the group consisting of Egra20_3833476, Egra20_3839851, Egra20_3849059, Egra20_3854638, Egra20_3857016, Egra20_3879982, and Egra20_3914662.

23. A method for conferring *Eustoma* fusarium wilt resistance to a *Eustoma* plant, the method comprising:
an introduction step of introducing a *Eustoma* fusarium wilt resistance locus on chromosome 20 to a *Eustoma* plant.

24. The conferring method according to claim 15, wherein;
the *Eustoma* fusarium wilt resistance locus on chromosome 20 is introduced by crossing with the *Eustoma* fusarium wilt-resistant *Eustoma* plant according to any one of claims 1 to 10.

25. A method for detecting *Eustoma* fusarium wilt resistance of a *Eustoma* plant, the method comprising:
a detection step of detecting a *Eustoma* fusarium wilt resistance locus on chromosome 20 in a *Eustoma* plant to be examined.

26. A screening method for a *Eustoma* fusarium wilt-resistant *Eustoma* plant, the screening method comprising:
a selection step of selecting a *Eustoma* plant that comprises a *Eustoma* fusarium wilt resistance locus on chromosome 20 as a *Eustoma* fusarium wilt-resistant *Eustoma* plant from one or more *Eustoma* plants to be examined.
